(19) Europäisches Patentamt
European Patent Office
Office européen des brevets

(11) EP 3 586 741 B1

(12) **EUROPEAN PATENT SPECIFICATION**

(45) Date of publication and mention of the grant of the patent:
**12.04.2023 Bulletin 2023/15**

(21) Application number: **17898104.9**

(22) Date of filing: **21.02.2017**

(51) International Patent Classification (IPC):
***A61B 5/11*** *(2006.01)* *A61B 5/00* *(2006.01)*

(52) Cooperative Patent Classification (CPC):
**A61B 5/1114; A61B 5/1116;** A61B 5/1122; A61B 5/6823; A61B 5/6829; A61B 2562/0219

(86) International application number:
**PCT/JP2017/006362**

(87) International publication number:
**WO 2018/154626 (30.08.2018 Gazette 2018/35)**

(54) **MOVEMENT DETECTION SYSTEM, MOVEMENT DETECTION DEVICE, AND MOVEMENT DETECTION METHOD**

BEWEGUNGSDETEKTIONSSYSTEM, BEWEGUNGSDETEKTIONSVORRICHTUNG UND BEWEGUNGSDETEKTIONSVERFAHREN

SYSTÈME DE DÉTECTION DE DÉPLACEMENT, DISPOSITIF DE DÉTECTION DE DÉPLACEMENT ET PROCÉDÉ DE DÉTECTION DE DÉPLACEMENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**01.01.2020 Bulletin 2020/01**

(73) Proprietor: **Fujitsu Limited**
**Kawasaki-shi, Kanagawa 211-8588 (JP)**

(72) Inventors:
- **KAMAKARI, Ryota**
  **Kawasaki-shi**
  **Kanagawa 211-8588 (JP)**
- **HOTTA, Shinji**
  **Kawasaki-shi**
  **Kanagawa 211-8588 (JP)**

(74) Representative: **Haseltine Lake Kempner LLP**
**Cheapside House**
**138 Cheapside**
**London EC2V 6BJ (GB)**

(56) References cited:
**WO-A1-2016/006479**
**JP-A- 2002 291 723**
**JP-A- 2006 326 174**
**JP-A- 2006 326 174**
**US-A1- 2004 015 103**
**US-A1- 2014 330 172**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

TECHNICAL FIELD

The present invention relates to a motion detecting system, a motion detecting device, and a motion detecting method.

BACKGROUND ART

In the recent medical field and the like, it is required to objectively grasp a patient's state in order to provide fulfilling medical treatment to each patient along with increase in elderly patients. Additionally, in recent years, it is known that there is a possibility that body abnormality that has been hardly found only from a walking motion can be discovered because motions of standing up and sitting down are complex exercises that apply more loads to knees and a lower back than walking does and require a person to keep balance on one's own.

For this reason, conventionally, there is a known technology in which motions of a patient are monitored by using a sensor and the motions of standing up and sitting down are detected from characteristics of these motions.

CITATION LIST

PATENT DOCUMENT

Patent Document 1: Japanese Laid-open Patent Publication No. 2016-158887
Patent Document 2: Japanese Laid-open Patent Publication No. 2015-177863
Patent Document 3: U.S. Patent Application Publication No. US 2014/0330172 A1, disclosing a system for identifying sit-to-stand and stand-to-sit phases of a patient.

SUMMARY OF INVENTION

TECHNICAL PROBLEM

However, since motions of standing up and sitting down are detected only from tilting of an upper body in the conventional art, there may be a case where motions similar to the motions of standing up and sitting down, such as picking up an object or looking into something, are erroneously detected as the motion of standing up or sitting down.

The disclosed technology is directed to providing a motion detecting system, a motion detecting device, and a motion detecting method capable of improving accuracy of detecting the motions of standing up and sitting down.

SOLUTION TO PROBLEM

A system according to claim 1, which defines the present invention.

According to an aspect of the embodiments, a motion detecting system includes: a section candidate extraction processing unit that extracts, as a candidate section, a section in which a standing-up motion or a sitting-down motion of a subject to be a target of motion detection is detected from first sensor information that indicates a motion of an upper body of the subject; and an identification unit that identifies, from second sensor information that indicates a motion of a lower body of the subject, a section in which the motion of the lower body cooperatively moved with the motion of the upper body is detected in the candidate section.

Each of the units can be: a program that causes a computer to execute a procedure to implement each of the units and make each of the units function as processing; and a computer readable recording medium storing the program.

ADVANTAGEOUS EFFECTS OF INVENTION

It is possible to improve the accuracy of detecting the motions of standing up and sitting down.

BRIEF DESCRIPTION OF DRAWINGS

FIG. 1 is a diagram to describe an outline of a motion detecting system of a first embodiment.
FIG. 2 is a diagram to describe correspondence between an axial direction of each of sensors and a direction with respect to a subject.
FIG. 3 is a diagram illustrating an exemplary system configuration of a motion detecting system of the first embodiment.
FIG. 4 is a diagram to describe a method of detecting a characteristic 1.
FIG. 5 is a diagram to describe a method of detecting a characteristic 2.
FIG. 6 is a diagram to describe a method of detecting a characteristic 3.
FIG. 7 is a diagram to describe a method of detecting a characteristic 4.
FIG. 8 is a diagram to describe a method of detecting a characteristic 5.
FIG. 9 is a diagram to describe a method of detecting a characteristic 6.
FIG. 10 is a diagram to describe a method of detecting a characteristic 7.
FIG. 11 is a diagram to describe a method of detecting a characteristic 8.
FIG. 12 is a diagram illustrating an exemplary hardware configuration of a motion detecting device.
FIG. 13 is a diagram to describe functions of a standing-up/sitting-down section identification unit of the first embodiment.
FIG. 14 is a flowchart to describe operation of the standing-up/sitting-down section identification unit of the first embodiment.
FIG. 15 is a flowchart to describe operation of a section candidate extraction processing unit of the first embodiment.
FIG. 16 is a diagram to describe processing of the section candidate extraction processing unit of the first embodiment.
FIG. 17 is a flowchart to describe section identification processing by a section identification processing unit of the first embodiment.
FIG. 18 is a diagram to describe the processing of the section identification processing unit of the first embodiment.
FIG. 19 is a diagram illustrating exemplary identification results by a section output unit of the first embodiment.
FIG. 20 is a flowchart to describe operation of the section identification processing unit of a second embodiment.
FIG. 21 is a diagram illustrating exemplary identification results by a section output unit of the second embodiment.
FIG. 22 is a diagram to describe functions of a standing-up/sitting-down section identification unit of a third embodiment.
FIG. 23 is a diagram to describe a previous section and a subsequent section.
FIG. 24 is a first diagram to describe determination by a previous section motion determination unit.
FIG. 25 is a second diagram to describe the determination by the previous section motion determination unit.
FIG. 26 is a flowchart to describe operation of the standing-up/sitting-down section identification unit of the third embodiment.
FIG. 27 is a flowchart to describe processing of the previous section motion determination unit and a subsequent section motion determination unit of the third embodiment.
FIG. 28 is a diagram to describe section identification by a section identification unit of the third embodiment.
FIG. 29 is a diagram illustrating exemplary identification results by a section output unit of the third embodiment.
FIG. 30 is a diagram illustrating an exemplary system configuration of a motion detecting system of a fourth embodiment.
FIG. 31 is a diagram illustrating an exemplary system configuration of a motion detecting system of a fifth embodiment.

DESCRIPTION OF EMBODIMENTS

(First Embodiment)

A first embodiment will be described below with reference to the drawings. FIG. 1 is a diagram to describe an outline of a motion detecting system of the first embodiment.

In the motion detecting system of the present embodiment, a motion detecting device 100 acquires pieces of sensor information 210 and 310 respectively from a sensor 200 attached to an upper body of a subject P to be a motion detecting target and a sensor 300 attached to a lower body of the subject P. Then, the motion detecting device 100 detects a motion of standing up and a motion of sitting down of the subject P based on the sensor information 210 and the sensor information 310.

In the present embodiment, the motion of standing up represents a motion in order that the subject P may become a standing state St that is a state of having stood up from a sitting state Se that is a state of a sitting posture as illustrated in FIG. 1. Additionally, in the present embodiment, the motion of sitting down represents a motion in order that the subject P may become the sitting state Se from the standing state St. In the following description, the motion of standing up will be referred to as a standing-up motion, and the motion of sitting down will be referred to as a sitting-down motion.

In the present embodiment, the sensor 200 and the sensor 300 are attached to the upper body and lower body of the subject P respectively, and the standing-up motion and the sitting-down motion are detected by using cooperative movement of a motion of the upper body and a motion of the lower body.

In the present embodiment, the upper body represents an upper part of a body of the subject P, and the lower body represents a lower part of the body of the subject P. Note that a lower back is a region connecting the upper body and the lower body.

The sensor 200 of the present embodiment is, for example, an inertial sensor such as a gyro sensor. The sensor 200 is to be attached to the upper body of the subject P, but is attached to the proximity of the lower back in order to acquire the sensor information 210 indicating a motion of the entire upper body. Note that the sensor 200 may also be attached to a head, a chest, or the like. The sensor 200 may also be mounted on a terminal device such as a smartphone held by the subject P, for example.

The sensor 300 of the present embodiment may be, for example: an inertial sensor such as a gyro sensor; an atmospheric pressure sensor; or the like. In the present embodiment, in a case where the sensor 300 is the gyro sensor, the sensor is to be attached to the lower body of the subject P, but it is preferable to attach the sensor in each of right and left ankles in order to acquire sensor information 310 indicating motions of both lower limbs of the lower body. Additionally, in a case where the sensor 300 is the atmospheric pressure sensor, the sensor 300 is attached to the proximity of the lower back, a head, a chest, or the like in order to acquire the sensor information 310 indicating motions of the lower body.

The motion detecting device 100 of the present embodiment may be, for example, a computer or the like installed in a medical institution. In the present embodiment, for example, when a patient who has been hospitalized in the medical institution leaves the medical institution or the like, the patient may be made to attach the sensors 200 and 300, and the patient may be set as a subject P. Then, for example, when the subject P returns to the medical institution for follow-up visit or the like, the motion detecting device 100 may acquire, from the respective sensors 200 and 300, the pieces of the pieces of sensor information 210 and 310 stored in the sensors 200 and 300.

In the present embodiment, with use of the motion detecting system as described above, motions of a patient like an elderly person having a motor function needed to be carefully observed can be grasped by a health care provider or the like not only based on a subjective view of the patient but also based on an objective view. Furthermore, in the present embodiment, since the standing-up motion and the sitting-down motion of the subject P can be detected, for example, it is possible to contribute to quantification of a characteristic amount indicating a characteristic of the standing-up motion and a characteristic amount indicating a characteristic of the sitting-down motion.

In a case where the characteristic amounts of the standing-up motion and the sitting-down motion can be quantified, it becomes easy to discover, for example, abnormality in the standing-up motion or the sitting-down motion even for a subject or the like regarding whom no abnormality can be found from a walking motion. In a case where such a discovery can be achieved, the motor function of the subject can be easily grasped by, for example, the health care provider and the like, and therefore, appropriate treatment can be provided by adding standing-up and sitting-down training and the like.

The motion detecting device 100 of the present embodiment may be, for example, a portable terminal or the like, such as a smart phone or a tablet terminal, and the portable terminal may be carried by the subject P, for example. In this case, the subject P who has attached the sensors 200 and 300 can acquire the pieces of sensor information 210 and 310 from the portable terminal and can objectively grasp own motions.

Next, correspondence between an axial direction of each of the sensors 200 and 300 and each of directions with respect to the subject P will be described with reference to FIG. 2. FIG. 2 is a diagram to describe the correspondence between the axial direction of each of the sensors and each of the directions with respect to the subject.

FIG. 2(A), (B), and (C) are diagrams illustrating the axial directions of the sensors 200 and 300, and FIG. 2(D) is a diagram illustrating the correspondence between the axial directions and the directions with respect to the subject P.

A plane H1 illustrated in FIG. 2(A) is a sagittal plane that divides a body into a left side and a right side in parallel to a midline of the body of the subject P. Note that the sagittal plane is preferably a midline sagittal plane that equally divides the body into the left side and the right side along the midline, but a parallel plane deviated to the left side or the right side is also a sagittal plane.

A plane H2 illustrated in FIG. 2(B) is a traverse section that is a plane perpendicular to a body axis of the subject P. A plane H3 illustrated in FIG. 2(C) is a coronal plane illustrating an arbitrary plane that divides the body of the subject P into a front side (ventral side) and a rear side (dorsal side).

As illustrated in FIG. 2(D), in the present embodiment, the plane H1 (sagittal plane) is a Y-Z plane, a Z-axis direction in the plane H1 will be referred to as a vertical direction, and a Y-axis direction will be referred to as a front-rear direction. Additionally, in the present embodiment, the plane H2 (traverse section) is an X-Y plane, and an X-axis direction in the plane H2 will be referred to as a right-left direction. Also, the plane H3 (coronal plane) is an X-Z plane.

Next, a motion detecting system of the present embodiment will be described with reference to FIG. 3. FIG. 3 is a diagram illustrating an exemplary system configuration of the motion detecting system of the first embodiment.

A motion detecting system 400 of the present embodiment includes the motion detecting device 100 and the sensors 200 and 300.

In the motion detecting system 400 of the present embodiment, the motion detecting device 100 and the sensors 200 and 300 may be connected via a network or the like. Additionally, the motion detecting device 100 and the sensors 200 and 300 may be connected by wire or the like based on a predetermined communication standard.

The motion detecting device 100 of the present embodiment includes a standing-up/sitting-down section identification unit 110. The standing-up/sitting-down section identification unit 110 of the present embodiment includes a sensor information acquisition unit 120, a section candidate extraction processing unit 130, and a section identification processing unit 140.

The sensor information acquisition unit 120 of the present embodiment acquires the sensor information 210 from the sensor 200. Additionally, the sensor information acquisition unit 120 acquires the sensor information 310 from the sensor 300. Note that the sensor information acquisition unit 120 of the present embodiment acquires information indicating types of the respective sensors from the sensor 200 and the sensor 300 respectively together with the pieces of sensor information 210 and 310.

Specifically, for example, the sensor information acquisition unit 120 acquires information indicating whether each of the pieces of sensor information 210 and 310 is information acquired by a gyro sensor or information acquired by an atmospheric pressure sensor.

The section candidate extraction processing unit 130 extracts, based on the sensor information 210, a candidate of a section in which the subject P performed the standing-up motion or the sitting-down motion. More specifically, the section candidate extraction processing unit 130 detects, from fluctuation of an acceleration rate in the Y-axis direction included in the sensor information 210, a motion in which the subject P tilts the upper body forward once and then returns to a state before tilting the upper body forward. In the following description, the motion in which the subject P tilts the upper body forward once and then returns to the state before tilting the upper body forward will be referred to as a forward tilting motion.

Then, the section candidate extraction processing unit 130 extracts a section in which the above-mentioned motion is detected as a section candidate in which the subject P performed the standing-up motion or the sitting-down motion. In the following description, the candidate of the section extracted by the section candidate extraction processing unit 130 will be referred to as a candidate section. In other words, a candidate section of the present embodiment represents a section in which the subject P performs the forward tilting motion. Details of the processing of the section candidate extraction processing unit 130 will be described later.

The section identification processing unit 140 identifies, based on the sensor information 210 and the sensor information 310, a section in which the subject P performed the standing-up motion or the sitting-down motion from the candidate section extracted by the section candidate extraction processing unit 130. Details of the processing of the section identification processing unit 140 will be described later.

The sensor 200 of the present embodiment includes a storage unit 220 in which the sensor information 210 detected by the sensor 200 is stored. The sensor 300 includes a storage unit 320 in which sensor information 310 detected by the sensor 300 is stored.

In the present embodiment, for example, the subject P makes some actions for a predetermined period in a state of attaching the sensors 200 and 300, and the pieces of sensor information 210 and 310 detected by the sensors 200 and 300 during the period may be stored in the storage units 220 and 320 respectively. Additionally, after the sensors 200 and 300 are detached from the subject P, the pieces of sensor information 210 and 310 stored in the storage units 220 and 320 may be transmitted to the motion detecting device 100.

Here, a description will be provided for identifying a section in which the standing-up motion is performed and a section in which the sitting-down motion is performed in the present embodiment.

In the present embodiment, whether the standing-up motion or the sitting-down motion is performed is identified by detecting a characteristic motion of the lower body while focusing on a fact that there is a characteristic motion of the lower body cooperatively moved with a motion of the upper body in a case of performing the standing-up motion and a case of performing the sitting-down motion respectively.

Specifically, in the present embodiment, a section in which any one of following characteristics 1 to 3 is detected is identified as a section in which the standing-up motion is performed.

Characteristic 1; both legs/feet are slightly moved before performing the forward tilting motion of the upper body.

Characteristic 2; both legs/feet stay still in a second half of the forward tilting motion of the upper body.

Characteristic 3; a state of both legs/feet is changed from a bent state to an upright state in the second half of the forward tilting motion of the upper body.

Additionally, in the present embodiment, a section in which any one of following characteristics 4 to 8 is detected is identified as a section in which the sitting-down motion is performed.

Characteristic 4; both legs/feet are slightly moved in the second half of the forward tilting motion of the upper body.

Characteristic 5: both legs/feet stay still in a first half of the forward tilting motion of the upper body.

Characteristic 6; a state of both legs/feet is changed from the upright state to the bent state in the first half of the forward tilting motion of the upper body.

Characteristic 7; there is landing impact in the lower body in the second half of the forward tilting motion of the upper body.

Characteristic 8; the lower body is rotated in the middle of the forward tilting motion of the upper body.

Note that the characteristic 3 may be detected in a case where an atmospheric pressure sensor is included in the sensor 200. Additionally, the characteristic 7 may be detected in a case where an atmospheric pressure sensor is included in the sensor 300.

The section identification processing unit 140 of the present embodiment determines whether any one of the characteristics 1 to 8 is detected in each candidate section extracted by the section candidate extraction processing unit 130. Then, the section identification processing unit 140 identifies the candidate section in which any one of the characteristics 1 to 8 is detected as a section in which a motion corresponding to the detected characteristic is performed.

In the following, a method of detecting the characteristics 1 to 8 will be described with reference to FIGS. 4 to 11.

FIG. 4 is a diagram to describe a method of detecting the characteristic 1. FIG. 4 illustrates a case where both the sensor 200 and the sensor 300 are gyro sensors.

FIG. 4(A) is a waveform diagram illustrating the sensor information 210 of the sensor 200, and FIG. 4(B) is a waveform diagram illustrating the sensor information 310 of the sensor 300. In other words, FIG. 4(A) illustrates an acceleration rate in the Y-axis direction included in the sensor information 210, and FIG. 4(B) illustrates an angular velocity in the Z-axis direction of the sensor information 310.

In FIG. 4, a section from timing Tks1 to timing Tke1 is set as a candidate section K1, and whether both legs/feet of the subject P are slightly moved before the candidate section K1 is detected. Additionally, in FIG. 4, a section Kb from the timing Tks1 to T1 seconds before is set as a section "before performing the forward tilting motion of the upper body". The timing Tks1 is the time of a start point of the candidate section K1, and the timing Tke1 is the time of an end point of the candidate section K1.

Furthermore, timing Tp1 in the candidate section K1 is the time of a peak point at which a peak of the acceleration rate indicated by the sensor information 210 appears in the candidate section K1. Details of the peak of the acceleration rate will be described later.

In the present embodiment, in the section Kb in the sensor information 310 in each of the right leg/foot and the left leg/foot illustrated in FIG. 4(B), when a standard deviation of fluctuation of a value in the Z-axis direction of the sensor information 310 is within a predetermined range, it is determined that "both legs/feet are slightly moved". The standard deviation of the fluctuation of the value in the Z-axis direction of the sensor information 310 is calculated by Expression (1) below. Note that th1 and th2 in Expression (1) are threshold values defining the predetermined range. Additionally, in Expression (1), $X_i$ represents a value of the sensor information 310 at an i-th time point, X-bar represents a sample average of the values of the sensor information 310, and N represents the number of samples of the values of the sensor information 310.

[Expression 1]

$$th1 < \sqrt{\frac{1}{N}\sum_{i=1}^{N}(x_i - \bar{x})^2} < th2 \quad \text{... Expression (1)}$$

That is, in the present embodiment, the characteristic 1 is detected in a case where the standard deviation of the fluctuation of the value in the Z-axis direction of the sensor information 310 is within the predetermined range in the section from the time of the start point of the candidate section to predetermined seconds before. Note that the predetermined range may be obtained in advance by experiments or the like.

FIG. 5 is a diagram to describe a method of detecting the characteristic 2. FIG. 5 illustrates a case where both the sensor 200 and the sensor 300 are gyro sensors.

FIG. 5(A) illustrates the acceleration rate in the Y-axis direction included in the sensor information 210, and FIG. 5(B) illustrates the angular velocity in the Z-axis direction of the sensor information 310.

In FIG. 5, in a section Kpa from the timing Tp1 to T2 seconds later in the candidate section K1, when the standard deviation of the fluctuation of the value in the Z-axis direction of the sensor information 310 in each of the right leg/foot and the left leg/foot illustrated in FIG. 5(B) is a predetermined value or less, it is determined that "both legs/feet stay still". Since the section Kp is a section later than the timing Tp1 that is the time of the peak point, the section Kp is a section in the second half of the forward tilting motion.

That is, in the present embodiment, the characteristic 2 is detected in a case where the standard deviation of the fluctuation of the value in the Z-axis direction of the sensor information 310 is the predetermined value or less during predetermined seconds from the time of the peak point in the candidate section. Note that the predetermined value may be obtained in advance by experiments or the like.

FIG. 6 is a diagram to describe a method of detecting the characteristic 3. FIG. 6 illustrates a case where the sensor 200 is a gyro sensor and the sensor 300 is an atmospheric pressure sensor. FIG. 6(A) illustrates the acceleration rate in the Y-axis direction included in the sensor information 210, and FIG. 6(B) illustrates an atmospheric pressure indicated by the sensor information 310.

In FIG. 6, a section from the timing Tp1 that is the time of the peak point of the candidate section K1 to the timing Tke1 that is the time when the candidate section K1 ends is set as a section Kpe, and whether fluctuation of the atmospheric pressure in the section Kpe of FIG. 6(B) is a predetermined threshold value or more is determined. Since the section Kpe is a section later than the timing Tp1 that is the time of the peak point, the section Kpe is a section in the second half of the forward tilting motion.

Then, in the present embodiment, in a case where the fluctuation of the atmospheric pressure is the predetermined threshold value or more, it is determined that the state of both legs/feet is changed from "the bent state to the upright state" because a height of the upper body is increased.

That is, in the present embodiment, the characteristic 3 is detected in the case where the fluctuation of the atmospheric pressure detected by the sensor 300 is the threshold value or more during the section from the time of the peak point to the time of the end point of the candidate section. Note that the predetermined threshold value may be obtained in advance by experiments or the like.

FIG. 7 is a diagram to describe a method of detecting the characteristic 4. FIG. 7 illustrates a case where both of the sensors 200 and 300 are gyro sensors. FIG. 7(A) illustrates the acceleration rate in the Y-axis direction included in the sensor information 210, and FIG. 7(B) illustrates the angular velocity in the Z-axis direction of the sensor information 310.

In FIG. 7, in a section Kpa2 from the timing Tp2 to T4 seconds later in a candidate section K2, when the standard deviation of the fluctuation of the value in the Z-axis direction of the sensor information 310 in each of the right leg/foot and the left leg/foot illustrated in FIG. 7(B) is within the predetermined range, it is determined that "both legs/feet are slightly moved". Since the section Kpa2 is a section later than the timing Tp2 that is the time of the peak point, this section Kpa2 is a section in the second half of the forward tilting motion. Note that T2 seconds = T4 seconds may be possible.

Note that, in FIG. 7, a period from the timing Tp2 to timing Tke2 is longer than T4 seconds.

That is, in the present embodiment, the characteristic 4 is detected in a case where the standard deviation of the fluctuation of the value in the Z-axis direction of the sensor information 310 is within the predetermined range during a period from the time of the peak point of the candidate section to the predetermined seconds later.

FIG. 8 is a diagram to describe a method of detecting the characteristic 5. FIG. 8 illustrates a case where both of the sensors 200 and 300 are gyro sensors. FIG. 8(A) illustrates the acceleration rate in the Y-axis direction included in the sensor information 210, and FIG. 8(B) illustrates the angular velocity in the Z-axis direction of the sensor information 310.

In FIG. 8, in a section Kpb2 from the timing Tp2 to T5 seconds before in the candidate section K2, when the standard deviation of the fluctuation of the value in the Z-axis direction of the sensor information 310 in each of the right leg/foot and the left leg/foot illustrated in FIG. 8(B) is a predetermined value or less, it is determined that "both legs/feet stay still". Since the section Kpb2 is the section before the timing Tp2 that is the time of the peak point, the section Kpb2 is a section in the first half of the forward tilting motion.

Note that, in FIG. 8, a period from the timing Tks2 to the timing Tp2 is longer than T5 seconds.

That is, in the present embodiment, the characteristic 5 is detected in the case where the standard deviation of the fluctuation of the value in the Z-axis direction of the sensor information 310 is the predetermined value or less during a period from the time of the peak point of the candidate section to the predetermined seconds before.

FIG. 9 is a diagram to describe a method of detecting the characteristic 6. FIG. 9 illustrates a case where the sensor 200 is a gyro sensor and the sensor 300 is an atmospheric pressure sensor. FIG. 9(A) illustrates the acceleration rate in the Y-axis direction included in the sensor information 210, and FIG. 9(B) illustrates the atmospheric pressure indicated by the sensor information 310.

In FIG. 9, a section from the timing Tks2 that is the time of start of the candidate section K2 to the timing Tp2 that is the time of the peak point of the candidate section K2 is defined as a section Ksp, and whether fluctuation of the atmospheric pressure in the section Ksp of FIG. 9(B) is a predetermined threshold value or more is determined. Then, in the present embodiment, in a case where the fluctuation of the atmospheric pressure is the predetermined threshold value or more, it is determined that the state of both legs/feet is changed from "the upright state to the bent state" because the height of the upper body is increased. Since the section Ksp is the section before the timing Tp2 that is the time of the peak point, the section Ksp is a section in the first half of the forward tilting motion.

That is, in the present embodiment, the characteristic 6 is detected in the case where the fluctuation of the atmospheric pressure detected by the sensor 300 is the threshold value or more during a period from the time of the start point of the candidate section to the time of the peak point.

FIG. 10 is a diagram to describe a method of detecting the characteristic 7. FIG. 10 illustrates a case where both of the sensors 200 and 300 are gyro sensors. FIG. 10(A) illustrates the acceleration rate in the Y-axis direction included in the sensor information 210, and FIG. 10(B) illustrates the acceleration rate in the Z-axis direction included in the sensor information 310.

In FIG. 10, in a section Kpe2 from the timing Tp2 that is the time of the peak point of the candidate section K2 to the timing Tke2 that is the end time of the candidate section K2, a peak of the acceleration rate in the Z-axis direction illustrated and included in FIG. 10(B) is detected. Then, in the present embodiment, when amplitude at the peak of the acceleration rate in the Z-axis direction included in the sensor information 310 becomes a predetermined amplitude threshold value or more, it is determined that there is landing impact in the lower body. Since the section Kpe2 is the section later than the timing Tp2 that is the time of the peak point, the section Kpe2 is a section in the second half of the forward tilting motion.

That is, in the present embodiment, in the section from the time of the peak point to the time of the end point of the candidate section, the characteristic 7 is detected in a case where the peak of the acceleration rate in the Z-axis direction of the sensor 300 is the amplitude threshold value or more

FIG. 11 is a diagram to describe a method of detecting the characteristic 8. FIG. 11 illustrates a case where both of the sensors 200 and 300 are gyro sensors. FIG. 11(A) illustrates the acceleration rate in the Y-axis direction included in the sensor information 210, and FIG. 11(B) illustrates the angular velocity in the Y-axis direction included in the sensor information 310.

In FIG. 11, a rotation angle R is calculated by integrating the sensor information 310 of FIG. 11(B) in a section Kpp from timing Tpb that is T6 seconds before the timing Tp2 to timing Tpa that is T6 seconds later from the timing Tp2, and the timing Tp2 is the time of the peak point of the candidate section K2. Then, in the present embodiment, when the rotation angle R in the Y-axis direction included in the sensor information 310 in the section Kpp is a predetermined angle threshold value or more, it is determined that the lower body is rotated with respect to the X-Y plane by a predetermined rotation threshold value or more in order to perform the sitting-down motion.

Since the section Kpp is the section centering the timing Tp2 that is the time of the peak point, the section Kpp is a middle section of the forward tilting motion.

Note that the rotation angle R is calculated by Expression (2) below. In Expression (2), $y_i$ represents a value of the angular velocity in the Y-axis direction included in the sensor information 310 at the i-th time point.

[Expression 2]

$$R = \left| \sum_{i=1}^{T} y_i \right| \quad \text{... Expression (2)}$$

That is, in the present embodiment, the characteristic 8 is detected when the rotation angle of the lower body with respect to the X-Y plane becomes the rotation threshold value or more in the section from the time of the peak point of the candidate section to the predetermined seconds before.

Thus, the section identification processing unit 140 of the present embodiment identifies a section in which the standing-up motion or the sitting-down motion is performed from a candidate section based on cooperative movement of the motion of the lower body relative to the motion of the upper body.

Next, the motion detecting device 100 of the present embodiment will be described with reference to FIG. 12. FIG. 12 is a diagram illustrating an exemplary hardware configuration of the motion detecting device.

The motion detecting device 100 of the present embodiment is an information processing device including an input device 11, an output device 12, a drive device 13, an auxiliary storage device 14, a memory device 15, an arithmetic processing device 16, and an interface device 17 which are mutually connected by a bus B.

The input device 11 is used to input various kinds of information and is implemented by, for example, a keyboard, a mouse, and the like. The output device 12 is used to output various kinds of information, and is implemented by, for example, a display or the like. The interface device 17 includes a modem, a LAN card, and the like, and is used for connection to a network.

A motion detecting program is at least a part of various kinds of programs controlling the motion detecting device 100. The motion detecting program is provided by, for example, distributing a recording medium 18, being download from the network, and the like. As the recording medium 18 recording the motion detecting program, it is possible to use various types of recording media such as: recording media that optically, electrically, or magnetically records information, like a CD-ROM, a flexible disk, or a magneto-optical disc; a semiconductor memory that electrically records information, like a ROM or a flash memory; and the like.

Additionally, the motion detecting program is installed in the auxiliary storage device 14 from the recording medium 18 via the drive device 13 when the recording medium 18 recording the motion detecting program is set in the drive device 13. The motion detecting program downloaded from the network is installed in the auxiliary storage device 14 via the interface device 17.

The auxiliary storage device 14 stores the installed motion detecting program and also stores necessary files, data, and the like. The memory device 15 reads out and stores the motion detecting program from the auxiliary storage device 14 at the time of staring the computer. Additionally, the arithmetic processing device 16 implements various kinds of processing as described later in accordance with the motion detecting program stored in the memory device 15.

Next, functions of the standing-up/sitting-down section identification unit 110 of the motion detecting device 100 will be described with reference to FIG. 13. FIG. 13 is a diagram to describe the functions of the standing-up/sitting-down section identification unit of the first embodiment.

First, the section candidate extraction processing unit 130 included in the standing-up/sitting-down section identification unit 110 of the present embodiment will be described.

The section candidate extraction processing unit 130 of the present embodiment includes an acceleration rate acquisition unit 131, a filter processing unit 132, a peak detection unit 133, and a section candidate extraction unit 134.

The acceleration rate acquisition unit 131 of the present embodiment acquires the sensor information 210 from the sensor 200, and acquires acceleration rate information in the Y-axis direction (front-rear direction) from the sensor information 210.

The filter processing unit 132 performs, for the acceleration rate information acquired by the acceleration rate acquisition unit 131, processing to shape a waveform by applying a low-pass filter. Note that the acceleration rates in the Y-axis direction included in the sensor information 210 illustrated in FIGS. 4 to 11 described above correspond to the acceleration rate information after the filter processing by the filter processing unit 132.

The peak detection unit 133 detects a peak in the acceleration rate information subjected to the filter processing. At this time, the peak detection unit 133 also detects the time of the start point and the time of the end point of the peak.

The section candidate extraction unit 134 determines whether a value of amplitude at the peak detected by the peak detection unit 133 is in a negative direction and also determines whether an absolute value is a threshold value or more. In other words, the section candidate extraction unit 134 determines whether the subject P performs the forward tilting motion of the upper body and also determines whether the absolute value of the amplitude at the peak is the threshold value or more.

Then, the section candidate extraction unit 134 extracts, as a candidate section, a section in which the subject P performs the forward tilting motion of the upper body and the absolute value of the amplitude at the peak is the threshold value or more. At this time, the section candidate extraction unit 134 sets, as the candidate section, a section from the time of the start point of the peak detected by the peak detection unit 133 to the time of the end point of the peak.

Next, the section identification processing unit 140 will be described. The section identification processing unit 140 of the present embodiment includes a sensor type determination unit 141, a standing-up motion determination unit 142, a sitting-down motion determination unit 143, a section identification unit 144, and a section output unit 145.

The sensor type determination unit 141 determines sensor types of the sensors 200 and 300 based on information indicating sensor types acquired from the sensors 200 and 300 by the sensor information acquisition unit 120.

The standing-up motion determination unit 142 determines whether any one of the characteristics 1 to 3 is detected in a candidate section extracted by the section candidate extraction processing unit 130.

The sitting-down motion determination unit 143 determines whether any one of the characteristics 4 to 8 is detected in the candidate section extracted by the section candidate extraction processing unit 130.

The section identification unit 144 identifies, as a section in which the standing-up motion or the sitting-down motion is performed, a candidate section in which the characteristics 1 to 8 are detected by the standing-up motion determination unit 142 and the sitting-down motion determination unit 143.

The section output unit 145 outputs the section identified by the section identification unit 144 as an identification result.

Next, operation of the standing-up/sitting-down section identification unit 110 of the present embodiment will be described with reference to FIG. 14. FIG. 14 is a flowchart to describe operation of the standing-up/sitting-down section identification unit of the first embodiment.

The standing-up/sitting-down section identification unit 110 of the present embodiment causes the sensor information acquisition unit 120 to acquire the sensor information 210 and the sensor information 310 (step S1401) . Subsequently, the standing-up/sitting-down section identification unit 110 causes the section candidate extraction processing unit 130 to extract, based on the sensor information 210, a candidate section in which that the standing-up motion or the sitting-down motion seems to be performed (step S1402).

Subsequently, the standing-up/sitting-down section identification unit 110 causes the section identification processing unit 140 to identify, from the candidate section, a section in which the standing-up motion or the sitting-down motion is performed based on a motion of the lower body detected by the sensor information 310 (step S1403). Then, the standing-up/sitting-down section identification unit 110 causes the section identification processing unit 140 to output the identified section (step S1404), and the processing ends.

Note that, in FIG. 14, the processing to acquire the pieces of sensor information 210 and 310 by the sensor information acquisition unit 120 is performed consecutively with the processing of the section candidate extraction processing unit 130 and the processing of the section identification processing unit 140, but not limited thereto. The processing to acquire the pieces of sensor information 210 and 310 by the sensor information acquisition unit 120 may also be performed separately and independently from the section candidate extraction processing unit 130 and the section identification processing unit 140, for example. In this case, the section candidate extraction processing unit 130 and the section identification processing unit 140 each execute the processing by referring to the pieces of sensor information 210 and 310 stored in the motion detecting device 100.

Additionally, in FIG. 14, the output processing of an identification result by the section output unit 145 is performed consecutively with the processing of the section candidate extraction processing unit 130 and the processing of the section identification processing unit 140, but not limited thereto. The identification result by the section identification processing unit 140 may be held in the motion detecting device 100, and the section output unit 145 may output the held identification result at arbitrary timing.

Next, operation of the section candidate extraction processing unit 130 of the present embodiment will be described with reference to FIG. 15. FIG. 15 is a flowchart to describe the operation of the section candidate extraction processing unit of the first embodiment.

In the section candidate extraction processing unit 130 of the present embodiment, the acceleration rate acquisition unit 131 acquires the acceleration rate information in the Y-axis direction from the sensor information 210 acquired by the sensor information acquisition unit 120 (step S1501).

Subsequently, the section candidate extraction processing unit 130 causes the filter processing unit 132 to perform the processing of applying a low-pass filter for the acceleration rate information acquired in step S1501 (step S1502) .

Subsequently, the section candidate extraction processing unit 130 causes the peak detection unit 133 to detect a peak from the acceleration rate information after the filter processing (step S1503). At this time, the peak detection unit 133 also detects the time of the start point and the time of the end point of the peak.

Subsequently, the section candidate extraction processing unit 130 causes the section candidate extraction unit 134 to identify a peak indicating that the upper body of the subject P is tilted forward, and acquires the time of a start point and the time of an end point of the identified peak, and determines a range of the peak (step S1504).

Subsequently, the section candidate extraction processing unit 130 causes the section candidate extraction unit 134 to identify a peak at which an absolute value of amplitude at the peak identified in step S1504 is the threshold value or more (step S1505) .

Then, the section candidate extraction unit 134 extracts, as a candidate section, a section from the time of the start point to the time of the end point of the peak at which the absolute value of the amplitude is the threshold value or more (step S1506), and the processing ends.

In the following, the processing in the section candidate extraction processing unit 130 of the present embodiment will be further described with reference to FIG. 16. FIG. 16 is a diagram to describe the processing of the section candidate extraction processing unit of the first embodiment. FIG. 16(A) is a diagram illustrating exemplary acceleration rate information acquired from the sensor information 210. FIG. 16(B) is a diagram illustrating exemplary acceleration rate information after the filter processing. FIG. 16(C) is a diagram to describe peak detection.

The section candidate extraction processing unit 130 causes the acceleration rate acquisition unit 131 to acquire, from the sensor information 210, the acceleration rate information illustrated in FIG. 16(A), and causes the filter processing unit 132 to execute the filter processing.

As a result, the acceleration rate information after the filter processing comes to have a waveform indicated by a thick line L illustrated in FIG. 16(B).

The peak detection unit 133 of the present embodiment detects peaks from the thick line L. In the example of FIG. 16(C), three peaks Pab1, Pab2, and Pab3 are detected. At this time, the peak detection unit 133 detects: time Ta1 at a start point of the peak Pab1 and time Tb1 at an end point thereof; time Ta2 at a start point of the peak Pab2 and time Tb2 at an end point thereof; and time Ta3 at a start point of the peak Pab3 and an end point Time Tb3 thereof.

Here, the section candidate extraction unit 134 determines whether an absolute value H1 of amplitude at the peak Pab1, an absolute value H2 of amplitude at the peak Pab2, and an absolute value H3 of amplitude at the peak Pab3 are the threshold value or more. In the example of FIG. 16(C), since all of them are determined to be the threshold value or more, the section candidate extraction unit 134 extracts a candidate section Kab1 from the time Ta1 to the time Tb1, a candidate section Kab2 from the time Ta2 to the time Tb2, and a candidate section Kab3 from the time Ta3 to the time Tb3.

Next, the processing to identify a section by the section identification processing unit 140 of the present embodiment will be described with reference to FIG. 17. FIG. 17 is a flowchart to describe section identification processing by the section identification processing unit of the first embodiment. The processing in FIG. 17 illustrates details of step S1403 of FIG. 14.

When the section identification processing unit 140 of the present embodiment selects the candidate section extracted by the section candidate extraction processing unit 130 (step S1701), the sensor type determination unit 141 determines whether the sensor information 310 is acquired by an inertial sensor for the lower body (step S1702). In other words, the sensor type determination unit 141 determines whether the sensor 300 is a gyro sensor.

In step S1702, in a case where the sensor information is not acquired by the inertial sensor, the section identification processing unit 140 proceeds to step S1710 described later.

In step S1702, in a case where the sensor information is acquired by the inertial sensor, the section identification processing unit 140 causes the standing-up motion determination unit 142 to determine, for the selected candidate section, whether both legs/feet are slightly moved before performing the forward tilting motion of the upper body (step S1703). In other words, in the section from time of a start point of the candidate section to predetermined seconds before, the standing-up motion determination unit 142 determines whether the characteristic 1 in which fluctuation of a value in the Z-axis direction of the sensor information 310 is within the predetermined range is detected.

In a case where the characteristic 1 is detected in step S1703, the section identification unit 144 identifies the candidate section as the section in which the standing-up motion is performed (step S1704), and proceeds to step S1714 described later.

In a case where the characteristic 1 is not detected in step S1703, the standing-up motion determination unit 142 determines whether both legs/feet stay still in a second half of the forward tilting motion of the upper body in the selected candidate section (step S1705). In other words, the standing-up motion determination unit 142 determines whether the characteristic 2 in which the standard deviation of the fluctuation of the value in the Z-axis direction of the sensor information 310 is the predetermined value or less is detected during the predetermined seconds from the time of the peak point in the candidate section.

When the characteristic 2 is detected in step S1705, the section identification processing unit 140 proceeds to step S1704.

In the case where characteristic 2 is not detected in step S1705, the section identification processing unit 140 causes the sitting-down motion determination unit 143 to determine whether both legs/feet are slightly moved in the second half of the forward tilting motion of the upper body in the selected candidate section (step S1706). In other words, the sitting-down motion determination unit 143 determines whether the characteristic 4 in which the standard deviation of the fluctuation of the value in the Z-axis direction of the sensor information 310 is in the predetermined range is detected during the period from the time of the peak point of the candidate section to the predetermined seconds later.

In step S1706, in a case where the characteristic 4 is detected, the section identification unit 144 identifies this section as the section in which the sitting-down motion is performed (step S1707), and proceeds to step S1714 described later.

In a case where the characteristic 4 is not detected in step S1706, the sitting-down motion determination unit 143 determines whether both legs/feet stay still state in a first half of the forward tilting motion of the upper body in the selected candidate section (step S1708). In other words, the sitting-down motion determination unit 143 determines whether the characteristic 5 in which the standard deviation of the fluctuation in the value in the Z-axis direction of the sensor information 310 becomes the predetermined value or less is detected during the period from the time of the peak point of the candidate section to the predetermined seconds before.

In a case where the characteristic 5 is detected in step S1708, the section identification processing unit 140 proceeds to step S1707.

In a case where the characteristic 5 is not detected in step S1708, the sitting-down motion determination unit 143 determines whether the lower body is rotated in the middle of the forward tilting motion of the upper body (step S1709). In other words, the sitting-down motion determination unit 143 determines whether the characteristic 8 in which the rotation angle of the lower body with respect to the X-Y plane becomes the rotation threshold value or more is detected in the section from the time of the peak point of the candidate section to the predetermined seconds before.

In a case where the characteristic 8 is detected in step S1709, the section identification processing unit 140 proceeds to step S1707.

In a case where the characteristic 8 is not detected in step S1709, the section identification processing unit 140 proceeds to step S1714 described later.

In step S1702, in a case where the sensor information is not acquired by the inertial sensor, the section identification processing unit 140 causes the sensor type determination unit 141 to determine whether the sensor information 310 is acquired by the atmospheric pressure sensor (step S1710). In other words, the sensor type determination unit 141 determines whether the sensor 300 is the atmospheric pressure sensor.

In step S1710, in a case where the information is not acquired by the atmospheric pressure sensor, the section identification processing unit 140 proceeds to step S1713 described later.

In step S1710, in a case where the information is acquired by the atmospheric pressure sensor, the section identification processing unit 140 causes the standing-up motion determination unit 142 to determine whether the state of both legs/feet is changed from the bent state to the upright state in the second half of the forward tilting motion of the upper body in the selected candidate section (step S1711). In other words, the standing-up motion determination unit 142 determines whether the characteristic 3 in which the fluctuation of the atmospheric pressure detected by the sensor 300 is the threshold value or more is detected in a period from the time of the peak point to the time of the end point of the candidate section.

In a case where the characteristic 3 is detected in step S1711, the section identification processing unit 140 proceeds to step S1704.

In a case where the characteristic 3 is not detected in step S1711, the section identification processing unit 140 causes the sitting-down motion determination unit 143 to determine whether the state of both legs/feet is changed from the upright state to the bent state in the first half of the forward tilting motion of the upper body (step S1712). In other words, the sitting-down motion determination unit 143 determines whether the characteristic 6 in which the fluctuation of the atmospheric pressure detected by the sensor 300 becomes the threshold value or more is detected during a period from the time of the start point of the candidate section to the time of the peak point.

In a case where the characteristic 6 is detected in step S1712, the section identification processing unit 140 proceeds to step S1707, and in a case where the characteristic 6 is not detected in step S1712, the section identification processing unit 140 proceeds to step S1714 described later.

In step S1710, in a case where the sensor information is not acquired by the atmospheric pressure sensor, the section identification processing unit 140 causes the sitting-down motion determination unit 143 to determine whether there is any landing impact in the lower body in the second half of the forward tilting motion of the upper body (step S1713). In other words, the sitting-down motion determination unit 143 determines whether the sensor 300 detects the characteristic 7 in which the peak of the acceleration rate in the Z-axis direction becomes the amplitude threshold value or more in the section from the time of the peak point and the time of the end point of the candidate section.

In a case where the characteristic 7 is detected in step S1713, the section identification processing unit 140 proceeds to step S1707.

In a case where the characteristic 7 is not detected in step S1713, the section identification processing unit 140 causes the section candidate extraction processing unit 130 to determine whether the processing from step S1702 to step S1713 is executed for all of candidate sections extracted by the section candidate extraction processing unit 130 (step S1714).

In a case where the processing is not executed for all of the candidate sections in step S1714, the section identification processing unit 140 selects a next candidate section (step S1715), and returns to step S1702. In a case where the processing is executed for all of the candidate sections in step S1714, the processing ends. Note that a result identified by the processing in FIG. 17 is output by the section output unit 145.

Thus, in the present embodiment, in the case where the sensor 300 is the inertial sensor, in a case where either the characteristic 1 or the characteristic 2 is detected, such a section is identified as a section in which the standing-up motion is performed, and in a case where any one of the characteristic 4, 5, and 8 is detected, such a section is identified as a section in which the sitting-down motion is performed. Therefore, in the present embodiment, when any one of the characteristics 1 to 8 is detected, such a section is identified as the section in which the standing-up motion or the sitting-down motion is performed. Therefore, the present embodiment can contribute to reduction of a processing load in the motion detecting device 100.

Next, the processing of the section identification processing unit 140 of the present embodiment will be further described with reference to FIG. 18.

FIG. 18 is a diagram to describe the processing of the section identification processing unit of the first embodiment. FIG. 18(A) illustrates exemplary sensor information 210 and exemplary sensor information 310. FIG. 18(B) is a diagram illustrating extracted section candidates. FIG. 18(C) is a diagram illustrating identified sections.

Acceleration rate information 181 illustrated in FIG. 18(A) represents an acceleration rate after the filter processing in the section candidate extraction processing unit 130. Additionally, gyro information 182 and 183 illustrated in FIG. 18(A) each represent an angular velocity in the Z-axis direction of the sensor information 310.

As illustrated in FIG. 18(B), when candidate sections K81, K82, and K83 are extracted from the acceleration rate information 181, the section identification processing unit 140 performs detection of the characteristics 1 to 8 in these candidate sections.

As illustrated in FIG. 18(C), as for the candidate section K81, since the characteristic 4 is detected in a section K84 from a peak point to an end point in a second half because both legs/feet are slightly moved, the candidate section K81 is identified as a section in which the sitting-down motion is performed.

Additionally, as for the candidate section K82, since the characteristic 1 is detected in a section K85 before the candidate section K82 because both legs/feet are slightly moved, the candidate section K82 is identified as a section in which the standing-up motion is performed.

Furthermore, the characteristics 1 to 8 are not detected in the candidate section K83. Therefore, the candidate section K83 is not identified as a section in which the standing-up motion or the sitting-down motion is performed.

Thus, in the present embodiment, a section in which the standing-up motion or the sitting-down motion is performed is identified from a candidate section extracted in accordance with the motion of the upper body based on a unique motion of the lower body cooperatively moved with the motion of the upper body. Therefore, according to the present embodiment, it is possible to improve accuracy of detecting the motions of standing up and sitting down.

Next, output of an identification result by the section output unit 145 will be described with reference to FIG. 19. FIG. 19 is a diagram illustrating exemplary identification results by the section output unit of the first embodiment.

The section output unit 145 of the present embodiment outputs an identification result after execution of the processing to identify a section by the section identification processing unit 140.

A screen 191 illustrated in FIG. 19 is an exemplary screen displayed on the display or the like of the motion detecting device 100. Note that a screen 191 may be displayed on an external display device connected to the motion detecting device 100.

As for candidate sections K81 to K83, the screen 191 displays the example in which the candidate section K81 is identified, based on a second half section K84, as a section in which sitting-down motion is performed, and the candidate section K82 is identified, based on a section K85 before the candidate section K82, as a section in which the standing-up motion is performed.

Additionally, in the present embodiment, which one of the characteristics is detected is displayed in each section in which the standing-up motion or the sitting-down motion is performed.

The screen 191 displays a notification 192 indicating, for example, that the candidate section K81 is identified as the section in which the sitting-down motion is performed because the characteristic 4 is detected. Additionally, the face 191 displays a notification 193 indicating, for example, that the candidate section K82 is identified as the section in which the standing-up motion is performed because that the characteristic 1 is detected.

Thus, in the present embodiment, when a section in which the standing-up motion or the sitting-down motion is performed is identified, a detected characteristic is also displayed together, and therefore, for example, it is possible to present the characteristic of the motion of the subject P to a health care provider or the like.

Thus, in the present embodiment, since the identification result is displayed, it is possible to objectively present a state of the motion of the subject P to, for example, the health care provider or the like who provides care, treatment, and the like relating to a motor function of the subject P in a medical institution.

(Second Embodiment)

In the following, a second embodiment will be described with reference to the drawings. The second embodiment differs from a first embodiment in that a section identification processing unit 140 performs processing to detect each of the characteristics 1 to 8 per candidate section. Therefore, in a description of the second embodiment below, only differences from the first embodiment will be described, and those having functional configurations similar to those in the first embodiment will be denoted by reference signs similar to reference sign used in the description of the first embodiment, and a description thereof will be omitted.

FIG. 20 is a flowchart to describe operation of the section identification processing unit of the second embodiment.

When the section identification processing unit 140 of the present embodiment selects a candidate section extracted by a section candidate extraction processing unit 130 (step S2001), whether sensor information 310 is acquired by an inertial sensor for a lower body is determined by a sensor type determination unit 141 (step S2002). In other words, the sensor type determination unit 141 determines whether the sensor 300 is a gyro sensor.

In step S2002, when the sensor information is not acquired by the inertial sensor, the section identification processing unit 140 proceeds to step S2014 described later.

In step S2002, in a case where the sensor information is acquired by the inertial sensor, the section identification processing unit 140 causes a standing-up motion determination unit 142 to determine whether both legs/feet are slightly moved before a forward tilting motion of an upper body in the selected candidate section, and holds the determination result (step S2003). In other words, the standing-up motion determination unit 142 determines whether the characteristic 1 is detected.

Next, the standing-up motion determination unit 142 determines whether both legs/feet stay still in a second half of the forward tilting motion of the upper body in the selected candidate section, and holds the determination result (step S2004). In other words, the standing-up motion determination unit 142 determines whether the characteristic 2 is detected.

Next, the standing-up motion determination unit 142 determines whether either the characteristic 1 or the characteristic 2 is detected (step S2005). In step S2005, when neither characteristic 1 nor characteristic 2 is detected, the section identification processing unit 140 proceeds to step S2007 described later.

In a case where either the characteristic 1 or the characteristic 2 is detected in step S2005, the section identification unit 144 identifies this candidate section as a section in which the standing-up motion is performed (step S2006) .

Subsequently, the section identification processing unit 140 causes a sitting-down motion determination unit 143 to determine whether both legs/feet are slightly moved in the second half of the forward tilting motion of the upper body in the selected candidate section, and holds the determination result (step S2007). That is, the sitting-down motion determination unit 143 determines whether the characteristic 4 is detected.

Next, the sitting-down motion determination unit 143 determines whether both legs/feet stay still in a first half of the forward tilting motion of the upper body, and holds the determination result (step S2008). That is, the sitting-down motion determination unit 143 determines whether the characteristic 5 is detected.

Next, the sitting-down motion determination unit 143 determines whether the lower body is rotated in a middle of the forward tilting motion of the upper body, and holds the determination result (step S2009). That is, the sitting-down motion determination unit 143 determines whether the characteristic 8 is detected.

Subsequently, the sitting-down motion determination unit 143 determines whether any one of the characteristics 4, 5, and 8 is detected (step S2010). In a case where none of the characteristics is detected in step S2010, the processing proceeds to step S2021 described later.

In a case where one of these characteristics is detected in step S2010, the section identification unit 144 identifies the candidate section as a section in which a sitting-down motion is performed (step S2111) and proceeds to step S2021 described later.

In step S2002, in the case where the sensor information is not acquired by the inertial sensor, the section identification processing unit 140 causes the sensor type determination unit 141 to determine whether the sensor information 310 is acquired by an atmospheric pressure sensor (step S2012) .

In step S2012, in a case where the sensor information is not acquired by the atmospheric pressure sensor, the section identification processing unit 140 proceeds to step S2018 described later.

In step S2012, in a case where the sensor information is acquired by the atmospheric pressure sensor, the section identification processing unit 140 causes the standing-up motion determination unit 142 to determine that a state of both legs/feet is changed from a bent state to an upright state in the second half of the forward tilting motion of the upper body in the selected candidate section, and holds the result. (Step S2013). That is, the standing-up motion determination unit 142 determines whether the characteristic 3 is detected.

In a case where the characteristic 3 is not detected in step S2013, the section identification processing unit 140 proceeds to step S2015 described later.

In a case where the characteristic 3 is detected in step S2013, the standing-up motion determination unit 142 identifies this candidate section as a section in which the standing-up motion is performed (step S2014) .

Subsequently, the section identification processing unit 140 causes the sitting-down motion determination unit 143 to determine whether the state of both legs/feet is changed from the upright state to the bent state in the first half of the forward tilting motion of the upper body, and holds the result (step S2015). That is, the sitting-down motion determination unit 143 determines whether the characteristic 6 is detected.

In a case where the characteristic 6 is not detected in step S2015, the section identification processing unit 140 proceeds to step S2018 described later.

In a case where the characteristic 6 is detected in step S2015, the sitting-down motion determination unit 143 identifies this candidate section as a section in which the sitting-down motion is performed (step S2016), and proceeds to step S2018 described later.

In a case where the sensor information is not acquired by the atmospheric pressure sensor in step S2012, the section identification processing unit 140 causes the sitting-down motion determination unit 143 to determine whether there is any landing impact in the lower body in the second half of the forward tilting motion of the upper body, and holds the result (step S2017) . That is, the sitting-down motion determination unit 143 determines whether the characteristic 7 is detected.

In a case where the characteristic 7 is detected in step S2017, the sitting-down motion determination unit 143 proceeds to step S2016.

In a case where the characteristic 7 is not detected in step S2017, the section identification processing unit 140 proceeds to step S2018.

Since the processing in step S2018 and step S2019 of FIG. 20 is same as processing in step S1714 and step S1715 of FIG. 17, a description thereof will be omitted.

Next, output of an identification result by a section output unit 145 of the present embodiment will be described with reference to FIG. 21.

FIG. 21 is a diagram illustrating exemplary identification results by the section output unit of the second embodiment. The section output unit 145 of the present embodiment may output a table 211 illustrated in FIG. 21 as identification results. The table 211 may be output as data from a motion detecting device 100 or may be displayed on a display or the like of the motion detecting device 100.

In the table 211, detection results of the characteristics 1 to 8 are associated with each candidate section ID that is provided per candidate section and used to discriminate between respective candidate sections.

More specifically, in the table 211, determination results on detection/non-detection of the characteristics 1 to 3 by the standing-up motion determination unit 142, determination results on detection/non-detection of the characteristics 4 to 8 by the sitting-down motion determination unit 143, and a motion (standing-up motion or sitting-down motion) in accordance with the respective determination results are associated with each candidate section ID.

For example, in the table 211, it can be grasped that the characteristic 1 and the characteristic 2 are detected in a candidate section having a candidate section ID "1", and the candidate section is identified as a section in which the standing-up motion is performed. Additionally, in the table 211, it can be grasped that the characteristic 4 and the characteristic 5 are detected in a candidate section having a candidate section ID "2", and the candidate section is identified as a section in which the sitting-down motion is performed.

As described above, according to the present embodiment, in the case where the sensor 300 is the inertial sensor, whether each of the characteristics 1, 2, 4, 5, and 8 is detected is determined. Therefore, according to the present embodiment, it is possible to improve accuracy of determining whether a candidate section is a section in which the standing-up motion or the sitting-down motion is performed, and it is also possible to improve accuracy of detecting the motions of standing up and sitting down.

(Third Embodiment)

In the following, a third embodiment will be described with reference to the drawings. The third embodiment differs from a first embodiment in which whether a standing-up motion or a sitting-down motion is performed is determined not only by using a motion of a lower body cooperatively moved with a motion of an upper body but also by using motions of the lower body in sections before and after a candidate section. Therefore, in a description of the third embodiment below, only differences from the first embodiment will be described, and those having functional configurations similar to those in the first embodiment will be denoted by reference signs similar to reference sign used in the description of the first embodiment, and a description thereof will be omitted.

FIG. 22 is a diagram to describe functions of a standing-up/sitting-down section identification unit of the third embodiment. A standing-up/sitting-down section identification unit 110A of the present embodiment includes a sensor information acquisition unit 120, a section candidate extraction processing unit 130, and a section identification processing unit 140A.

The section identification processing unit 140A includes a sensor type determination unit 141, a standing-up motion determination unit 142, a sitting-down motion determination unit 143, a section identification unit 144A, a section output unit 145, a previous section motion determination unit 146, and a subsequent section motion determination unit 147.

The section identification unit 144A of the present embodiment identifies, from a candidate section, a section in which the standing-up motion or the sitting-down motion is performed based on: determination results by the standing-up motion determination unit 142 and the sitting-down motion determination unit 143; and determination results by the previous section motion determination unit 146 and the subsequent section motion determination unit 147.

Specifically, in a case where a determination result by the standing-up motion determination unit 142 or the sitting-down motion determination unit 143 coincides with the determination results by the previous section motion determination unit 146 and the subsequent section motion determination unit 147, the section identification unit 144A of the present embodiment identifies a candidate section as a section in which a motion corresponding to the determination result is performed.

The previous section motion determination unit 146 of the present embodiment identifies a section before the candidate section, and determines whether a subject P is in a state of standing or the subject P is in a state of sitting based on a motion of the lower body in the previous section. In the following description, a section that is located before the candidate section and to be determined by the previous section motion determination unit 146 will be referred to as a previous section. Additionally, in the following description, a state in which the subject P is standing will be referred to as a standing state, and a state in which the subject P is sitting will be referred to as a sitting state.

The subsequent section motion determination unit 147 of the present embodiment identifies a section after the candidate section, and determines whether the subject is in the standing state or the sitting state based on a motion of the lower body in this section. In the following description, the section that is located after the candidate section and to be determined by the subsequent section motion determination unit 147 will be referred to as a subsequent section.

In the following, determination by the previous section motion determination unit 146 of the present embodiment will be described with reference to FIGS. 23 to 25.

FIG. 23 is a diagram to describe a previous section and a subsequent section. In the present embodiment, a section from timing Tks1 that is the time of a start point of a candidate section K1 to D1 seconds before is set as a previous section Kd1.

Additionally, in the present embodiment, a section from timing Tke1 that is the time of an end point of the candidate section K1 to D2 seconds later is set as a subsequent section Kd2.

Next, determination on whether a state is in the standing state by the previous section motion determination unit 146 will be described with reference to FIG. 24. FIG. 24 is a first diagram to describe determination by the previous section motion determination unit.

The previous section motion determination unit 146 of the present embodiment performs, for sensor information 310, filter processing applying a low-pass filter and detects a peak in a positive direction, and the sensor information 310 is acquired from each of sensors 300 attached to left and right legs/feet.

Then, the previous section motion determination unit 146 detects a part in which an interval between a peak detected from the sensor information 310 of the left leg/foot and a peak detected from the sensor information 310 of the right leg/foot is within a predetermined range.

Note that, in the present embodiment, the sensor 300 is an inertial sensor, and the sensor information 310 indicates an angular velocity in the Y-axis direction.

In FIG. 24, a solid line 241 represents the sensor information 310 of the left leg/foot subjected to the filter processing, and a solid line 242 represents the sensor information 310 of the right leg/foot subjected to the filter processing.

In the example of FIG. 24, an interval B1 is an interval between a peak Pl1 in the positive direction in the solid line 241 and a peak Pr1 in the solid line 242. Additionally, an interval B2 is an interval between the peak Pr1 in the solid line 242 and a peak Pr2 in the solid line 241. Furthermore, an interval B3 is an interval between the peak Pr2 in the solid line 241 and a peak Pr2 in the solid line 242. Note that, in the example of FIG. 24, the intervals B1, B2, and B3 are intervals within the predetermined range.

In a case where parts in each of which an interval B between a peak Pl and a peak Pr is within the predetermined range appears in the left and right order as illustrated in FIG. 24, it can be deemed that the subject P is walking. Therefore, the previous section motion determination unit 146 of the present embodiment determines that a state of the subject P in the previous section Kd1 is the standing state.

Next, determination on whether the subject is in the sitting state by the subsequent section motion determination unit 147 will be described with reference to FIG. 25. FIG. 25 is a second diagram to describe the determination by the previous section motion determination unit.

The previous section motion determination unit 146 of the present embodiment performs, for the sensor information 310, the filter processing of applying the low-pass filter and detects a peak in the positive direction, and the sensor information 310 is acquired from each of the sensors 300 attached to the left and right legs/feet.

Then, in a case where a difference in the number of detected peaks between the sensor information 310 of the left leg/foot and the sensor information 310 of the right leg/foot is the predetermined number or more and a standard deviation of values of the sensor information 310 of both legs/feet is a predetermined value or less, the previous section motion determination unit 146 determines that the subject is in the sitting state.

In FIG. 25, a solid line 251 represents the sensor information 310 of the left leg/foot subjected to the filter processing, and a solid line 252 represents the sensor information 310 of the right leg/foot subjected to the filter processing.

In the example of FIG. 25, the number of peaks detected from the solid line 251 is nine, the number of peaks detected from the solid line 252 is two, and the difference is seven. Additionally, in the example of FIG. 25, the predetermined number is set to ten.

Furthermore, in the example of FIG. 25, assume that each of standard deviation calculated from values of respective points of the solid line 251 and standard deviation calculated from values of respective points of the solid line 251 is the predetermined value or less.

In this case, the previous section motion determination unit 146 determines that the state of the subject P in the previous section Kd1 is the sitting state.

Since a method in which the subsequent section motion determination unit 147 of the present embodiment determines the state of the subject P in the subsequent section Kd2 is similar to that of the previous section motion determination unit 146, and a description thereof will be omitted.

Next, operation of the standing-up/sitting-down section identification unit 110A of the present embodiment will be described with reference to FIG. 26. FIG. 26 is a flowchart to describe the operation of the standing-up/sitting-down section identification unit of the third embodiment.

Since processing from step S2601 to step S2603 in FIG. 26 is similar to processing from step S1401 to step S1403 in FIG. 14, a description thereof will be omitted.

Subsequent to step S2603, the standing-up/sitting-down section identification unit 110 causes the previous section motion determination unit 146 and the subsequent section motion determination unit 147 to determine, from the sensor information 310, the state of the subject P in each of a previous section and a subsequent section of a candidate section (step S2604), and proceeds to step S2605.

Since processing in step S2605 is similar to that of step S1404 in FIG. 14, a description thereof will be omitted.

Next, a description will be provided for determination on the states in the previous section and the subsequent section by the previous section motion determination unit 146 and the subsequent section motion determination unit 147 of the section identification processing unit 140A of the present embodiment with reference to FIG. 27.

FIG. 27 is a flowchart to describe processing of the previous section motion determination unit and the subsequent section motion determination unit of the third embodiment. FIG. 27 illustrates details of step S2604 of FIG. 26.

The section identification processing unit 140A of the present embodiment selects, from among sections identified in determination by the standing-up motion determination unit 142 and the sitting-down motion determination unit 143, a section to be subjected to state determination on a previous section and a subsequent section (step S2701) .

Subsequently, the section identification processing unit 140A causes the previous section motion determination unit 146 and the subsequent section motion determination unit 147 to identify the previous section and the subsequent section of the selected section (step S2702) . Subsequently, the previous section motion determination unit 146 determines, based on the sensor information 310 acquired from the sensors 300 of the both legs/feet of the subject P, whether the state of the subject P in the previous section is the sitting state, and holds the determination result (step S2703). Next, the previous section motion determination unit 146 determines whether the state of the subject P in the previous section is the standing state, and holds the determination result (step S2704).

Subsequently, the section identification processing unit 140A causes the subsequent section motion determination unit 147 to determine, based on the sensor information 310 acquired from the sensors 300 of both legs/feet of the subject P, whether the state of the subject P in the subsequent section is the sitting state, and holds the determination result (step S2705). Next, the subsequent section motion determination unit 147 determines whether the state of the subject P in the subsequent section is the standing state, and holds the determination result (step S2706) .

Subsequently, the section identification processing unit 140A causes the section identification unit 144A to identify again a section in which the standing-up motion or the sitting-down motion is performed in accordance with the determination results of the previous section motion determination unit 146 and subsequent section motion determination unit 147 (step S2707), and the processing ends.

Here, section identification of the section identification unit 144A of the present embodiment will be described with reference to FIG. 28. FIG. 28 is a diagram to describe section identification by the section identification unit of the third embodiment. FIG. 28(A) is an example illustrating sections identified based on the determination results of the standing-up motion determination unit 142 and the sitting-down motion determination unit 143. FIG. 28(B) is a diagram to describe section identification based on determination results by the previous section motion determination unit 146 and the subsequent section motion determination unit 147.

As illustrated in FIG. 28(A), the section identification unit 144A causes the standing-up motion determination unit 142 and the sitting-down motion determination unit 143 to determine, out of candidate sections K81, K82, and K83 which are candidate sections, the candidate sections K81 and K82 as sections in which the standing-up motion or the sitting-down motion is performed.

Accordingly, the section identification unit 144A identifies again, based on the determination results of the previous section motion determination unit 146 and the subsequent section motion determination unit 147, sections in which the standing-up motion or the sitting-down motion out of the candidate section K81 and the candidate section K82 is performed.

In FIG. 28 (B), a previous section K91 and a subsequent section K92 are identified as a previous section and a subsequent section of the candidate section K81.

Then, the previous section motion determination unit 146 determines whether the state of the subject P in the previous section K91 is the standing state or the sitting state. In the previous section K91, the subject P is determined to be the standing state because the state is a walking state.

Additionally. The subsequent section motion determination unit 147 determines whether the state of the subject P in the subsequent section K92 is the standing state or the sitting state. In the subsequent section K92, the subject P is determined to be the sitting state.

Here, from the determination results by the standing-up motion determination unit 142 and the sitting-down motion determination unit 143, the candidate section K81 is identified as the section in which the sitting-down motion is performed.

Therefore, in the example of FIG. 28 (B), it is grasped that the subject P is in the standing state in the previous section K91 of the candidate section K81, performs the sitting-down motion in the candidate section K81, and becomes the sitting state in the subsequent section K92 of the candidate section K81, and there is no contradiction. Therefore, the section identification unit 144A identifies the candidate section K81 as the section in which the sitting-down motion is performed.

Similarly, also in the candidate section K82, the previous section motion determination unit 146 determines the state of the subject P in the previous section K93 as the sitting state, and the subsequent section motion determination unit 147 determines the state of the subject P in the subsequent section K94 as the standing state.

Therefore, the subject P is in the sitting state in the previous section K93 of the candidate section K82, performs the standing-up motion in the candidate section K82, and becomes the standing state in the subsequent section K94. Accordingly, the section identification unit 144A identifies the candidate section K82 as the section in which the standing-up motion is performed.

Thus, the section identification unit 144A of the present embodiment identifies, from candidate sections extracted based on the forward tilting motion of the upper body, a candidate section in which the standing-up motion or the sitting-down motion is performed, based on a characteristic motion of the lower body cooperatively moved with a motion of the upper body.

Next, the section identification unit 144A identifies, from the identified candidate section, a section in which the standing-up motion or the sitting-down motion is performed, based on whether the state of the subject P in each of the previous section and the subsequent section of the identified candidate section is the standing state or the sitting state.

When a kind of motion (standing up/sitting down) in the identified candidate section differs from a kind of a state in the previous section and the kind of the motion (standing up/sitting down) in the candidate section coincides with a kind of a state in the subsequent section, the section identification unit 144A of the present embodiment determines the identified candidate section as a section in which the kind of motion is performed.

For example, in a case where a motion of the identified candidate section is determined to be the sitting-down motion, when it is determined that the state in the previous section of this candidate section is the standing state and the state in the subsequent section thereof is the sitting state, the candidate section is identified as a section in which the sitting-down motion is performed.

Thus, according to the present embodiment, the states of the subject P in the previous and subsequent sections are detected relative to the candidate section identified based on the characteristic motion of the lower body cooperatively moved with the motion of the upper body, and whether the motion in the candidate section contradicts the detected states is determined. Therefore, according to the present embodiment, it is possible to improve accuracy of detecting the motions of standing up and sitting down.

Next, output of identification results by a section output unit 145 of the present embodiment will be described with reference to FIG. 29. FIG. 29 is a diagram illustrating exemplary identification results by the section output unit of the third embodiment.

The section output unit 145 of the present embodiment may output a table 291 illustrated in FIG. 29 as identification results. The table 291 may be output as data from a motion detecting device 100 or may be displayed on a display or the like of the motion detecting device 100.

In the table 291, detection results of characteristics 1 to 8 and determination results of the motions in the previous section and the subsequent section are associated with each candidate section ID.

More specifically, in the table 291, determination results on detection/non-detection of the characteristics 1 to 3 by the standing-up motion determination unit 142 and determination results on detection/non-detection of the characteristics 4 to 8 by the sitting-down motion determination unit 143 are associated with each candidate section ID. Additionally, in the table 291, a determination result on the state of the subject P in the previous section by the previous section motion determination unit 146 and a determination result on the state of the subject P by the subsequent section motion determination unit 147 are associated with each candidate section ID. Furthermore, in the table 291, a motion (standing-up motion or sitting-down motion) in accordance with the detection results of the characteristics 1 to 8 by the section identification unit 144A and the determination results of the states in the previous section and the subsequent section are associated with each candidate section ID.

For example, in the table 291, in a candidate section having a candidate section ID "1", the characteristic 1 and the characteristic 2 are detected, a state in a previous section is the sitting state, and a state in a subsequent section is the standing state. Therefore, the candidate section having the candidate section ID "1" is identified as a section in which the standing-up motion is performed.

Additionally, in the table 291, in a candidate section having a candidate section ID "2", the characteristic 4 and the characteristic 5 are detected, a state in a previous section is the standing state, and a state in a subsequent section is the sitting state. Therefore, the candidate section having the candidate section ID "2" is identified as a section in which the sitting-down motion is performed.

Additionally, in the table 291, in a candidate section having a candidate section ID "3", the characteristic 1 is detected, a state in a previous section is the standing state, and a state in a subsequent section is the sitting state. Therefore, there is contradiction in a candidate section having the candidate section ID "3" because the candidate section is determined as a section in which the standing-up motion is performed despite a fact that a state before the candidate section is determined as the standing state.

Therefore, the candidate section having the candidate section ID "3" is not identified as the section in which the standing-up motion or the sitting-down motion is performed.

Thus, according to the present embodiment, a kind of motion in a candidate section is determined based on a motion of the lower body cooperatively moved with the upper body, and after that, states of both legs/feet in a previous section and a subsequent section are detected and whether the states of both legs/feet contradict the determination result is verified. Then, in the present embodiment, in a case it is determined that there is no contradiction, the candidate section is identified as a section in which the motion determined based on the motion of the lower body cooperatively moved with the upper body is performed.

Therefore, according to the present embodiment, accuracy of detecting the motions of standing up and sitting down is more improved than in the first embodiment.

(Fourth Embodiment)

In the following, a fourth embodiment will be described with reference to the drawing. The fourth embodiment differs from a first embodiment in that a terminal device acquires pieces of sensor information 210 and 310 and transmits the pieces of sensor information 210 and 310 from the terminal device to a motion detecting device 100. Therefore, in an invention of the fourth embodiment below, only differences from the first embodiment will be described, and those having functional configurations similar to those in the first embodiment will be denoted by reference signs similar to reference sign used in the description of the first embodiment, and a description thereof will be omitted.

FIG. 30 is a diagram illustrating an exemplary system configuration of a motion detecting system of the fourth embodiment. A motion detecting system 400A of the present embodiment includes a motion detecting device 100A, sensors 200 and 300, and a terminal device 500.

In the motion detecting system 400A of the present embodiment, the motion detecting device 100A and the terminal device 500 are connected via a network. Additionally, in the motion detecting system 400A of the present embodiment, the terminal device 500 and the sensors 200 and 300 are connected by wireless communication or the like. Note that the terminal device 500 and the sensors 200 and 300 may also be connected by wire.

The motion detecting device 100A of the present embodiment includes a standing-up/sitting-down section identification unit 110B. A motion detecting device 100B includes a section candidate extraction processing unit 130, a section identification processing unit 140, and a communication unit 150.

The communication unit 150 of the present embodiment communicates with an external device including the terminal device 500. Specifically, the communication unit 150 receives the pieces of sensor information 210 and 310 from the terminal device 500. Also, the communication unit 150 transmits, to the terminal device 500, an identification result output by a section output unit 145.

The terminal device 500 of the present embodiment may also be, for example, a smartphone, a tablet terminal, or the like, or may be a communication device including a memory device and a communication function.

The terminal device 500 of the present embodiment includes a sensor information acquisition unit 120, and acquires the pieces of sensor information 210 and 310 stored respectively in the storage unit 220 of the sensor 200 and the storage unit 320 of the sensor 300, and the sensors 200 and 300 are connected to the terminal device 500.

In the present embodiment, when the terminal device 500 acquires the pieces of sensor information 210 and 310, the terminal device 500 transmits the pieces of sensor information 210 and 310 to the motion detecting device 100A. The motion detecting device 100A causes the communication unit 150 to receive the pieces of sensor information 210 and 310 from the terminal device 500, and performs processing in the section candidate extraction processing unit 130 and the section identification processing unit 140.

Thus, in the present embodiment, since the pieces of sensor information 210 and 310 are transmitted from the terminal device 500 to the motion detecting device 100A, the present embodiment can also be applied to, for example, a case where the motion detecting device 100A is a web server or the like provided on a web.

(Fifth Embodiment)

In the following, a fifth embodiment will be described with reference to the drawing. The fifth embodiment differs from a first embodiment in that a terminal device held by a subject P is used instead of sensors 200 and 300. Therefore, in an invention of the fifth embodiment described below, only differences from the first embodiment will be described, and those having functional configurations similar to those in the first embodiment will be denoted by reference signs similar to reference sign used in the description of the first embodiment, and a description thereof will be omitted.

FIG. 31 is a diagram illustrating an exemplary system configuration of a motion detecting system of the fifth embodiment.

The present embodiment is an embodiment focusing on a point that a characteristic 7 can be detected from acceleration rate information included in sensor information 210 of the sensor 200 in a case where the sensor 200 is an inertial sensor.

A motion detecting system 400B of the present embodiment includes a motion detecting device 100 and a terminal device 500A.

The terminal device 500A is a portable terminal such as a general smartphone on which an acceleration rate sensor is mounted. The terminal device 500A of the present embodiment includes a storage unit 510. The storage unit 510 stores: the sensor information 210 acquired from the acceleration rate sensor; and acceleration rate information 310A obtained from the sensor information 210. The sensor information 210 is information indicating a motion of the upper body, and the acceleration rate information 310A is information indicating a motion of the lower body.

The terminal device 500A of the present embodiment transmits the sensor information 210 and the acceleration rate information 310A to the motion detecting device 100.

The motion detecting device 100 is to cause the section identification processing unit 140 to determine whether the characteristic 7 is detected from the acceleration rate information 310A.

Thus, according to the present embodiment, the sensor information 210 indicating the motion of the upper body can be acquired only by carrying the terminal device 500A without attaching the sensors 200 and 300 for the lower body, and a standing-up motion and a sitting-down motion can be detected by a simple configuration.

The present invention is not limited to the embodiments specifically disclosed above, and various modifications and changes can be made without departing from the scope of the claims.

REFERENCE SIGNS LIST

100, 100A motion detecting device
110, 110A standing-up/sitting-down section identification unit
120 sensor information acquisition unit
130 section candidate extraction processing unit
140, 140A section identification processing unit
142 standing-up motion determination unit
143 sitting-down motion determination unit
144 section identification unit
145 section output unit
146 previous section motion determination unit
147 subsequent section motion determination unit
200, 300 sensor
210, 310 sensor information
400, 400A motion detecting system
500, 500A terminal device

## Claims

**1.** A motion detecting system (400) comprising: a section candidate extraction processing unit (130) configured to:

acquire (S1501), from a first sensor (200) which is attached to an upper body of a subject to be a target of motion detection, first sensor information (210) that indicates acceleration rate information of a front-rear motion of the upper body of the subject;
apply (S1502) a low-pass filter for the first sensor information (210);
acquire (S1503) one or more peaks, times of start points of the one or more peaks, and times of end points of the one or more peaks from the first sensor information (210) after filter processing;
identify (S1504) a peak indicating that the upper body of the subject is tilted forward from among the one or more peaks, acquire the time of a start point and the time of an end point of the identified peak, and determine a range of the identified peak;
determine (S1505) whether an absolute value of amplitude of the identified peak is a threshold value or more; and
extract (S1506), as a candidate section, a section between the time of the start point and the time of the end point of the identified peak in which the absolute value of amplitude of the identified peak is the threshold value or more; and
a section identification processing unit unit (140) configured to identify, in the candidate section, a section in which a standing-up motion or a sitting-down motion of the subject is detected, using second sensor information (310) acquired from a second sensor (300) attached to a lower body of the subject, the second sensor information (310) indicating a motion of the lower body of the subject.

**2.** The motion detecting system (400) according to claim 1,

wherein the identification unit (140) includes a motion determination unit (142, 143) configured to determine whether a motion of the subject in the candidate section is the standing-up motion or the sitting-down motion,
wherein the motion determination unit (142, 143) determines that a motion of the subject in the candidate section is the standing-up motion when slight movement of both legs/feet is detected immediately before the candidate section as the motion of the lower body cooperatively moved with the motion of the upper body.

3. The motion detecting system according to claim 2, wherein the motion determination unit (142, 143) determines that a motion of the subject in the candidate section is the standing-up motion when a motion of both legs/feet staying still is detected in a second half of the candidate section as the motion of the lower body cooperatively moved with the motion of the upper body.

4. The motion detecting system according to claim 2 or 3, wherein the motion determination unit (142, 143) determines that a motion of the subject in the candidate section is the sitting-down motion when slight movement of both legs/feet is detected in the second half of the candidate section as the motion of the lower body cooperatively moved with the motion of the upper body.

5. The motion detecting system according to any one of claims 2 to 4, wherein the motion determination unit (142, 143) determines that a motion of the subject in the candidate section is the sitting-down motion when the motion of both legs/feet staying still is detected in a first half of the candidate section as the motion of the lower body cooperatively moved with the motion of the upper body.

6. The motion detecting system according to any one of claims 2 to 5, wherein the motion determination unit (142, 143) determines that a motion of the subject in the candidate section is the sitting-down motion when a rotational motion of the lower body is detected in middle of the candidate section as the motion of the lower body cooperatively moved with the motion of the upper body.

7. The motion detecting system according to claim 1,

wherein the identification unit (140) includes a motion determination unit (142, 143) configured to determine whether a motion of the subject in the candidate section is the standing-up motion or the sitting-down motion, wherein the motion determination unit (142, 143) determines that a motion of the subject in the candidate section is the sitting-down motion when landing impact in the lower body is detected in the second half of the candidate section as the motion of the lower body cooperatively moved with the motion of the upper body.

8. The motion detecting system according to any one of claims 2 to 7, wherein the first sensor (200) and the second sensor (300) are inertial sensors.

9. The motion detecting system according to claim 1,

wherein the identification unit (140) includes a motion determination unit (142, 143) configured to determine whether a motion of the subject in the candidate section is the standing-up motion or the sitting-down motion, wherein the motion determination unit (142, 143) determines a motion of the subject in the candidate section is the standing-up motion when a motion in which a state of both legs/feet is changed from a bent state to an upright state is detected in the second half of the candidate section as the motion of the lower body cooperatively moved with the motion of the upper body.

10. The motion detecting system according to claim 9, wherein the motion determination unit (142, 143) determines that a motion of the subject in the candidate section is the sitting-down motion when a motion in which a state of both legs/feet is changed from the upright state to the bent state is detected in the second half of the candidate section as the motion of the lower body cooperatively moved with the motion of the upper body.

11. The motion detecting system according to claim 9 or 10, wherein the first sensor (200) is an inertial sensor, and the second sensor (300) is an atmospheric pressure sensor.

12. The motion detecting system according to any one of claims 2 to 11,

wherein the identification unit (140) includes a state determination unit (146) configured to determine, from the second sensor information (310), whether a state of the subject in sections before and after the candidate section is a standing state or a sitting state,
wherein the identification unit (140) identifies the section based on a determination result by the motion determination unit (142, 143) and a determination result by the state determination unit (146).

**13.** The motion detecting system according to claim 12,
wherein in a case where a motion determined by the motion determination unit (142, 143) differs from a state in a previous section of the candidate section determined by the state determination unit (146), and a motion determined by the motion determination unit (142, 143) coincides with a state in a subsequent section of the candidate section determined by the state determination unit (146), the identification unit (140) identifies the candidate section as a section in which a motion of the lower body cooperatively moved with a motion of the upper body is detected.

**14.** The motion detecting system according to any one of clams 1 to 13,

wherein the identification unit (140) includes a section output unit (145) configured to output, as identification results, a section identified by the identification unit (140) as the section in which the motion of the lower body cooperatively moved with the motion of the upper body is detected, and the motion of the lower body cooperatively moved with the motion of the upper body detected in the section,
the section output unit (145) outputting, in an associated manner, the section identified by the identification unit (140), and the motion of the lower body cooperatively moved with the motion of the upper body detected in the section.

## Patentansprüche

**1.** Bewegungsdetektionssystem (400), umfassend:
eine Extraktionsverarbeitungseinheit (130) eines Abschnitts von Interesse, die zu Folgendem konfiguriert ist:

Erfassen (S1501), von einem ersten Sensor (200), der an einem Oberkörper einer Person angebracht ist, die ein Ziel der Bewegungsdetektion sein soll, von ersten Sensorinformationen (210), die Beschleunigungsrateninformationen einer Vorwärts-Rückwärts-Bewegung des Oberkörpers der Person angeben;
Anwenden (S1502) eines Tiefpassfilters auf die ersten Sensorinformationen (210);
Erfassen (S1503) einer oder mehrerer Spitzen, Zeiten von Startpunkten der einen oder mehreren Spitzen und Zeiten von Endpunkten der einen oder mehreren Spitzen aus den ersten Sensorinformationen (210) nach der Filterverarbeitung;
Identifizieren (S1504) einer Spitze, die angibt, dass der Oberkörper der Person nach vorne geneigt ist, aus der einen oder den mehreren Spitzen, Erfassen der Zeit eines Startpunkts und der Zeit eines Endpunkts der identifizierten Spitze und Bestimmen eines Bereichs der identifizierten Spitze;
Bestimmen (S1505), ob ein Absolutwert einer Amplitude der identifizierten Spitze ein Schwellenwert ist oder darüber liegt; und
Extrahieren (S1506) eines Abschnitts zwischen der Zeit des Startpunkts und der Zeit des Endpunkts der identifizierten Spitze, in dem der Absolutwert der Amplitude der identifizierten Spitze der Schwellenwert ist oder darüber liegt, als Abschnitt von Interesse; und
eine Abschnittsidentifizierungs-Verarbeitungseinheit (140), die dazu konfiguriert ist, in dem Abschnitt von Interesse einen Abschnitt zu identifizieren, in dem eine Aufstehbewegung oder eine Hinsetzbewegung der Person detektiert wird, unter Verwendung von zweiten Sensorinformationen (310), die von einem zweiten Sensor (300) erfasst werden, der an einem Unterkörper der Person angebracht ist, wobei die zweiten Sensorinformation (310) eine Bewegung des Unterkörpers der Person angeben.

**2.** Bewegungsdetektionssystem (400) nach Anspruch 1,

wobei die Identifizierungseinheit (140) eine Bewegungsbestimmungseinheit (142, 143) beinhaltet, die dazu konfiguriert ist, zu bestimmen, ob eine Bewegung der Person in dem Abschnitt von Interesse die Aufstehbewegung oder die Hinsetzbewegung ist,
wobei die Bewegungsbestimmungseinheit (142, 143) bestimmt, dass eine Bewegung der Person in dem Abschnitt von Interesse die Aufstehbewegung ist, wenn eine leichte Bewegung beider Beine/Füße unmittelbar vor dem Abschnitt von Interesse als die Bewegung des Unterkörpers, der zusammenwirkend mit der Bewegung des Oberkörpers bewegt wird, detektiert wird.

**3.** Bewegungsdetektionssystem nach Anspruch 2,
wobei die Bewegungsbestimmungseinheit (142, 143) bestimmt, dass eine Bewegung der Person in dem Abschnitt von Interesse die Aufstehbewegung ist, wenn eine Bewegung beider Beine/Füße als stillstehend in einer zweiten Hälfte des Abschnitts von Interesse als die Bewegung des Unterkörpers, der zusammenwirkend mit der Bewegung des Oberkörpers bewegt wird, detektiert wird.

**4.** Bewegungsdetektionssystem nach Anspruch 2 oder 3,
wobei die Bewegungsbestimmungseinheit (142, 143) bestimmt, dass eine Bewegung der Person in dem Abschnitt von Interesse die Hinsetzbewegung ist, wenn eine leichte Bewegung beider Beine/Füße in der zweiten Hälfte des Abschnitts von Interesse als die Bewegung des Unterkörpers, der zusammenwirkend mit der Bewegung des Oberkörpers bewegt wird, detektiert wird.

**5.** Bewegungsdetektionssystem nach einem der Ansprüche 2 bis 4, wobei die Bewegungsbestimmungseinheit (142, 143) bestimmt, dass eine Bewegung der Person in dem Abschnitt von Interesse die Hinsetzbewegung ist, wenn die Bewegung beider Beine/Füße als stillstehend in einer ersten Hälfte des Abschnitts von Interesse als die Bewegung des Unterkörpers, der zusammenwirkend mit der Bewegung des Oberkörpers bewegt wird, detektiert wird.

**6.** Bewegungsdetektionssystem nach einem der Ansprüche 2 bis 5, wobei die Bewegungsbestimmungseinheit (142, 143) bestimmt, dass eine Bewegung der Person in dem Abschnitt von Interesse die Hinsetzbewegung ist, wenn eine Drehbewegung des Unterkörpers in der Mitte des Abschnitts von Interesse als die Bewegung des Unterkörpers, der zusammenwirkend mit der Bewegung des Oberkörpers bewegt wird, detektiert wird.

**7.** Bewegungsdetektionssystem nach Anspruch 1,

wobei die Identifizierungseinheit (140) eine Bewegungsbestimmungseinheit (142, 143) beinhaltet, die dazu konfiguriert ist, zu bestimmen, ob eine Bewegung der Person in dem Abschnitt von Interesse die Aufstehbewegung oder die Hinsetzbewegung ist,
wobei die Bewegungsbestimmungseinheit (142, 143) bestimmt, dass eine Bewegung der Person in dem Abschnitt von Interesse die Hinsetzbewegung ist, wenn ein Landeaufprall in dem Unterkörper in der zweiten Hälfte des Abschnitts von Interesse als die Bewegung des Unterkörpers, der zusammenwirkend mit der Bewegung des Oberkörpers bewegt wird, detektiert wird.

**8.** Bewegungsdetektionssystem nach einem der Ansprüche 2 bis 7, wobei der erste Sensor (200) und der zweite Sensor (300) Trägheitssensoren sind.

**9.** Bewegungsdetektionssystem nach Anspruch 1,

wobei die Identifizierungseinheit (140) eine Bewegungsbestimmungseinheit (142, 143) beinhaltet, die dazu konfiguriert ist, zu bestimmen, ob eine Bewegung der Person in dem Abschnitt von Interesse die Aufstehbewegung oder die Hinsetzbewegung ist,
wobei die Bewegungsbestimmungseinheit (142, 143) bestimmt, dass eine Bewegung der Person in dem Abschnitt von Interesse die Aufstehbewegung ist, wenn eine Bewegung, in der sich ein Zustand beider Beine/Füße von einem abgebogenen Zustand in einen aufgerichteten Zustand ändert, in der zweiten Hälfte des Abschnitts von Interesse als die Bewegung des Unterkörpers, der zusammenwirkend mit der Bewegung des Oberkörpers bewegt wird, detektiert wird.

**10.** Bewegungsdetektionssystem nach Anspruch 9,
wobei die Bewegungsbestimmungseinheit (142, 143) bestimmt, dass eine Bewegung der Person in dem Abschnitt von Interesse die Hinsetzbewegung ist, wenn eine Bewegung, in der sich ein Zustand beider Beine/Füße von einem aufgerichteten Zustand in einen abgebogenen Zustand ändert, in der zweiten Hälfte des Abschnitts von Interesse als die Bewegung des Unterkörpers, der zusammenwirkend mit der Bewegung des Oberkörpers bewegt wird, detektiert wird.

**11.** Bewegungsdetektionssystem nach Anspruch 9 oder 10,
wobei der erste Sensor (200) ein Trägheitssensor ist und der zweite Sensor (300) ein Atmosphärendrucksensor ist.

**12.** Bewegungsdetektionssystem nach einem der Ansprüche 2 bis 11, wobei die Identifizierungseinheit (140) eine Zustandsbestimmungseinheit (146) beinhaltet, die dazu konfiguriert ist, anhand der zweiten Sensorinformationen (310) zu bestimmen, ob ein Zustand der Person in Abschnitten vor und nach dem Abschnitt von Interesse ein stehender Zustand oder ein sitzender Zustand ist, wobei die Identifizierungseinheit (140) den Abschnitt basierend auf einem Bestimmungsergebnis durch die Bewegungsbestimmungseinheit (142, 143) und einem Bestimmungsergebnis durch die Zustandsbestimmungseinheit (146) identifiziert.

**13.** Bewegungsdetektionssystem nach Anspruch 12,
wobei in einem Fall, in dem sich eine Bewegung, die durch die Bewegungsbestimmungseinheit (142, 143) bestimmt wird, von einem Zustand in einem vorherigen Abschnitt des Abschnitts von Interesse, der durch die Zustandsbestimmungseinheit (146) bestimmt wird, unterscheidet und eine Bewegung, die durch die Bewegungsbestimmungseinheit (142, 143) bestimmt wird, mit einem Zustand in einem nachfolgenden Abschnitt des Abschnitts von Interesse, der durch die Zustandsbestimmungseinheit (146) bestimmt wird, übereinstimmt, die Identifizierungseinheit (140) den Abschnitt von Interesse als einen Abschnitt identifiziert, in dem eine Bewegung des Unterkörpers, der zusammenwirkend mit einer Bewegung des Oberkörpers bewegt wird, detektiert wird.

**14.** Bewegungsdetektionssystem nach einem der Ansprüche 1 bis 13, wobei die Identifizierungseinheit (140) eine Abschnittsausgabeeinheit (145) beinhaltet, die dazu konfiguriert ist, als Identifizierungsergebnisse einen Abschnitt, der durch die Identifizierungseinheit (140) als der Abschnitt identifiziert wird, in dem sich die Bewegung des Unterkörpers, der zusammenwirkend mit der Bewegung des Oberkörpers bewegt wird, detektiert wird, und die Bewegung des Unterkörpers, der zusammenwirkend mit der in dem Abschnitt detektierten Bewegung des Oberkörpers bewegt wird, auszugeben, wobei die Abschnittsausgabeeinheit (145) den durch die Identifizierungseinheit (140) identifizierten Abschnitt und die Bewegung des Unterkörpers, der zusammenwirkend mit der in dem Abschnitt detektierten Bewegung des Oberkörpers bewegt wird, in zugeordneter Weise ausgibt.

## Revendications

**1.** Système de détection de mouvement (400) comprenant :
une unité de traitement d'extraction de candidate de section (130) configurée pour :

acquérir (S1501), à partir d'un premier capteur (200) qui est fixé au haut du corps d'un sujet devant être une cible de détection de mouvement, des premières informations de capteur (210) qui indiquent des informations de taux d'accélération d'un mouvement avant-arrière du haut du corps du sujet ;
appliquer (S1502) un filtre passe-bas pour les premières informations de capteur (210) ;
acquérir (S1503) un ou plusieurs pics, des instants de points de début des un ou plusieurs pics et des instants des points de fin des un ou plusieurs pics à partir des premières informations de capteur (210) après le traitement de filtre ;
identifier (S1504) un pic indiquant que le haut du corps du sujet est incliné vers l'avant parmi les un ou plusieurs pics, acquérir l'instant d'un point de début et l'instant d'un point de fin du pic identifié, et déterminer une plage du pic identifié ;
déterminer (S1505) si une valeur absolue d'amplitude du pic identifié est supérieure ou égale à une valeur seuil ; et
extraire (S1506), en tant que section candidate, une section entre l'instant du point de début et l'instant du point de fin du pic identifié dans lequel la valeur absolue d'amplitude du pic identifié est supérieure ou égale à la valeur seuil ; et
une unité de traitement d'identification de section (140) configurée pour identifier, dans la section candidate, une section dans laquelle un mouvement vers une position debout ou un mouvement vers une position assise du sujet est détecté, en utilisant des secondes informations de capteur (310) acquises à partir d'un second capteur (300) fixé au bas du corps du sujet, les secondes informations de capteur (310) indiquant un mouvement du bas du corps du sujet.

**2.** Système de détection de mouvement (400) selon la revendication 1,

dans lequel l'unité d'identification (140) comporte une unité de détermination de mouvement (142, 143) configurée pour déterminer si un mouvement du sujet dans la section candidate est le mouvement vers une position debout ou le mouvement vers une position assise,
dans lequel l'unité de détermination de mouvement (142, 143) détermine qu'un mouvement du sujet dans la section candidate est le mouvement vers une position debout lorsqu'un léger déplacement des deux jambes/pieds est détecté immédiatement avant la section candidate alors que le mouvement du bas du corps se déplace en coopération avec le mouvement du haut du corps.

3. Système de détection de mouvement selon la revendication 2, dans lequel l'unité de détermination de mouvement (142, 143) détermine qu'un mouvement du sujet dans la section candidate est le mouvement vers une position debout lorsqu'un mouvement des deux jambes/pieds restant immobiles est détecté dans une seconde moitié de la section candidate alors que le mouvement du bas du corps est déplacé en coopération avec le mouvement du haut du corps.

4. Système de détection de mouvement selon la revendication 2 ou 3, dans lequel l'unité de détermination de mouvement (142, 143) détermine qu'un mouvement du sujet dans la section candidate est le mouvement vers une position assise lorsqu'un léger déplacement des deux jambes/pieds est détecté dans la seconde moitié de la section candidate alors que le mouvement du bas du corps est déplacé en coopération avec le mouvement du haut du corps.

5. Système de détection de mouvement selon l'une quelconque des revendications 2 à 4, dans lequel l'unité de détermination de mouvement (142, 143) détermine qu'un mouvement du sujet dans la section candidate est le mouvement vers une position assise lorsque le mouvement des deux jambes/pieds restant immobiles est détecté dans une première moitié de la section candidate alors que mouvement du bas du corps est déplacé en coopération avec le mouvement du haut du corps.

6. Système de détection de mouvement selon l'une quelconque des revendications 2 à 5, dans lequel l'unité de détermination de mouvement (142, 143) détermine qu'un mouvement du sujet dans la section candidate est le mouvement vers une position assise lorsqu'un mouvement de rotation du bas du corps est détecté au milieu de la section candidate alors que le mouvement du bas du corps est déplacé en coopération avec le mouvement du haut du corps.

7. Système de détection de mouvement selon la revendication 1,

   dans lequel l'unité d'identification (140) comporte une unité de détermination de mouvement (142, 143) configurée pour déterminer si un mouvement du sujet dans la section candidate est le mouvement vers une position debout ou le mouvement vers une position assise,
   dans lequel l'unité de détermination de mouvement (142, 143) détermine qu'un mouvement du sujet dans la section candidate est le mouvement vers une position assise lorsque l'impact d'atterrissage dans le bas du corps est détecté dans la seconde moitié de la section candidate alors que le mouvement du bas du corps est déplacé en coopération avec le mouvement du haut du corps.

8. Système de détection de mouvement selon l'une quelconque des revendications 2 à 7, dans lequel le premier capteur (200) et le second capteur (300) sont des capteurs inertiels.

9. Système de détection de mouvement selon la revendication 1,

   dans lequel l'unité d'identification (140) comporte une unité de détermination de mouvement (142, 143) configurée pour déterminer si un mouvement du sujet dans la section candidate est le mouvement vers une position debout ou le mouvement vers une position assise,
   dans lequel l'unité de détermination de mouvement (142, 143) détermine qu'un mouvement du sujet dans la section candidate est le mouvement vers une position debout lorsqu'un mouvement dans lequel un état des deux jambes/pieds est changé d'un état fléchi à un état droit est détecté dans la seconde moitié de la section candidate alors que le mouvement du bas du corps est déplacé en coopération avec le mouvement du haut du corps.

10. Système de détection de mouvement selon la revendication 9, dans lequel l'unité de détermination de mouvement (142, 143) détermine qu'un mouvement du sujet dans la section candidate est le mouvement vers une position assise lorsqu'un mouvement dans lequel un état des deux jambes/pieds est changé de l'état droit à l'état fléchi est détecté dans la seconde moitié de la section candidate alors que le mouvement du bas du corps est déplacé en coopération avec le mouvement du haut du corps.

11. Système de détection de mouvement selon la revendication 9 ou 10, dans lequel le premier capteur (200) est un capteur inertiel, et le second capteur (300) est un capteur de pression atmosphérique.

**12.** Système de détection de mouvement selon l'une quelconque des revendications 2 à 11,

dans lequel l'unité d'identification (140) comporte une unité de détermination d'état (146) configurée pour déterminer, à partir des secondes informations de capteur (310), si un état du sujet dans des sections avant et après la section candidate est un état debout ou un état assis,
dans lequel l'unité d'identification (140) identifie la section sur la base d'un résultat de détermination par l'unité de détermination de mouvement (142, 143) et d'un résultat de détermination par l'unité de détermination d'état (146).

**13.** Système de détection de mouvement selon la revendication 12,
dans lequel dans un cas où un mouvement déterminé par l'unité de détermination de mouvement (142, 143) diffère d'un état dans une section précédente de la section candidate déterminé par l'unité de détermination d'état (146), et un mouvement déterminé par l'unité de détermination de mouvement (142, 143) coïncide avec un état dans une section suivante de la section candidate déterminé par l'unité de détermination d'état (146), l'unité d'identification (140) identifie la section candidate en tant que section dans laquelle un mouvement du bas du corps déplacé en coopération avec un mouvement du haut du corps est détecté.

**14.** Système de détection de mouvement selon l'une quelconque des revendications 1 à 13,

dans lequel l'unité d'identification (140) comporte une unité de sortie de section (145) configurée pour délivrer en sortie, en tant que résultats d'identification, une section identifiée par l'unité d'identification (140) comme étant la section dans laquelle le mouvement du bas du corps déplacé en coopération avec le mouvement du haut du corps est détecté, et le mouvement du bas du corps déplacé en coopération avec le mouvement du haut du corps détecté dans la section,
l'unité de sortie de section (145) délivre en sortie, de manière associée, la section identifiée par l'unité d'identification (140), et le mouvement du bas du corps déplacé en coopération avec le mouvement du haut du corps détecté dans la section.

FIG. 1

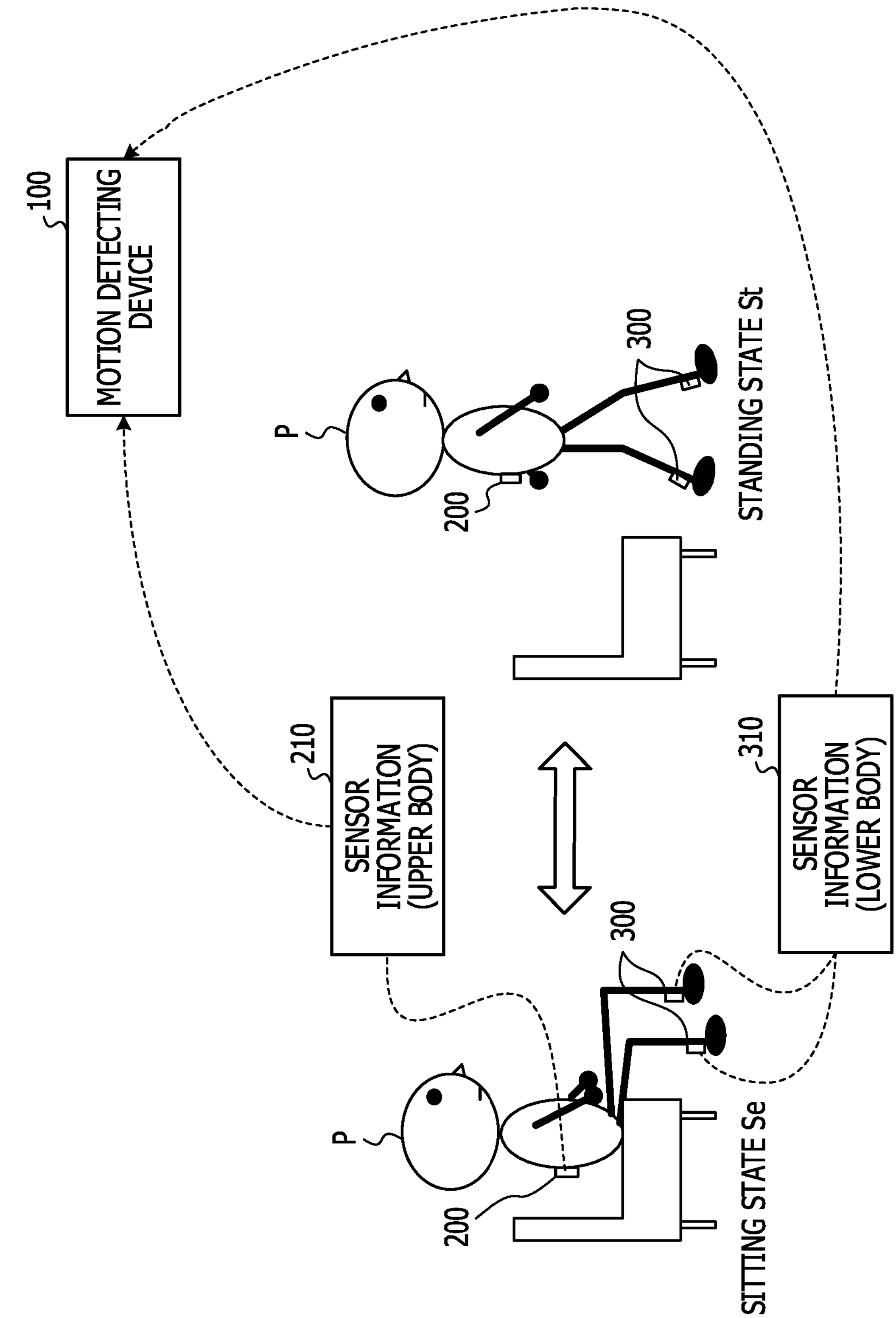
100
MOTION DETECTING DEVICE
P
200
300
STANDING STATE St
210
SENSOR INFORMATION (UPPER BODY)
310
SENSOR INFORMATION (LOWER BODY)
P
200
300
SITTING STATE Se

FIG. 2

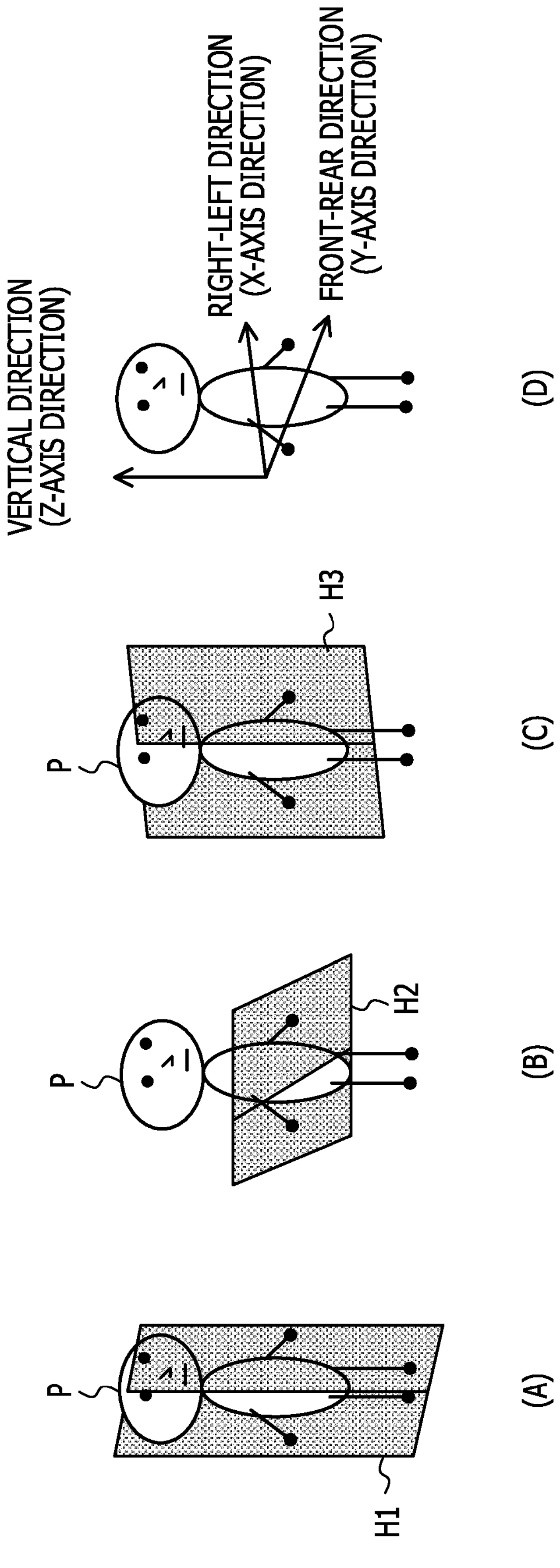

P
H1
(A)
P
H2
(B)
P
H3
(C)
VERTICAL DIRECTION
(Z-AXIS DIRECTION)
RIGHT-LEFT DIRECTION
(X-AXIS DIRECTION)
FRONT-REAR DIRECTION
(Y-AXIS DIRECTION)
(D)

FIG. 3

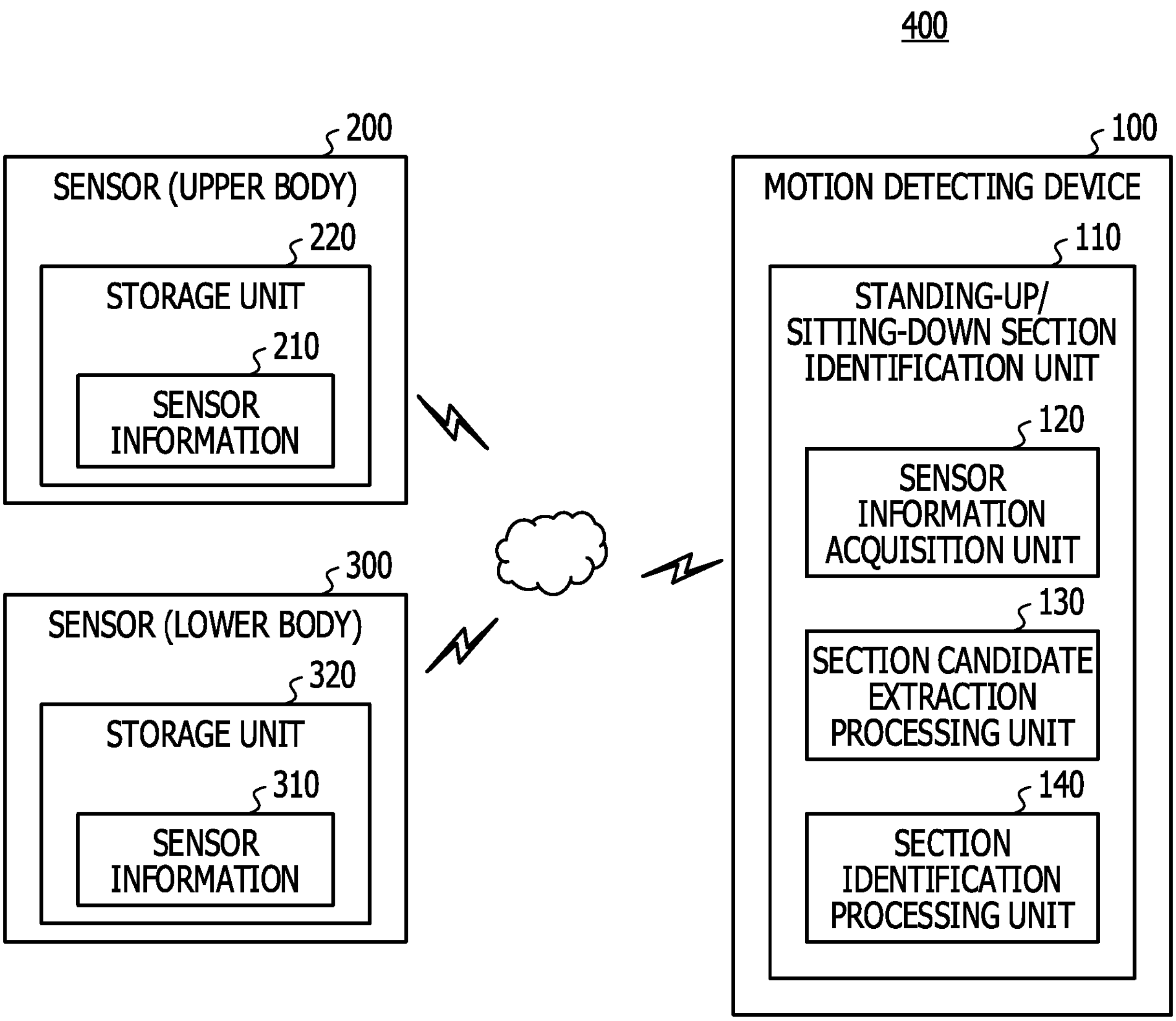
400
200
SENSOR (UPPER BODY)
220
STORAGE UNIT
210
SENSOR INFORMATION
300
SENSOR (LOWER BODY)
320
STORAGE UNIT
310
SENSOR INFORMATION
100
MOTION DETECTING DEVICE
110
STANDING-UP/ SITTING-DOWN SECTION IDENTIFICATION UNIT
120
SENSOR INFORMATION ACQUISITION UNIT
130
SECTION CANDIDATE EXTRACTION PROCESSING UNIT
140
SECTION IDENTIFICATION PROCESSING UNIT

FIG. 4

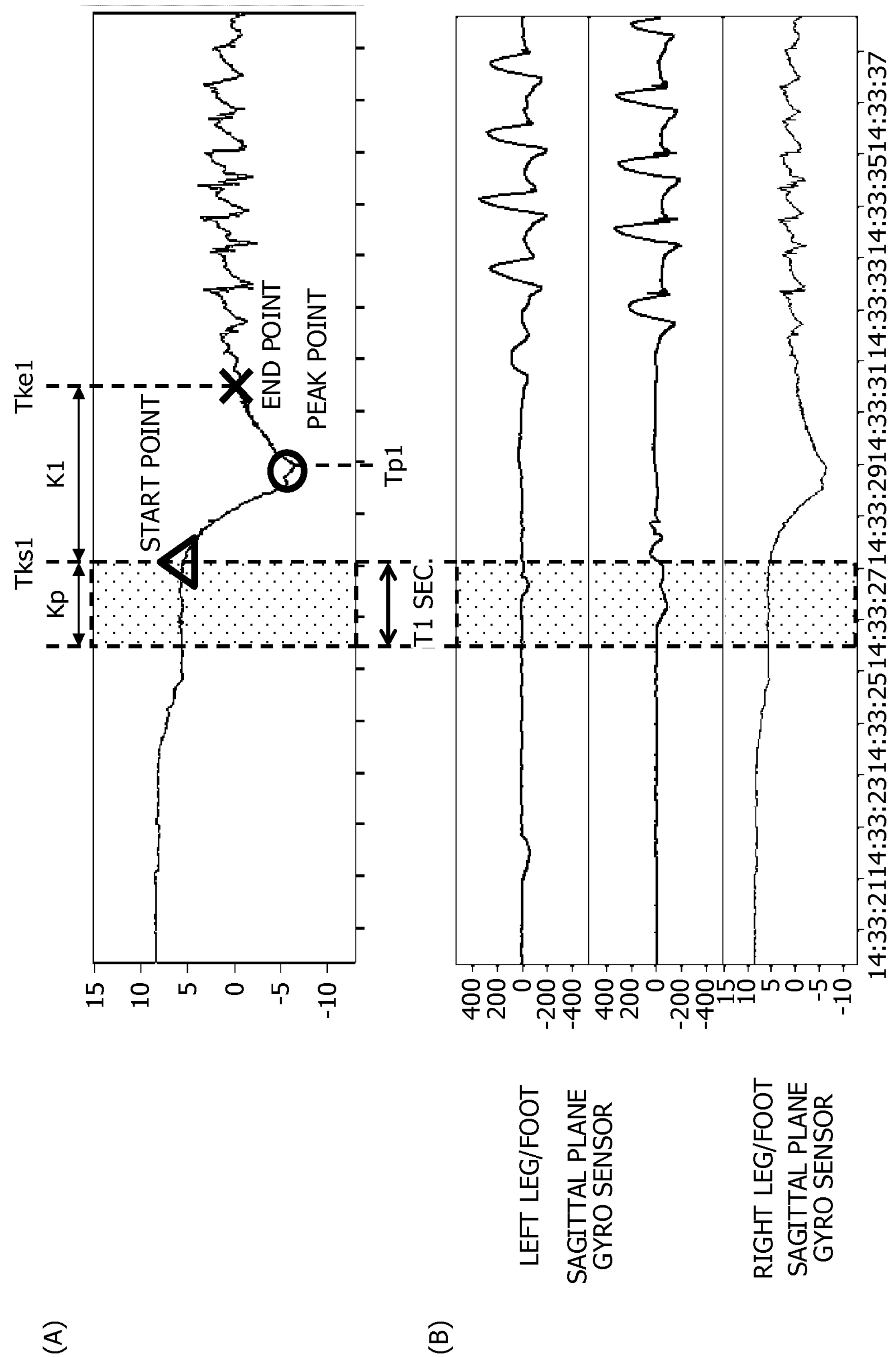
(A)
(B)
Kp
Tks1
K1
Tke1
START POINT
END POINT
PEAK POINT
Tp1
T1 SEC
15
10
5
0
-5
-10
400
200
0
-200
-400
LEFT LEG/FOOT
SAGITTAL PLANE
GYRO SENSOR
RIGHT LEG/FOOT
SAGITTAL PLANE
GYRO SENSOR
14:33:21
14:33:23
14:33:25
14:33:27
14:33:29
14:33:31
14:33:33
14:33:35
14:33:37

FIG. 5

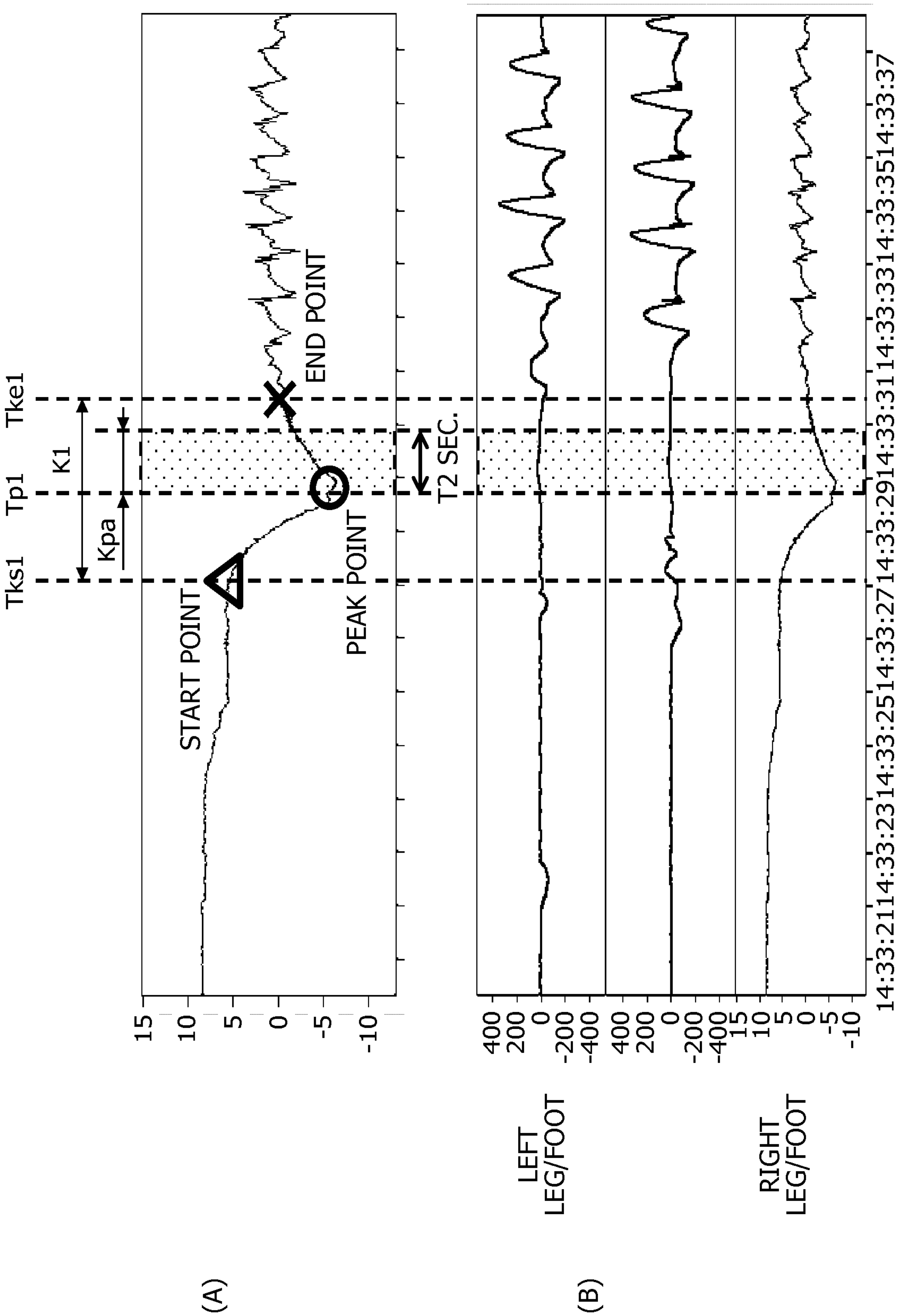

Tks1
Tp1
Tke1
K1
Kpa
START POINT
PEAK POINT
END POINT
T2 SEC.
15
10
5
0
-5
-10
400
200
0
-200
-400
LEFT LEG/FOOT
RIGHT LEG/FOOT
14:33:21
14:33:23
14:33:25
14:33:27
14:33:29
14:33:31
14:33:33
14:33:35
14:33:37
(A)
(B)

FIG. 6

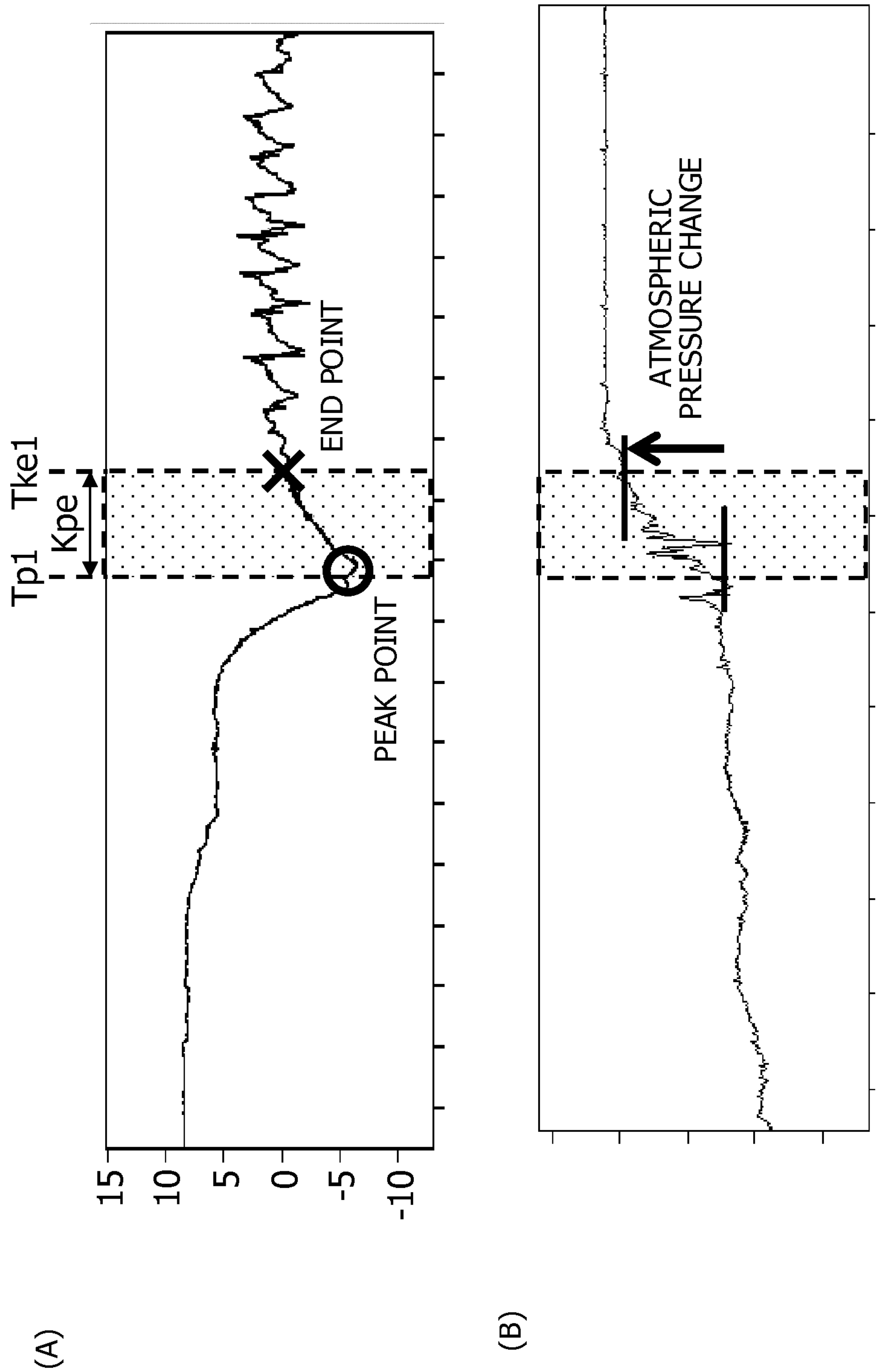
(A)
Tp1
Tke1
Kpe
END POINT
PEAK POINT
15
10
5
0
-5
-10
(B)
ATMOSPHERIC
PRESSURE CHANGE

FIG. 7

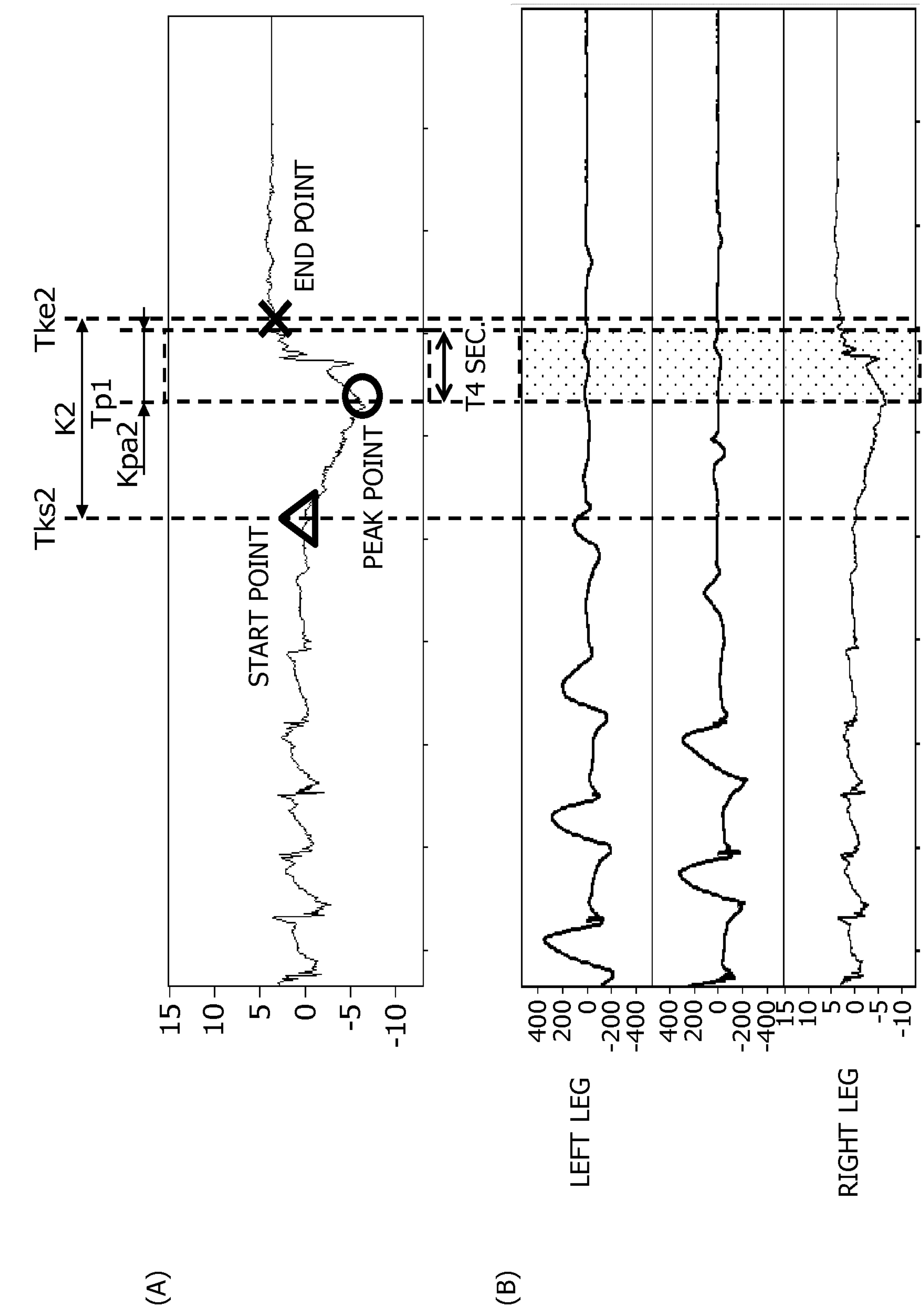
(A)
(B)
Tks2
K2
Kpa2
Tp1
Tke2
START POINT
PEAK POINT
END POINT
T4 SEC.
15
10
5
0
-5
-10
LEFT LEG
RIGHT LEG
400
200
0
-200
-400

FIG. 8

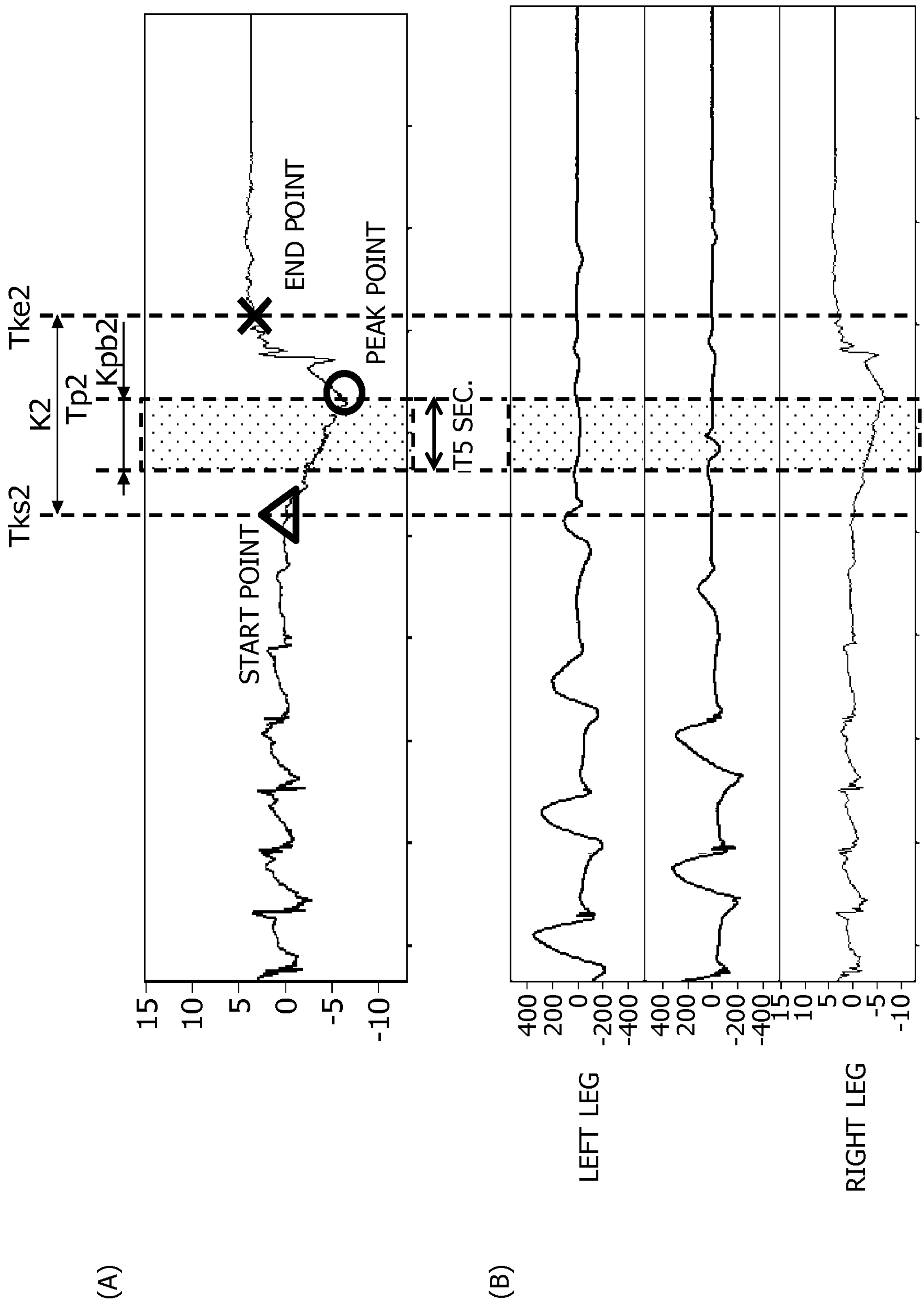

(A)
Tks2
K2
Tp2
Kpb2
Tke2
START POINT
PEAK POINT
END POINT
T5 SEC.
15
10
5
0
-5
-10
(B)
LEFT LEG
RIGHT LEG
400
200
0
-200
-400

FIG. 9

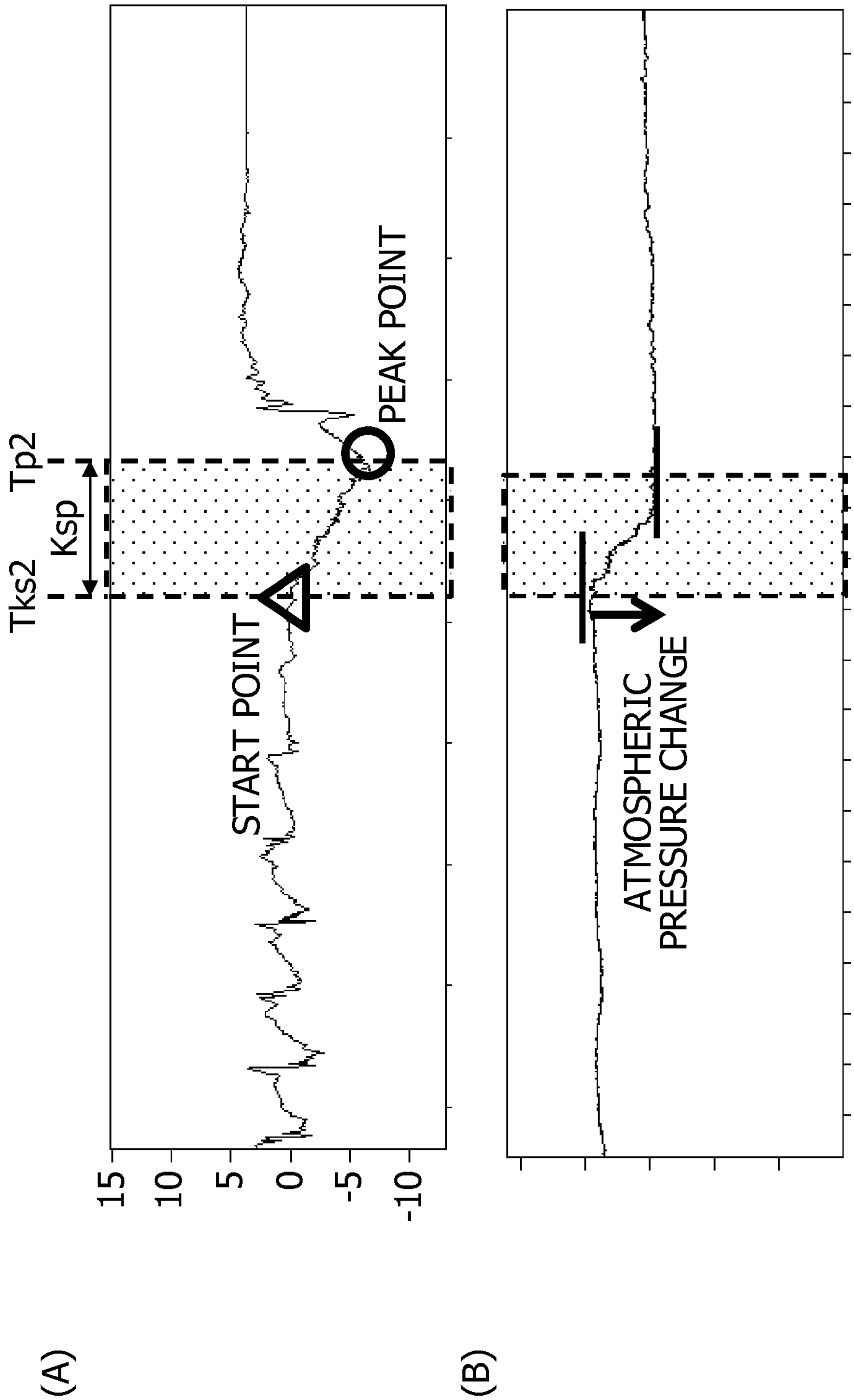
Tks2
Ksp
Tp2
PEAK POINT
START POINT
15
10
5
0
-5
-10
ATMOSPHERIC PRESSURE CHANGE
(A)
(B)

FIG. 10

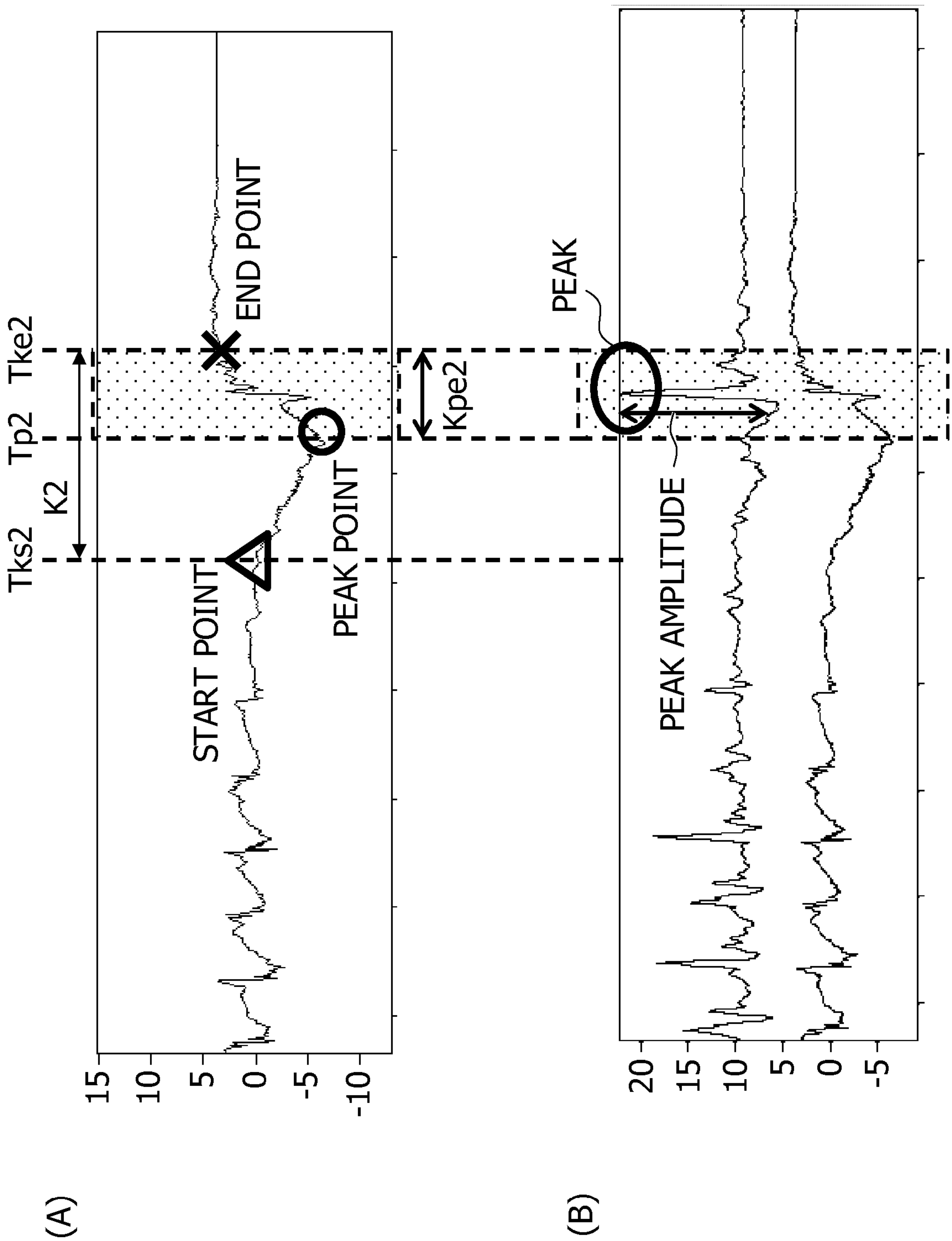
Tks2
K2
Tp2
Tke2
START POINT
PEAK POINT
END POINT
Kpe2
PEAK
PEAK AMPLITUDE
15
10
5
0
-5
-10
20
15
10
5
0
-5
(A)
(B)

FIG. 11

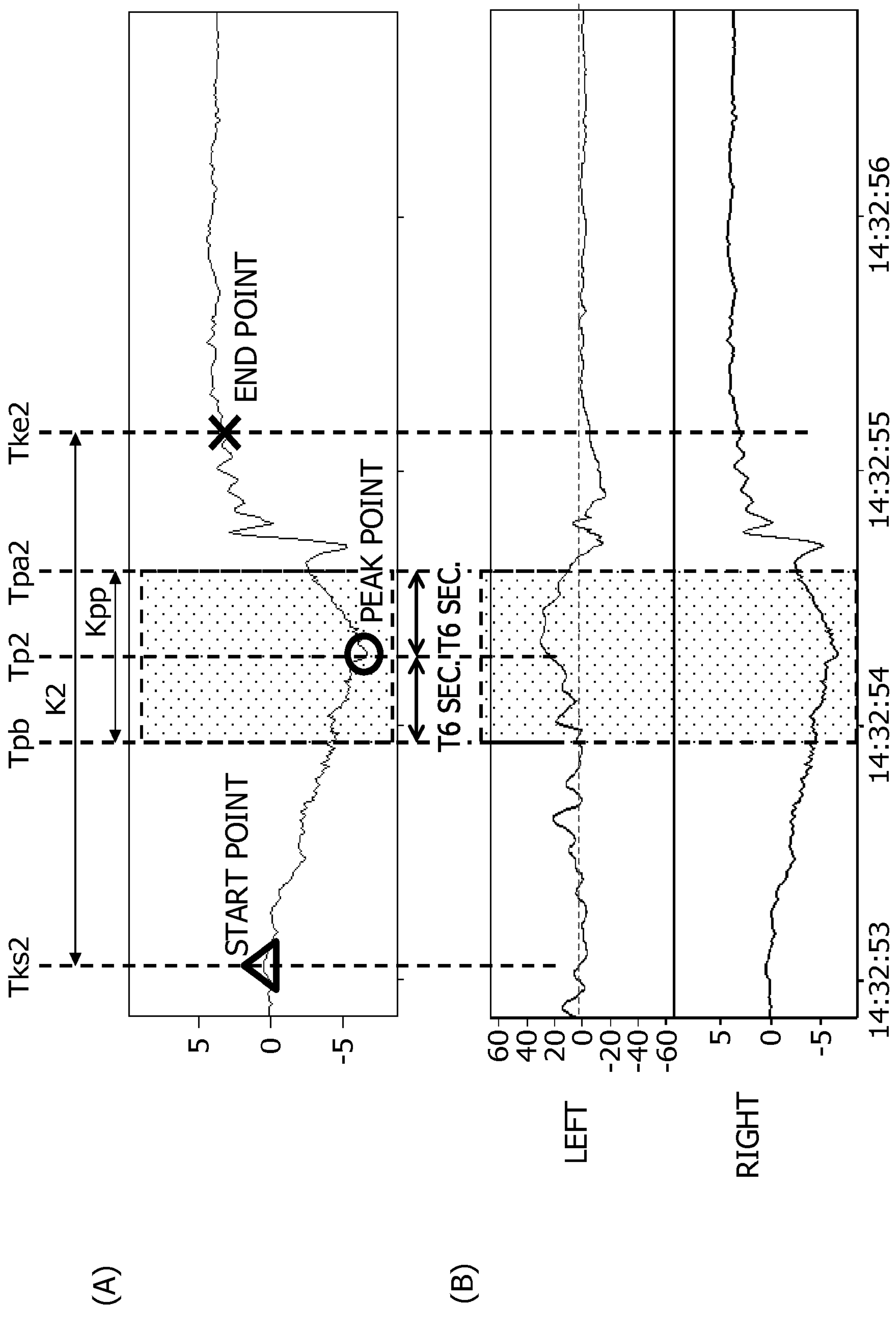
(A)
(B)
Tks2
Tpb
Tp2
Tpa2
Tke2
K2
Kpp
START POINT
PEAK POINT
END POINT
T6 SEC.
T6 SEC.
5
0
-5
LEFT
RIGHT
60
40
20
0
-20
-40
-60
14:32:53
14:32:54
14:32:55
14:32:56

FIG. 12

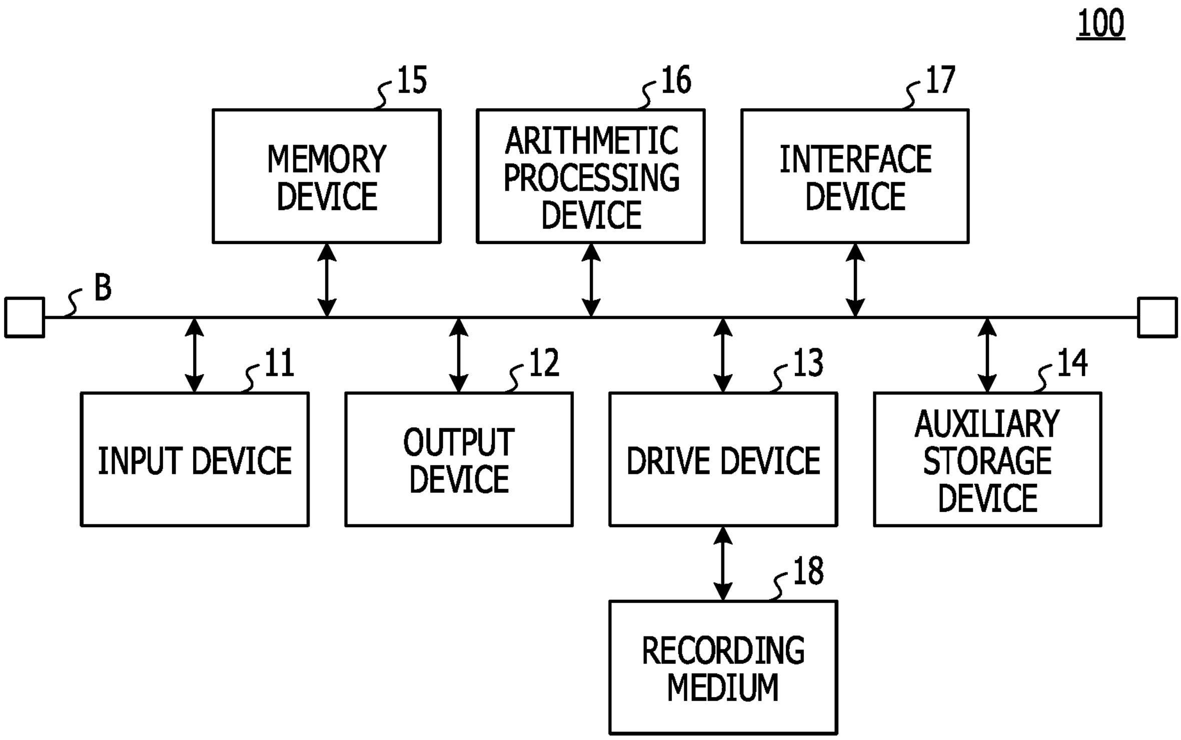
100
15
MEMORY DEVICE
16
ARITHMETIC PROCESSING DEVICE
17
INTERFACE DEVICE
B
11
INPUT DEVICE
12
OUTPUT DEVICE
13
DRIVE DEVICE
14
AUXILIARY STORAGE DEVICE
18
RECORDING MEDIUM

FIG. 13

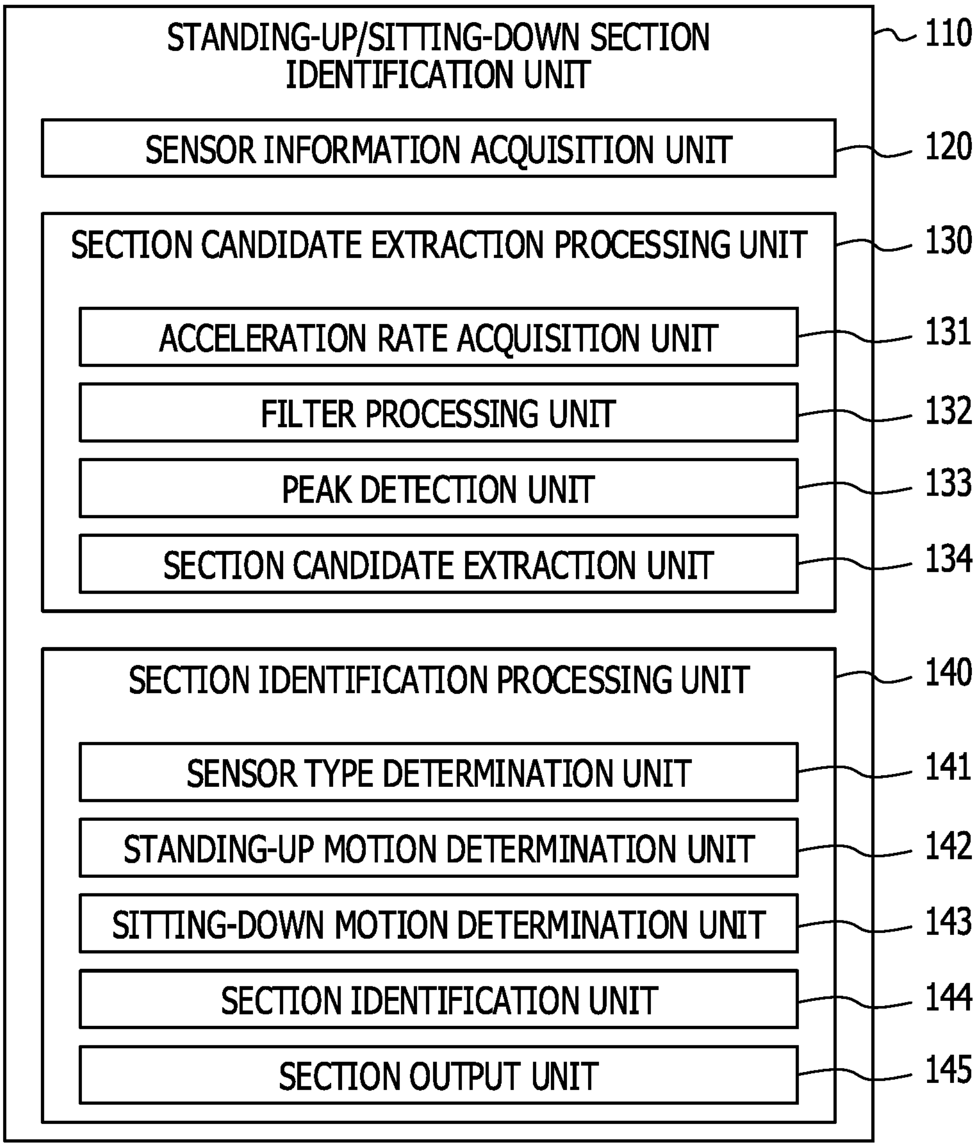
STANDING-UP/SITTING-DOWN SECTION IDENTIFICATION UNIT
110
SENSOR INFORMATION ACQUISITION UNIT
120
SECTION CANDIDATE EXTRACTION PROCESSING UNIT
130
ACCELERATION RATE ACQUISITION UNIT
131
FILTER PROCESSING UNIT
132
PEAK DETECTION UNIT
133
SECTION CANDIDATE EXTRACTION UNIT
134
SECTION IDENTIFICATION PROCESSING UNIT
140
SENSOR TYPE DETERMINATION UNIT
141
STANDING-UP MOTION DETERMINATION UNIT
142
SITTING-DOWN MOTION DETERMINATION UNIT
143
SECTION IDENTIFICATION UNIT
144
SECTION OUTPUT UNIT
145

FIG. 14

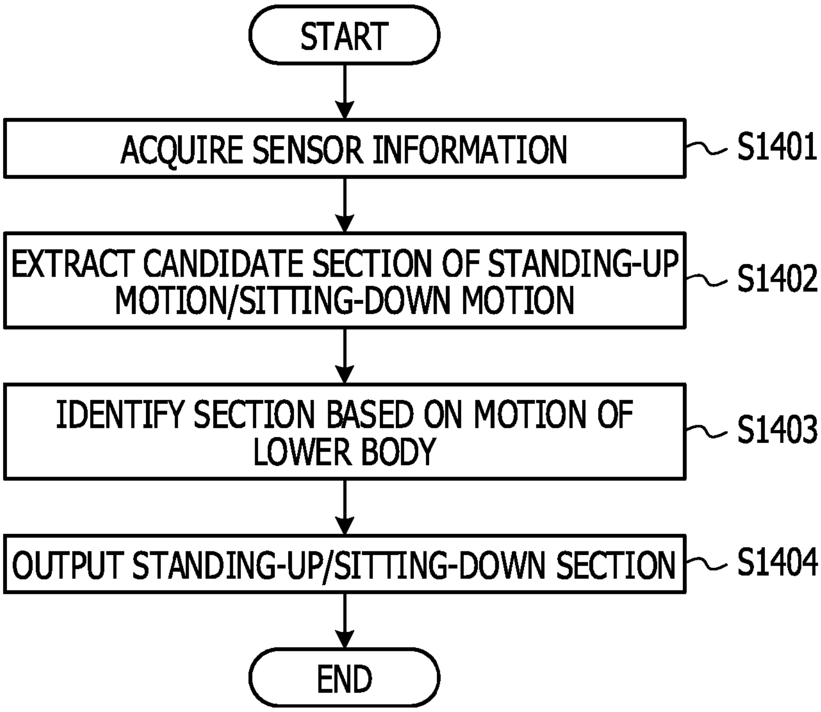
START
ACQUIRE SENSOR INFORMATION
S1401
EXTRACT CANDIDATE SECTION OF STANDING-UP MOTION/SITTING-DOWN MOTION
S1402
IDENTIFY SECTION BASED ON MOTION OF LOWER BODY
S1403
OUTPUT STANDING-UP/SITTING-DOWN SECTION
S1404
END

FIG. 15

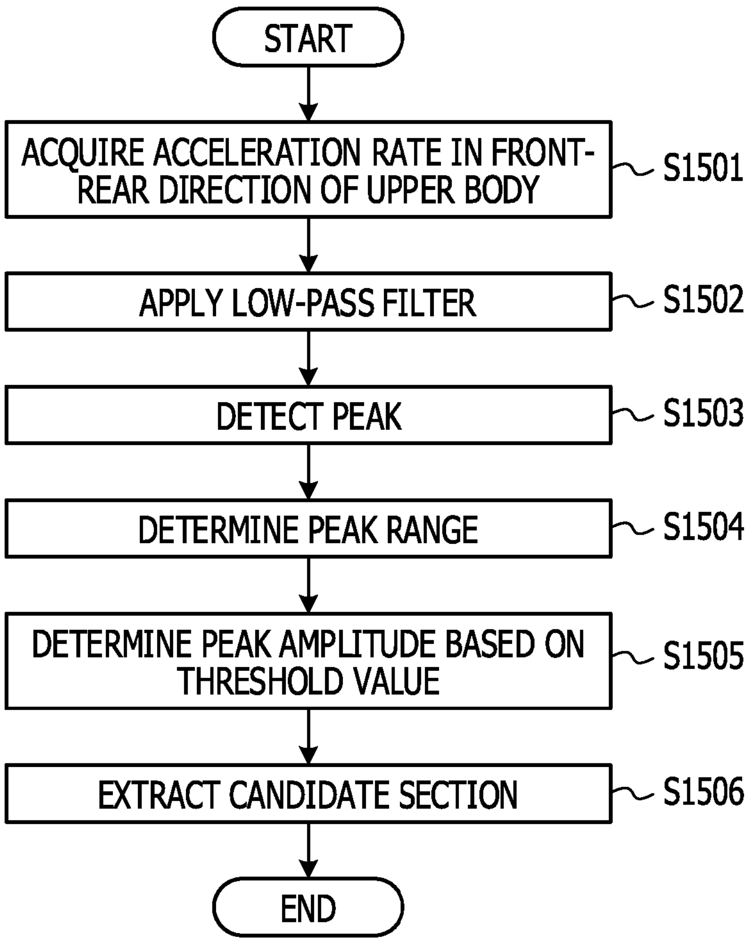
START
ACQUIRE ACCELERATION RATE IN FRONT-REAR DIRECTION OF UPPER BODY
S1501
APPLY LOW-PASS FILTER
S1502
DETECT PEAK
S1503
DETERMINE PEAK RANGE
S1504
DETERMINE PEAK AMPLITUDE BASED ON THRESHOLD VALUE
S1505
EXTRACT CANDIDATE SECTION
S1506
END

FIG. 16

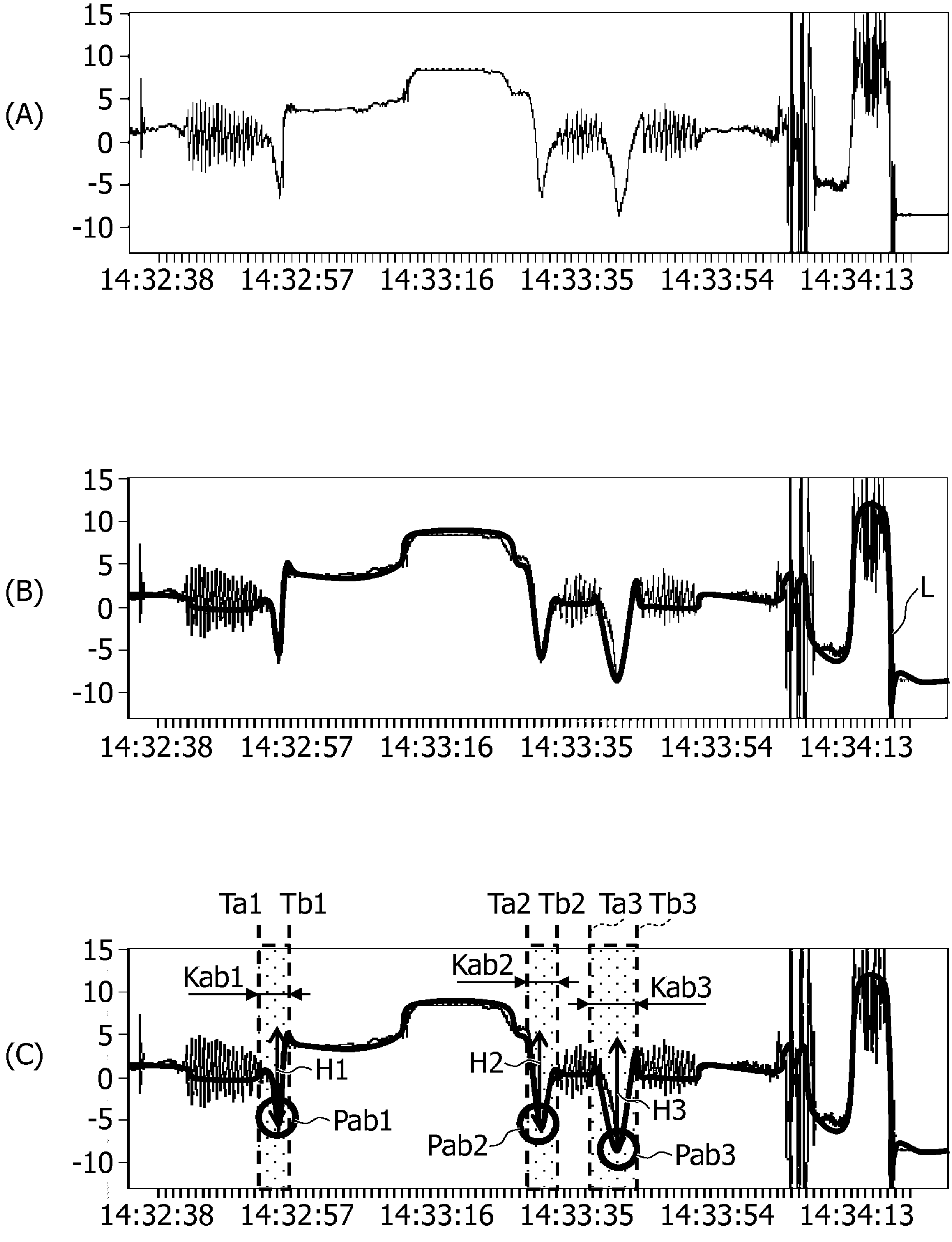
(A)
15
10
5
0
-5
-10
14:32:38
14:32:57
14:33:16
14:33:35
14:33:54
14:34:13
(B)
L
(C)
Ta1
Tb1
Ta2
Tb2
Ta3
Tb3
Kab1
Kab2
Kab3
H1
H2
H3
Pab1
Pab2
Pab3

FIG. 17A

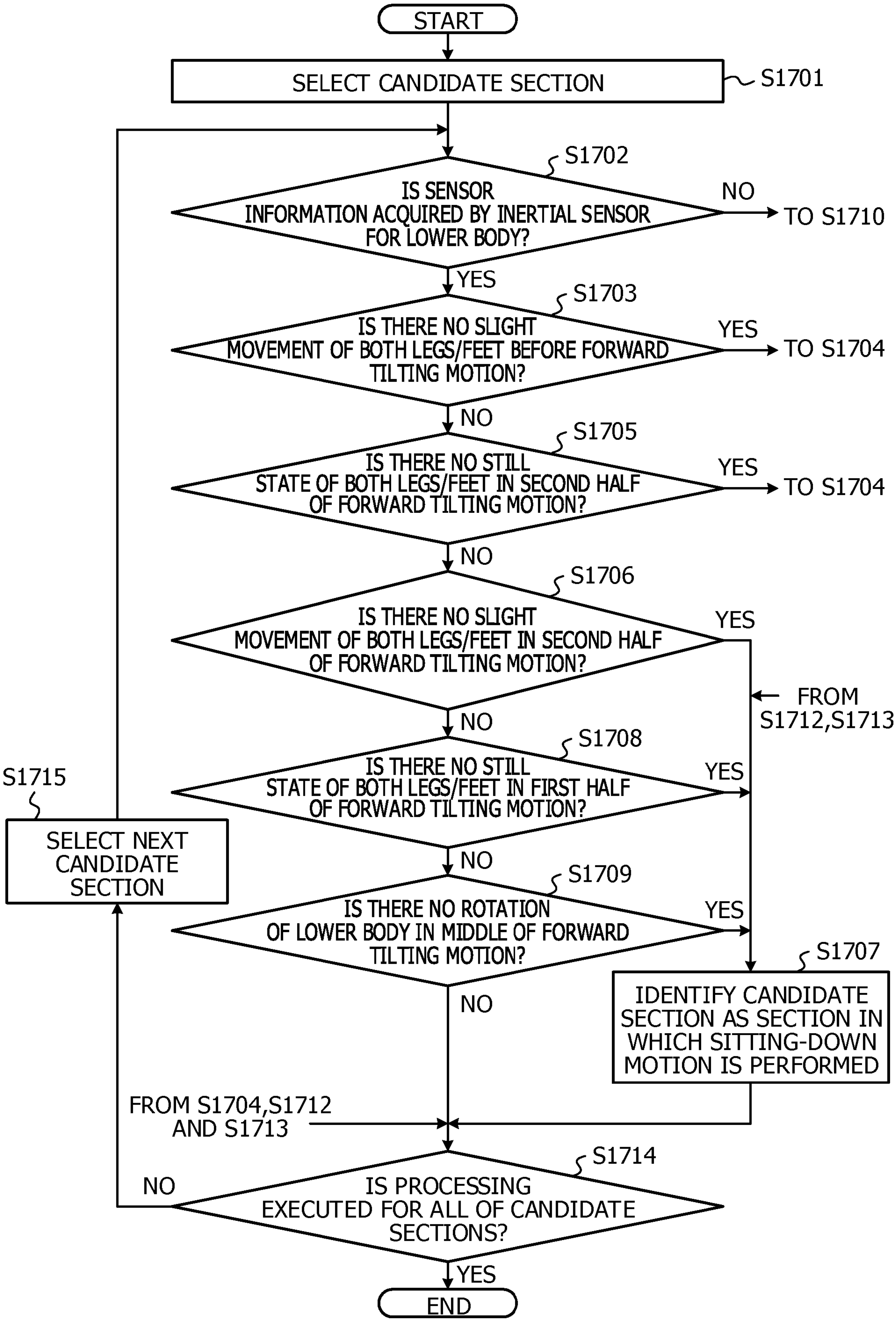
START
SELECT CANDIDATE SECTION
S1701
S1702
IS SENSOR INFORMATION ACQUIRED BY INERTIAL SENSOR FOR LOWER BODY?
NO
TO S1710
YES
S1703
IS THERE NO SLIGHT MOVEMENT OF BOTH LEGS/FEET BEFORE FORWARD TILTING MOTION?
YES
TO S1704
NO
S1705
IS THERE NO STILL STATE OF BOTH LEGS/FEET IN SECOND HALF OF FORWARD TILTING MOTION?
YES
TO S1704
NO
S1706
IS THERE NO SLIGHT MOVEMENT OF BOTH LEGS/FEET IN SECOND HALF OF FORWARD TILTING MOTION?
YES
FROM S1712,S1713
NO
S1708
IS THERE NO STILL STATE OF BOTH LEGS/FEET IN FIRST HALF OF FORWARD TILTING MOTION?
YES
S1715
SELECT NEXT CANDIDATE SECTION
NO
S1709
IS THERE NO ROTATION OF LOWER BODY IN MIDDLE OF FORWARD TILTING MOTION?
YES
S1707
IDENTIFY CANDIDATE SECTION AS SECTION IN WHICH SITTING-DOWN MOTION IS PERFORMED
NO
FROM S1704,S1712 AND S1713
S1714
NO
IS PROCESSING EXECUTED FOR ALL OF CANDIDATE SECTIONS?
YES
END

# FIG. 17B

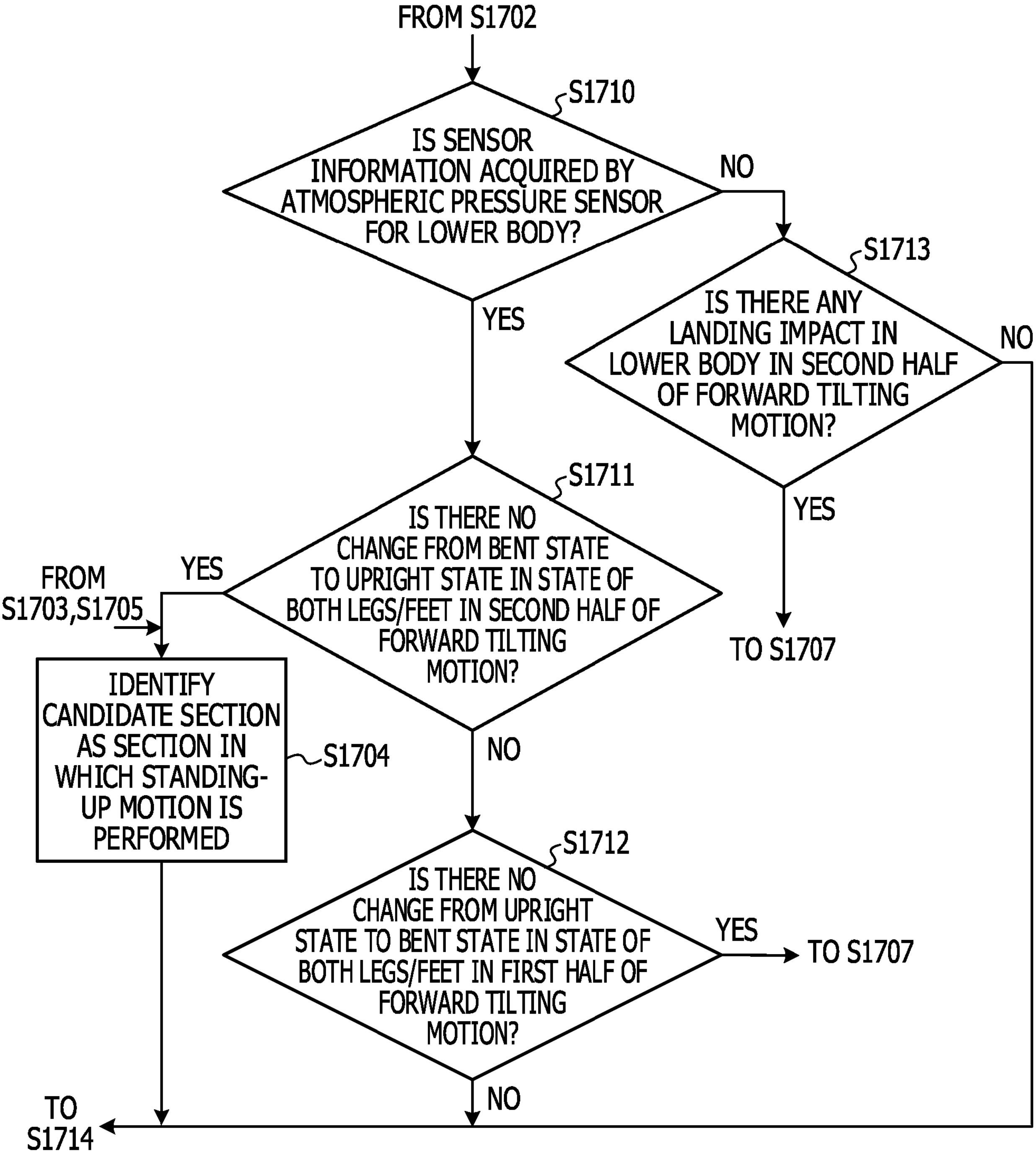
FROM S1702
S1710
IS SENSOR INFORMATION ACQUIRED BY ATMOSPHERIC PRESSURE SENSOR FOR LOWER BODY?
NO
YES
S1713
IS THERE ANY LANDING IMPACT IN LOWER BODY IN SECOND HALF OF FORWARD TILTING MOTION?
NO
YES
TO S1707
S1711
IS THERE NO CHANGE FROM BENT STATE TO UPRIGHT STATE IN STATE OF BOTH LEGS/FEET IN SECOND HALF OF FORWARD TILTING MOTION?
YES
FROM S1703,S1705
IDENTIFY CANDIDATE SECTION AS SECTION IN WHICH STANDING-UP MOTION IS PERFORMED
S1704
NO
S1712
IS THERE NO CHANGE FROM UPRIGHT STATE TO BENT STATE IN STATE OF BOTH LEGS/FEET IN FIRST HALF OF FORWARD TILTING MOTION?
YES
TO S1707
NO
TO S1714

FIG. 18

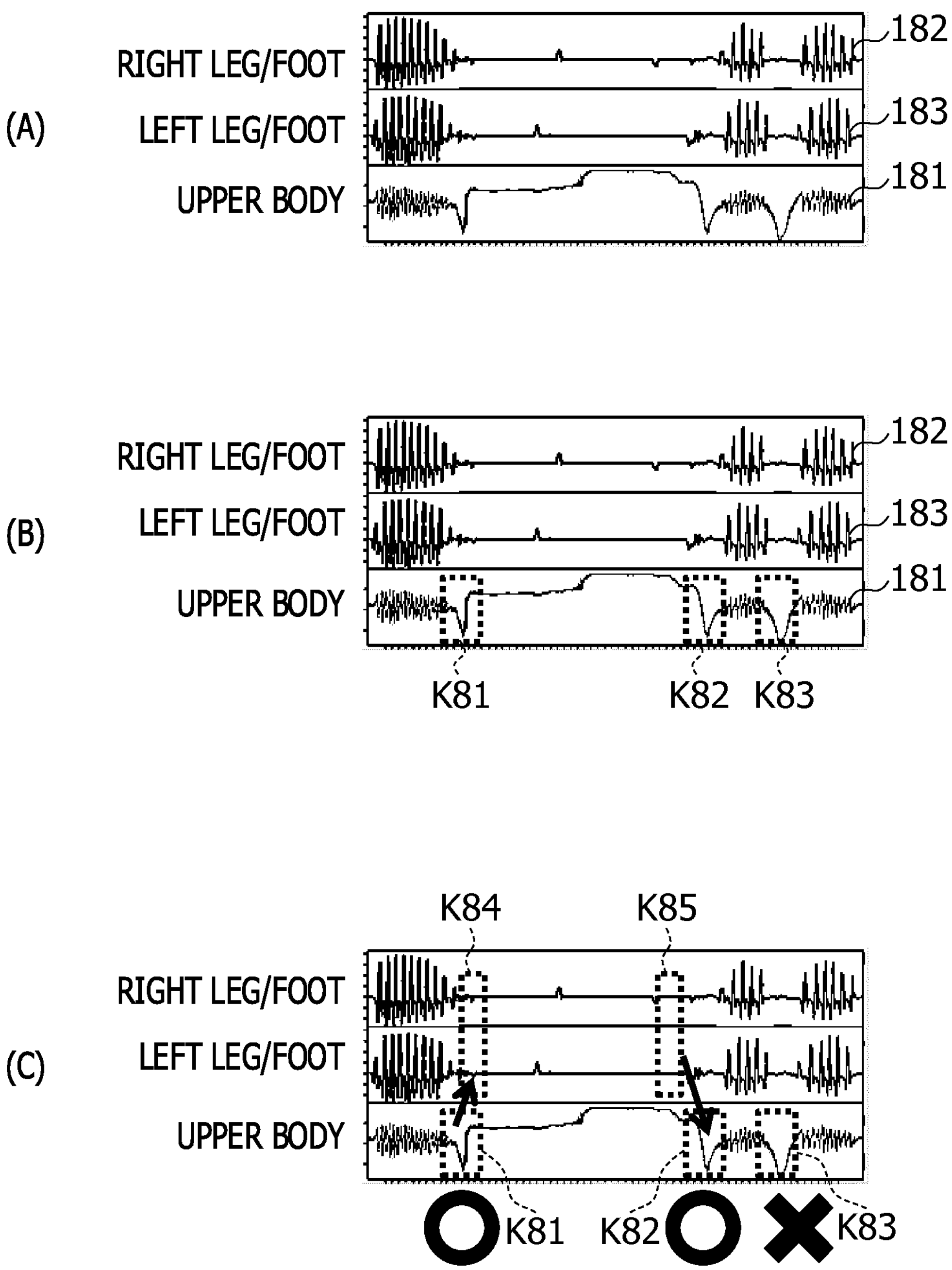
(A)
RIGHT LEG/FOOT
LEFT LEG/FOOT
UPPER BODY
182
183
181
(B)
RIGHT LEG/FOOT
LEFT LEG/FOOT
UPPER BODY
182
183
181
K81
K82
K83
(C)
K84
K85
RIGHT LEG/FOOT
LEFT LEG/FOOT
UPPER BODY
K81
K82
K83

FIG. 19

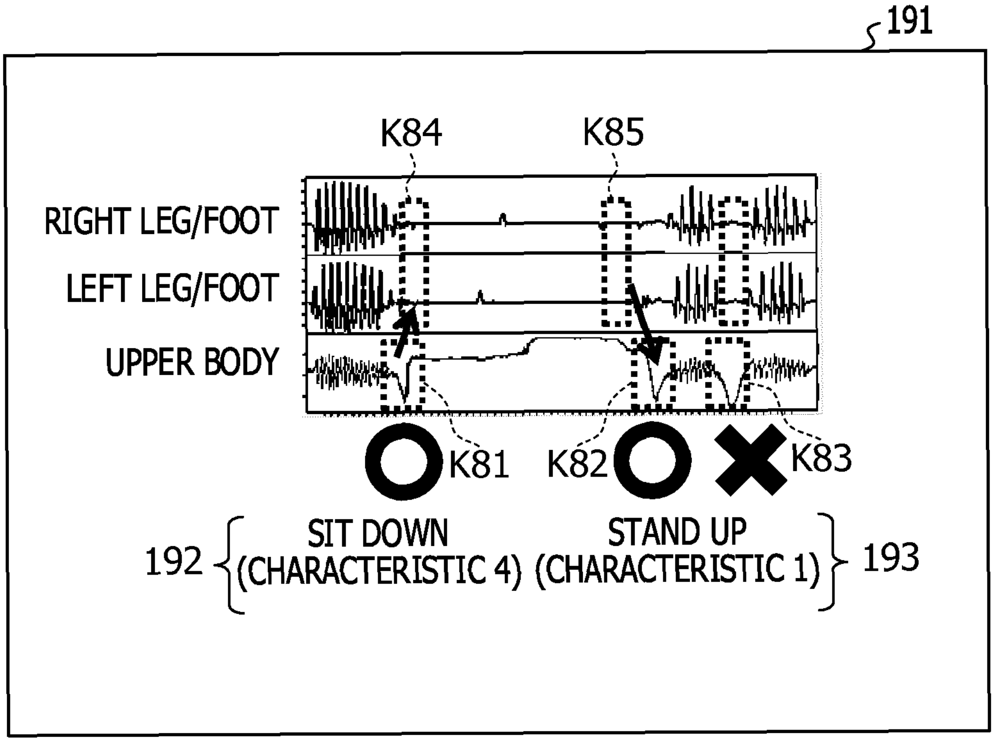
191
K84
K85
RIGHT LEG/FOOT
LEFT LEG/FOOT
UPPER BODY
K81
K82
K83
SIT DOWN
(CHARACTERISTIC 4)
STAND UP
(CHARACTERISTIC 1)
192
193

FIG. 20A

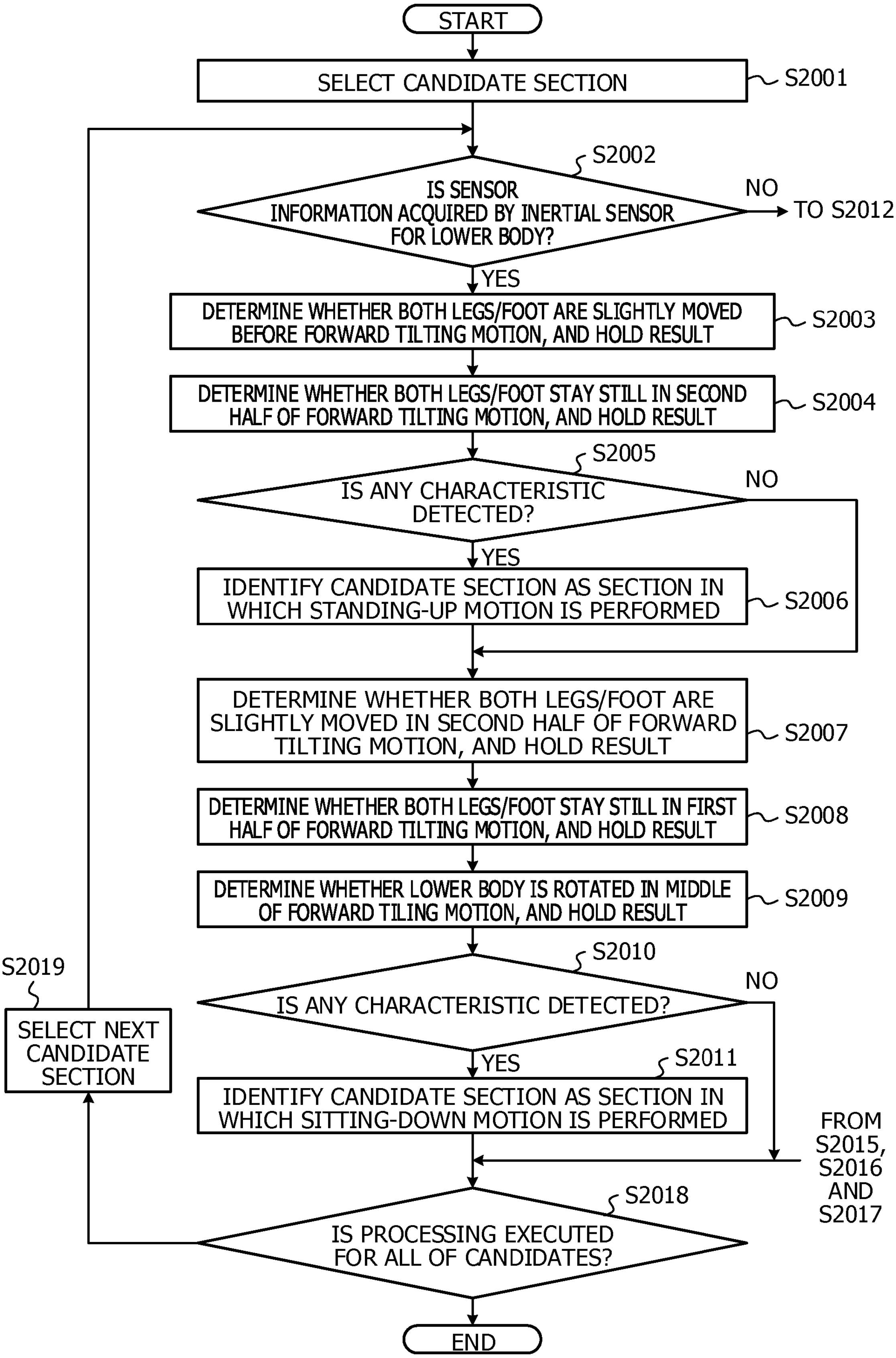
START
SELECT CANDIDATE SECTION
S2001
S2002
IS SENSOR INFORMATION ACQUIRED BY INERTIAL SENSOR FOR LOWER BODY?
NO
TO S2012
YES
DETERMINE WHETHER BOTH LEGS/FOOT ARE SLIGHTLY MOVED BEFORE FORWARD TILTING MOTION, AND HOLD RESULT
S2003
DETERMINE WHETHER BOTH LEGS/FOOT STAY STILL IN SECOND HALF OF FORWARD TILTING MOTION, AND HOLD RESULT
S2004
S2005
IS ANY CHARACTERISTIC DETECTED?
NO
YES
IDENTIFY CANDIDATE SECTION AS SECTION IN WHICH STANDING-UP MOTION IS PERFORMED
S2006
DETERMINE WHETHER BOTH LEGS/FOOT ARE SLIGHTLY MOVED IN SECOND HALF OF FORWARD TILTING MOTION, AND HOLD RESULT
S2007
DETERMINE WHETHER BOTH LEGS/FOOT STAY STILL IN FIRST HALF OF FORWARD TILTING MOTION, AND HOLD RESULT
S2008
DETERMINE WHETHER LOWER BODY IS ROTATED IN MIDDLE OF FORWARD TILING MOTION, AND HOLD RESULT
S2009
S2019
SELECT NEXT CANDIDATE SECTION
S2010
IS ANY CHARACTERISTIC DETECTED?
NO
YES
S2011
IDENTIFY CANDIDATE SECTION AS SECTION IN WHICH SITTING-DOWN MOTION IS PERFORMED
FROM S2015, S2016 AND S2017
S2018
IS PROCESSING EXECUTED FOR ALL OF CANDIDATES?
END

FIG. 20B

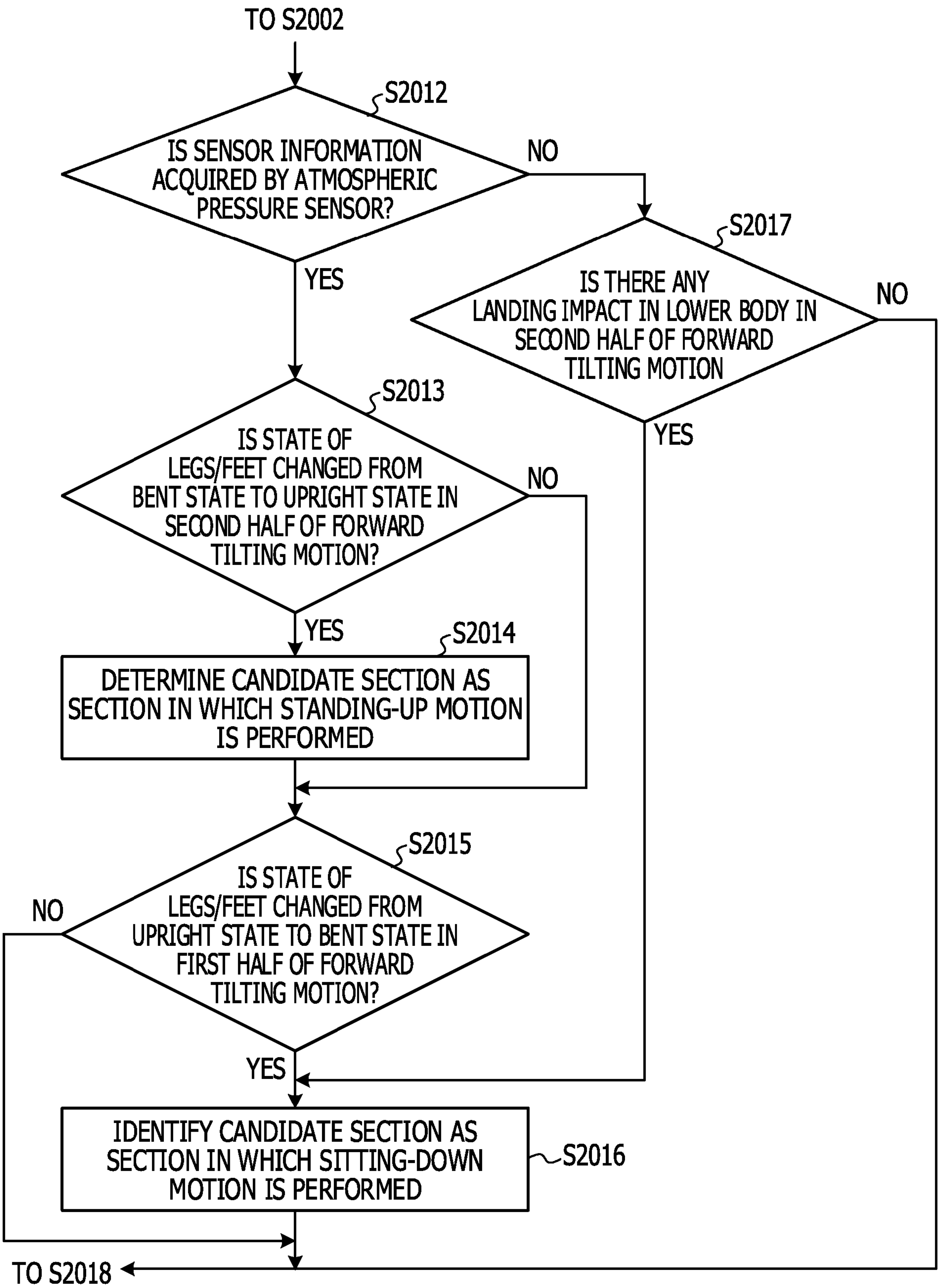
TO S2002
S2012
IS SENSOR INFORMATION ACQUIRED BY ATMOSPHERIC PRESSURE SENSOR?
NO
YES
S2017
IS THERE ANY LANDING IMPACT IN LOWER BODY IN SECOND HALF OF FORWARD TILTING MOTION
NO
YES
S2013
IS STATE OF LEGS/FEET CHANGED FROM BENT STATE TO UPRIGHT STATE IN SECOND HALF OF FORWARD TILTING MOTION?
NO
YES
S2014
DETERMINE CANDIDATE SECTION AS SECTION IN WHICH STANDING-UP MOTION IS PERFORMED
S2015
IS STATE OF LEGS/FEET CHANGED FROM UPRIGHT STATE TO BENT STATE IN FIRST HALF OF FORWARD TILTING MOTION?
NO
YES
IDENTIFY CANDIDATE SECTION AS SECTION IN WHICH SITTING-DOWN MOTION IS PERFORMED
S2016
TO S2018

# FIG. 21

211

| CANDIDATE SECTION ID | | | | 1 | 2 | 3 | ... |
|---|---|---|---|---|---|---|---|
| MOTION DETECTION RESULT | STANDING-UP MOTION | CHARACTERISTIC 1 | BOTH LEGS/FEET ARE SLIGHTLY MOVED BEFORE FORWARD TILTING MOTION OF UPPER BODY | ○ | × | × | ... |
| | | CHARACTERISTIC 2 | BOTH LEGS/FEET STAY STILL IN SECOND HALF OF FORWARD TILTING MOTION OF UPPER BODY | ○ | × | × | ... |
| | | ⋮ | ... | ... | ... | ... | ... |
| | SITTING-DOWN MOTION | CHARACTERISTIC 3 | BOTH LEGS/FEET ARE SLIGHTLY MOVED IN SECOND HALF OF FORWARD TILTING MOTION OF UPPER BODY | × | ○ | × | ... |
| | | CHARACTERISTIC 4 | BOTH LEGS/FEET STAY STILL IN FIRST HALF OF FORWARD TILTING MOTION OF UPPER BODY | × | ○ | × | ... |
| | | ⋮ | ... | × | ... | × | ... |
| IDENTIFICATION RESULT | | | | STAND UP | SIT DOWN | × | ... |

FIG. 22

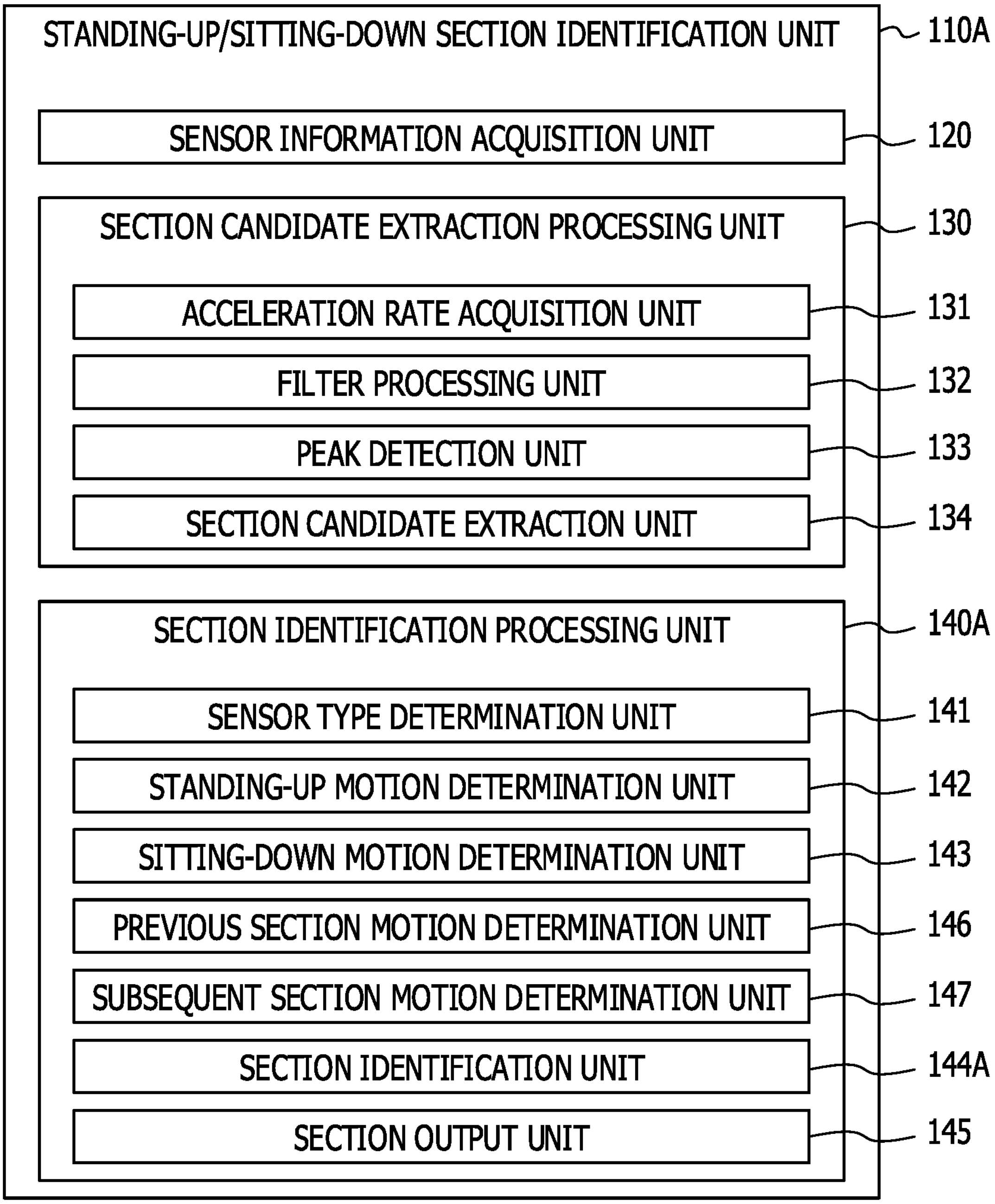
STANDING-UP/SITTING-DOWN SECTION IDENTIFICATION UNIT
110A
SENSOR INFORMATION ACQUISITION UNIT
120
SECTION CANDIDATE EXTRACTION PROCESSING UNIT
130
ACCELERATION RATE ACQUISITION UNIT
131
FILTER PROCESSING UNIT
132
PEAK DETECTION UNIT
133
SECTION CANDIDATE EXTRACTION UNIT
134
SECTION IDENTIFICATION PROCESSING UNIT
140A
SENSOR TYPE DETERMINATION UNIT
141
STANDING-UP MOTION DETERMINATION UNIT
142
SITTING-DOWN MOTION DETERMINATION UNIT
143
PREVIOUS SECTION MOTION DETERMINATION UNIT
146
SUBSEQUENT SECTION MOTION DETERMINATION UNIT
147
SECTION IDENTIFICATION UNIT
144A
SECTION OUTPUT UNIT
145

FIG. 23

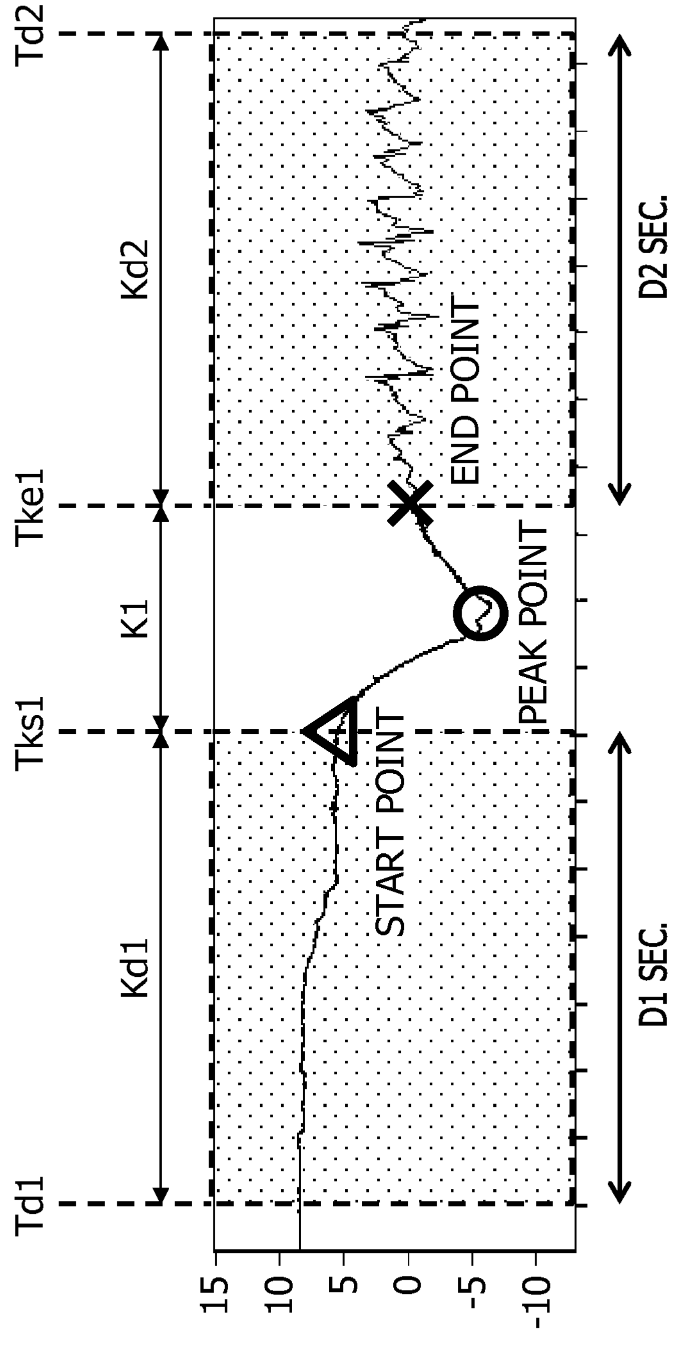
Td1
Tks1
Tke1
Td2
Kd1
K1
Kd2
START POINT
PEAK POINT
END POINT
D1 SEC.
D2 SEC.
15
10
5
0
-5
-10

FIG. 24

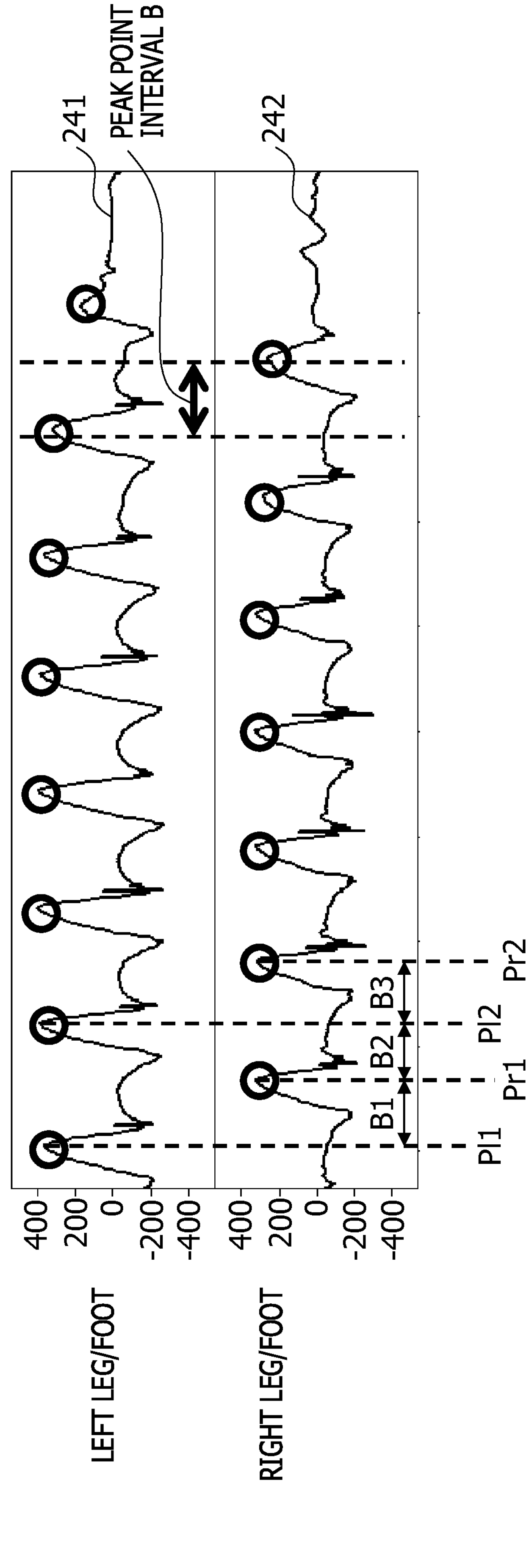
241
PEAK POINT
INTERVAL B
242
LEFT LEG/FOOT
RIGHT LEG/FOOT
400
200
0
-200
-400
400
200
0
-200
-400
B1
B2
B3
Pl1
Pr1
Pl2
Pr2

FIG. 25

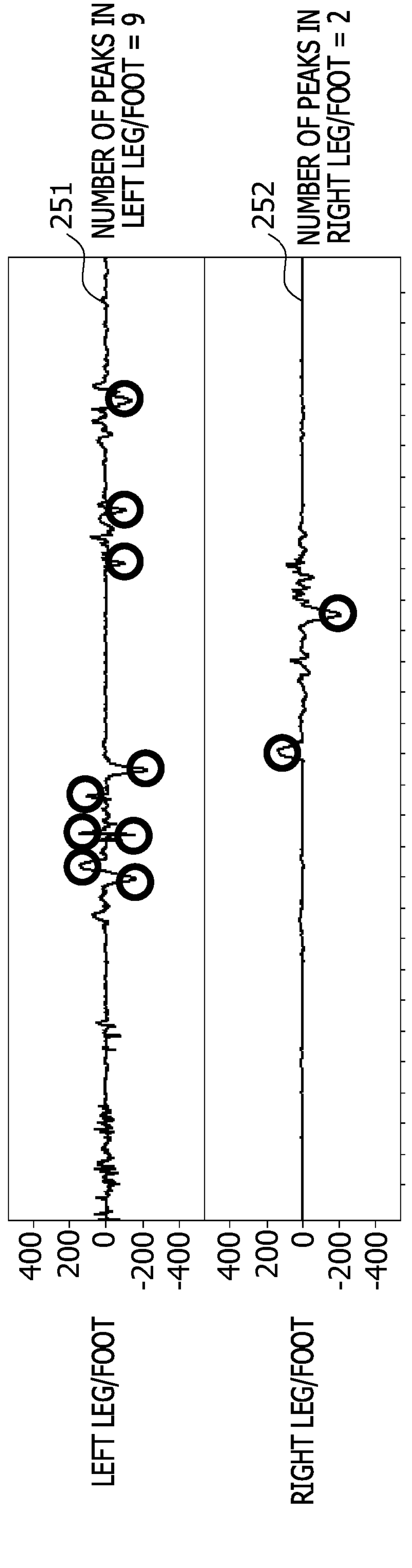

LEFT LEG/FOOT
RIGHT LEG/FOOT
400
200
0
-200
-400
400
200
0
-200
-400
251
NUMBER OF PEAKS IN LEFT LEG/FOOT = 9
252
NUMBER OF PEAKS IN RIGHT LEG/FOOT = 2

FIG. 26

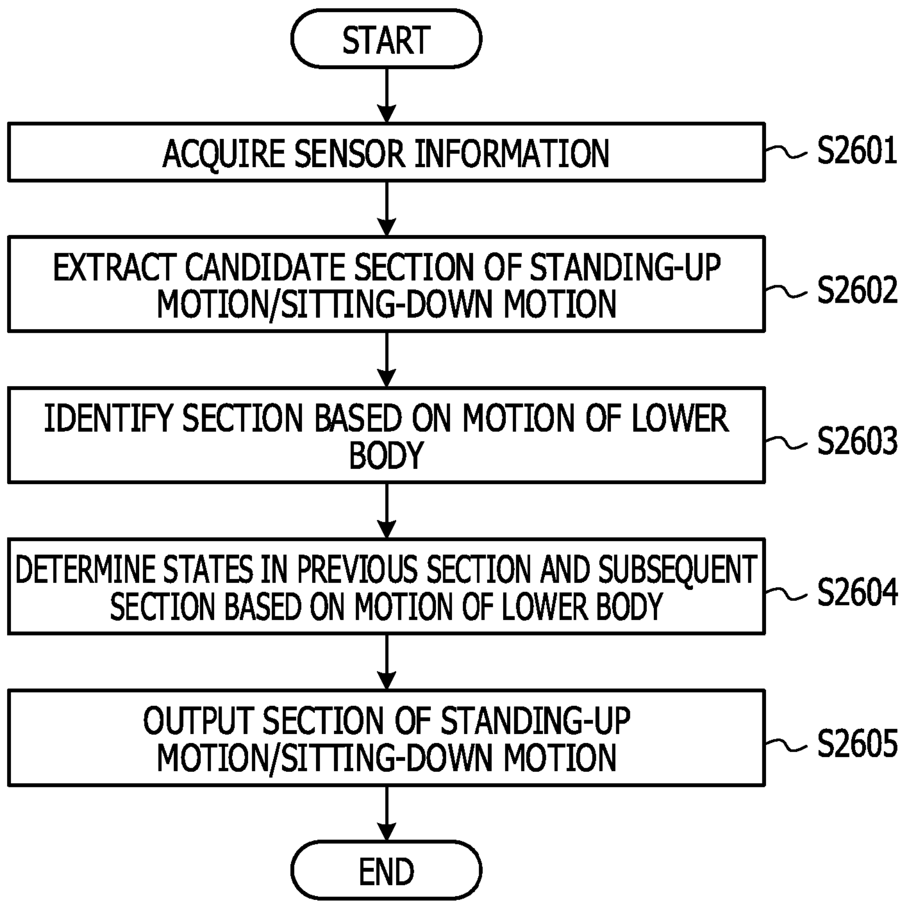
START
ACQUIRE SENSOR INFORMATION
S2601
EXTRACT CANDIDATE SECTION OF STANDING-UP MOTION/SITTING-DOWN MOTION
S2602
IDENTIFY SECTION BASED ON MOTION OF LOWER BODY
S2603
DETERMINE STATES IN PREVIOUS SECTION AND SUBSEQUENT SECTION BASED ON MOTION OF LOWER BODY
S2604
OUTPUT SECTION OF STANDING-UP MOTION/SITTING-DOWN MOTION
S2605
END

FIG. 27

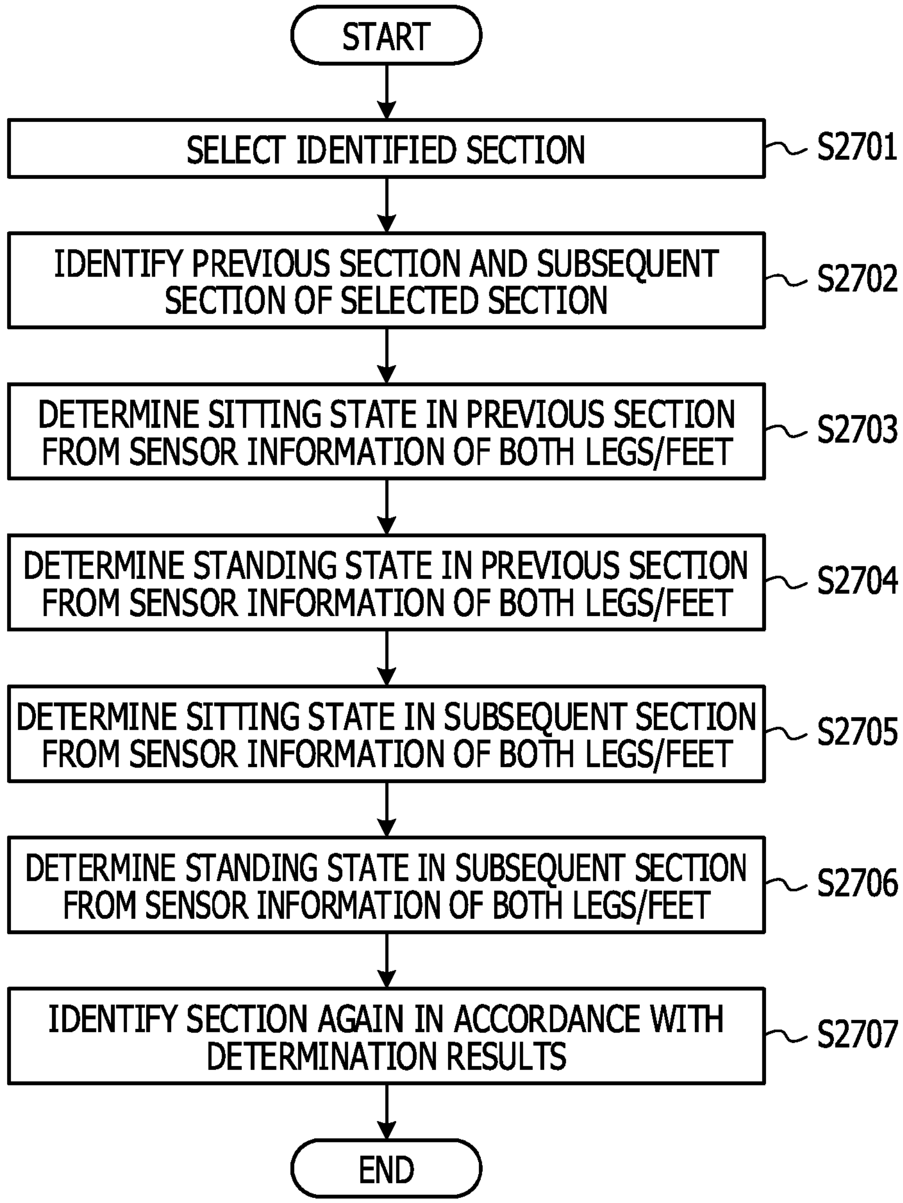
START
SELECT IDENTIFIED SECTION
S2701
IDENTIFY PREVIOUS SECTION AND SUBSEQUENT SECTION OF SELECTED SECTION
S2702
DETERMINE SITTING STATE IN PREVIOUS SECTION FROM SENSOR INFORMATION OF BOTH LEGS/FEET
S2703
DETERMINE STANDING STATE IN PREVIOUS SECTION FROM SENSOR INFORMATION OF BOTH LEGS/FEET
S2704
DETERMINE SITTING STATE IN SUBSEQUENT SECTION FROM SENSOR INFORMATION OF BOTH LEGS/FEET
S2705
DETERMINE STANDING STATE IN SUBSEQUENT SECTION FROM SENSOR INFORMATION OF BOTH LEGS/FEET
S2706
IDENTIFY SECTION AGAIN IN ACCORDANCE WITH DETERMINATION RESULTS
S2707
END

FIG. 28

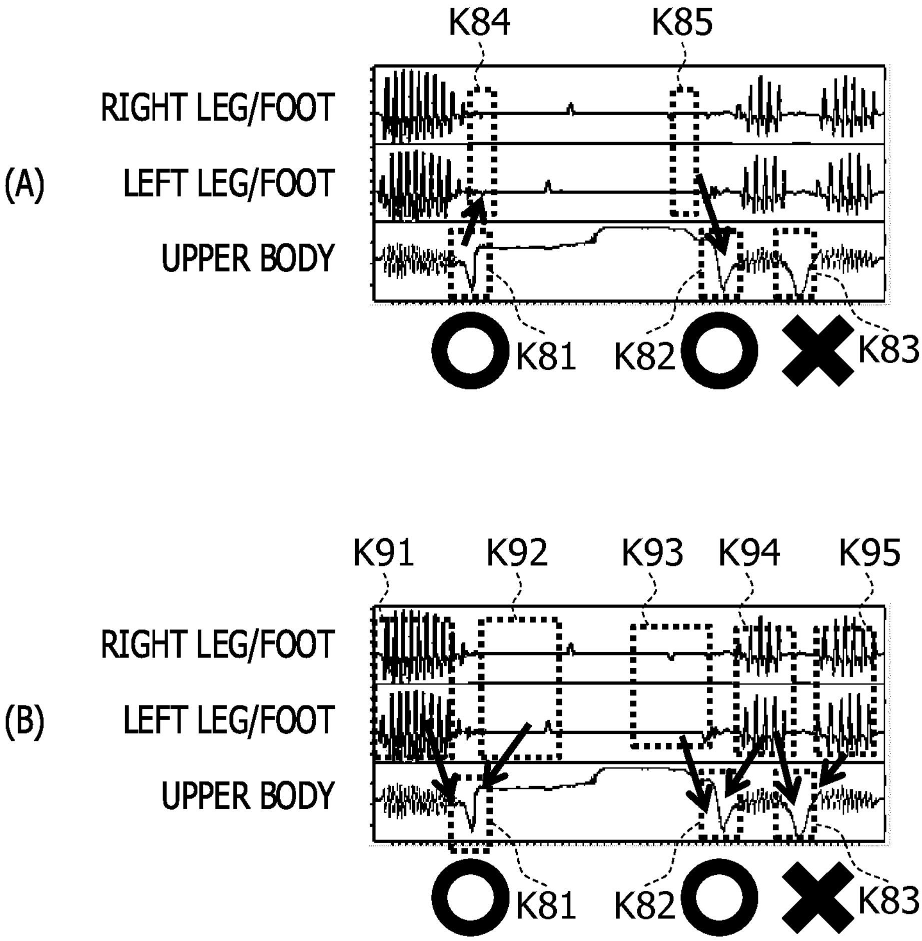
(A)
K84
K85
RIGHT LEG/FOOT
LEFT LEG/FOOT
UPPER BODY
K81
K82
K83
(B)
K91
K92
K93
K94
K95
RIGHT LEG/FOOT
LEFT LEG/FOOT
UPPER BODY
K81
K82
K83

# FIG. 29

291

| CANDIDATE SECTION ID | | | | 1 | 2 | 3 | ... |
|---|---|---|---|---|---|---|---|
| MOTION DETECTION RESULT | STANDING-UP MOTION | CHARACTERISTIC 1 | BOTH LEGS/FEET ARE SLIGHTLY MOVED BEFORE FORWARD TILTING MOTION OF UPPER BODY | ○ | × | × | ... |
| | | CHARACTERISTIC 2 | BOTH LEGS/FEET STAY STILL IN SECOND HALF OF FORWARD TILTING MOTION OF UPPER BODY | ○ | × | × | ... |
| | | ⋮ | ... | ... | ... | ... | ... |
| | SITTING-DOWN MOTION | CHARACTERISTIC 4 | BOTH LEGS/FEET ARE SLIGHTLY MOVED IN SECOND HALF OF FORWARD TILTING MOTION OF UPPER BODY | × | ○ | × | ... |
| | | CHARACTERISTIC 5 | BOTH LEGS/FEET STAY STILL IN FIRST HALF OF FORWARD TILTING MOTION OF UPPER BODY | × | ○ | × | ... |
| | | ⋮ | ... | × | ... | × | ... |
| STATES IN PREVIOUS AND SUBSEQUENT SECTIONS OF CANDIDATE SECTION | PREVIOUS SECTION “SITTING STATE” | | SUBSEQUENT SECTION “STANDING STATE” | ○ | × | × | ... |
| | PREVIOUS SECTION “STANDING STATE” | | SUBSEQUENT SECTION “SITTING SATE” | × | ○ | ○ | ... |
| IDENTIFICATION RESULT | | | | STAND UP | SIT DOWN | × | ... |

FIG. 30

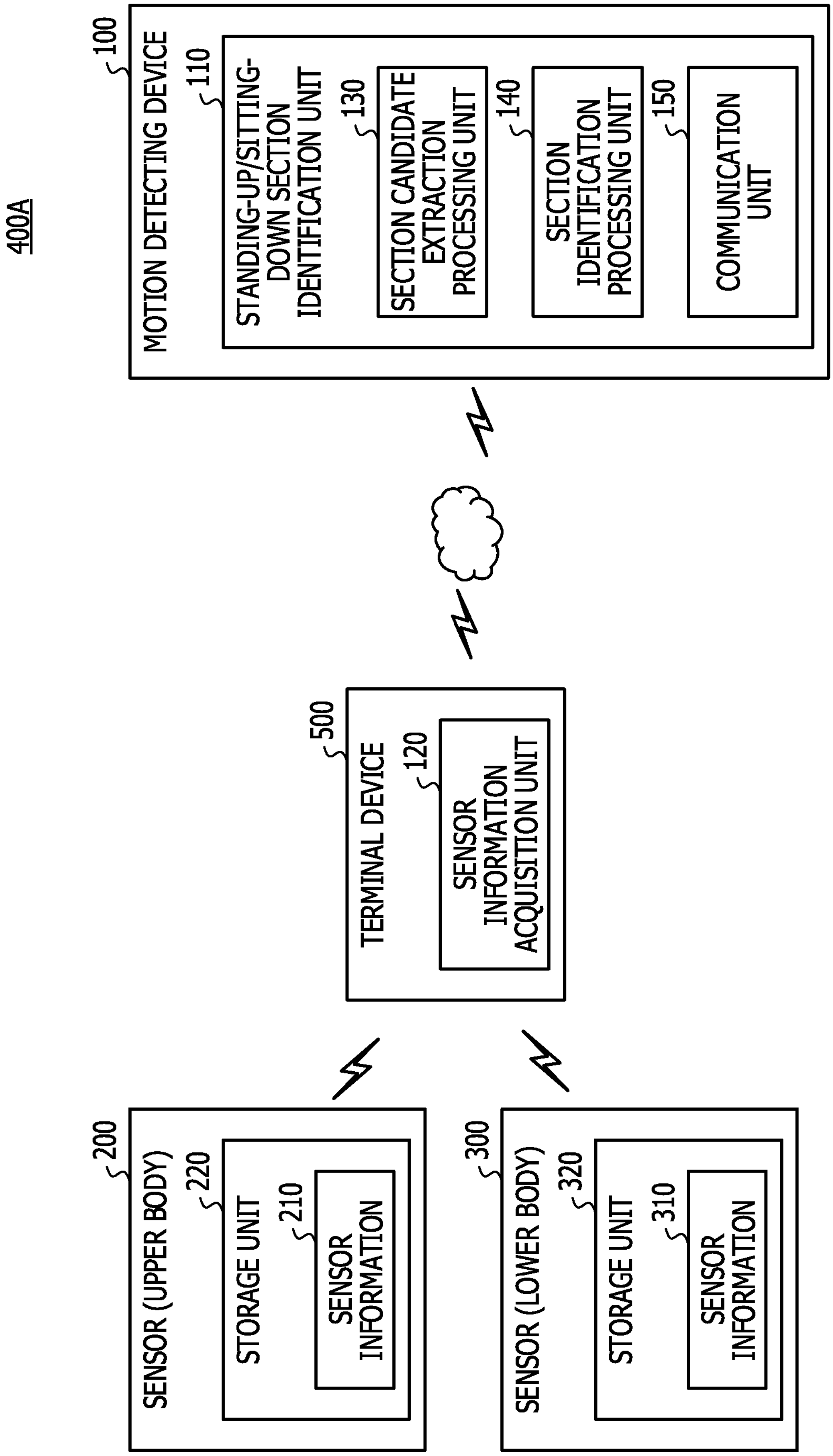
400A
100
MOTION DETECTING DEVICE
110
STANDING-UP/SITTING-DOWN SECTION IDENTIFICATION UNIT
130
SECTION CANDIDATE EXTRACTION PROCESSING UNIT
140
SECTION IDENTIFICATION PROCESSING UNIT
150
COMMUNICATION UNIT
500
TERMINAL DEVICE
120
SENSOR INFORMATION ACQUISITION UNIT
200
SENSOR (UPPER BODY)
220
STORAGE UNIT
210
SENSOR INFORMATION
300
SENSOR (LOWER BODY)
320
STORAGE UNIT
310
SENSOR INFORMATION

FIG. 31

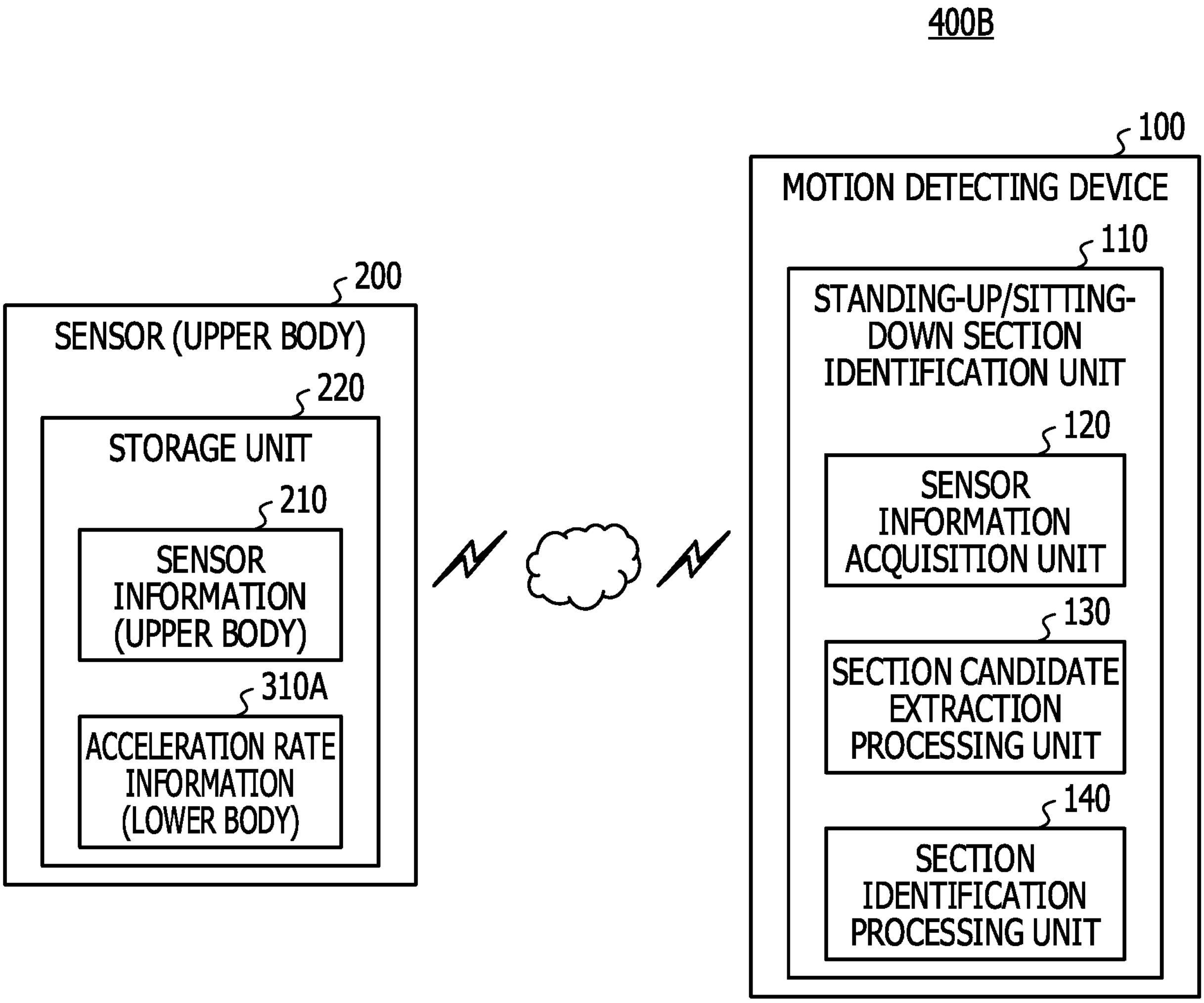
400B
100
MOTION DETECTING DEVICE
110
STANDING-UP/SITTING-DOWN SECTION IDENTIFICATION UNIT
120
SENSOR INFORMATION ACQUISITION UNIT
130
SECTION CANDIDATE EXTRACTION PROCESSING UNIT
140
SECTION IDENTIFICATION PROCESSING UNIT
200
SENSOR (UPPER BODY)
220
STORAGE UNIT
210
SENSOR INFORMATION (UPPER BODY)
310A
ACCELERATION RATE INFORMATION (LOWER BODY)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2016158887 A
- JP 2015177863 A
- US 20140330172 A1